(19) **Europäisches Patentamt / European Patent Office / Office européen des brevets**

(11) **EP 4 623 903 A1**

(12) # EUROPEAN PATENT APPLICATION

(43) Date of publication:
01.10.2025 Bulletin 2025/40

(21) Application number: 24167606.3

(22) Date of filing: **28.03.2024**

(51) International Patent Classification (IPC):
*A61K 9/08* (2006.01)        *A61K 39/395* (2006.01)
*A61K 47/12* (2006.01)       *A61K 47/18* (2017.01)
*A61K 47/26* (2006.01)       *A61K 47/34* (2017.01)
*A61P 11/02* (2006.01)       *A61P 11/06* (2006.01)
*A61P 17/00* (2006.01)       *A61P 37/00* (2006.01)

(52) Cooperative Patent Classification (CPC):
A61K 9/08; A61K 39/39591; A61K 47/12;
A61K 47/18; A61K 47/26; A61K 47/34;
A61P 11/02; A61P 11/06; A61P 17/00; A61P 37/00;
C07K 16/2866

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Fresenius Kabi Deutschland GmbH**
**61352 Bad Homburg (DE)**
• **Coriolis Pharma Research GmbH**
**82152 Martinsried (DE)**

(72) Inventors:
• **SYKES, Alison**
**1262 Eysins (CH)**
• **HAUF, Waldemar**
**82152 Martinsried (DE)**
• **SÃO PEDRO, André Luiz Santos**
**82152 Martinsried (DE)**

(74) Representative: **HGF**
**HGF Limited**
**1 City Walk**
**Leeds LS11 9DX (GB)**

(54) **PHARMACEUTICAL COMPOSITION COMPRISING DUPILUMAB**

(57)    A liquid pharmaceutical composition comprising dupilumab, a surfactant, and water, wherein the composition has a pH of 5.6-6.2; and is free of at least one of histidine, acetate, arginine, and sucrose.
Said composition for use in therapy.

**EP 4 623 903 A1**

## Description

### *INTRODUCTION*

The present invention relates to a pharmaceutical composition, particularly a liquid (e.g. aqueous) pharmaceutical composition comprising dupilumab ("dupi").

### *BACKGROUND*

Dupilumab (formulated by the originator as commercially available DUPIXENT®) is an interleukin-4 receptor alpha antagonist, a human monoclonal antibody of the IgG4 subclass that binds to the IL-4R$\alpha$ subunit and inhibits IL-4 and IL-13 signaling. Dupliumab is currently used for treating moderate-to-severe atopic dermatitis, moderate-to-severe asthma, and for the treatment of chronic rhinosinusitis with nasal polyposis (CRSwNP).

As per Section 11 ("Description") of the FDA Label for DUPIXENT®, revised September 2023 (Reference ID: 5253240), DUPIXENT® is an aqueous composition for subcutaneous (SC) administration, wherein dupilumab is provided in either a single-dose pre-filled syringe with needle shield or a single-dose pre-filled pen in a siliconized Type-1 clear glass syringe. Three formulations of DUPIXENT® are currently available, as shown below:

|  | Mw | 300 mg in 2mL | | | 200 mg in 1.14 mL | | | 100 mg in 0.67 mL | | |
|---|---|---|---|---|---|---|---|---|---|---|
|  |  | mg | mg/mL | mM | mg | mg/mL | mM | mg | mg/mL | mM |
| Dupilumab | 147000 | 300 | 150 | 1.02 | 200 | 175 | 1.19 | 100 | 150 | 1.02 |
| L-Arginine.HCl | 210.66 | 10.5 | 5.3 | 25 | 12 | 10.5 | 50 | 3.5 | 5.3 | 25 |
| L-Histidine | 155.15 | 6.2 | 3.1 | 20 | 3.5 | 3.1 | 20 | 2.1 | 3.1 | 20 |
| Polysorbate 80 | 1310 | 4 | 2 | 1.5 | 2.3 | 2 | 1.5 | 1.3 | 2 | 1.5 |
| Sodium acetate | 82.03 | 2 | 1 | 12.2 | 1.2 | 1.05 | 12.8 | 0.7 | 1.05 | 12.8 |
| Sucrose | 342.3 | 100 | 50 | 146 | 57 | 50 | 146 | 34 | 51 | 149 |
| Water |  | qs |  |  | qs |  |  | qs |  |  |
| pH |  | 5.9 |  |  | 5.9 |  |  | 5.9 |  |  |

Such aqueous dupilumab formulations are foreshadowed in WO2012047954A1 (e.g. Formulation A in Table 7 thereof).

The originator established viable, storable liquid formulations of dupilumab. However, there is still a need for viable alternative dupilumab formulations, for instance, whose performance (e.g. in respect of stability, such as the ability to withstand stresses, such as thermal, mechanical, oxidative, and/or light, to which drug products may be exposed during manufacture, transport, and storage) is comparable to or better than the originator's DUPIXENT® formulations - the present invention seeks to address such an objective. Desirably, any new formulations would solve at least one of the aforementioned problems and/or at least one problem inherent in the prior art, and may suitably solve two or more such problems. In some cases, the problem(s) of the prior art may be solved whilst reducing the complexity of the formulation (be this in terms of product- or process-related features). In some cases, it is an object of the invention to identify viable alternative dupilumab formations which can be simply diluted and/or concentrated to different degrees without needing to modify the relative molar ratios of the respective components of said formulation.

### *SUMMARY OF THE INVENTION*

According to an aspect of the invention, there is provided a liquid pharmaceutical composition comprising dupilumab.

A) Further aspects of the invention, regarding a liquid pharmaceutical composition, are set forth in the following numbered paragraphs A1-A186:

A1. A liquid pharmaceutical composition comprising dupilumab, wherein the composition is further characterised by:

∘ comprising one or more "included" ingredient(s)/component(s)/feature(s), suitably as defined herein (e.g. buffer, sugar, optional additional tonicifier, surfactant, viscosity modifier, water, or any combination thereof), said included ingredient(s)/component(s)/feature(s) optionally being present in any amounts, concentrations, relative concentrations (e.g. molar concentration ratios) as defined herein;

∘ an (substantial) absence of one or more "excluded" (or partially-excluded) ingredient(s)/component(s)/feature(s), suitably as defined herein (e.g. certain amino acids such as histidine and/or arginine, buffers such as acetate, and/or disaccharides such as sucrose);

∘ one or more parameters (e.g. pH, osmolality, ionic strength) and/or features as defined herein; and/or

∘ consisting of said included ingredient(s)/component(s)/feature(s), in which case said composition suitably also includes a (optionally additional) diluent, preferably water.

A2. A liquid pharmaceutical composition comprising dupilumab, wherein the composition is free of at least one of histidine, acetate, arginine, and sucrose.

A3. A liquid pharmaceutical composition comprising dupilumab, wherein the composition is free of at least two of histidine, acetate, arginine, and sucrose.

A4. A liquid pharmaceutical composition comprising dupilumab, wherein the composition is free of at least three of histidine, acetate, arginine, and sucrose.

A5. A liquid pharmaceutical composition comprising dupilumab, wherein the composition is free of all of histidine, acetate, arginine, and sucrose.

A6. A liquid pharmaceutical composition comprising dupilumab, wherein the composition is free of histidine.

A7. A liquid pharmaceutical composition comprising dupilumab, wherein the composition is free of acetate. This aspect may be further limited by the characteristics of A6.

A8. A liquid pharmaceutical composition comprising dupilumab, wherein the composition is free of arginine. This aspect may be further limited by the characteristics of any of A6-A7.

A9. A liquid pharmaceutical composition comprising dupilumab, wherein the composition is free of sucrose. This aspect may be further limited by the characteristics of any of A6-A8.

A10. A liquid pharmaceutical composition comprising dupilumab, wherein the composition has a pH of 5.6-6.2, and the composition is free of any buffers whose conjugate acid has a pKa within +/-1 units of the pH. Such a composition may be considered "bufferless" or "buffer free". This aspect may be further limited by the characteristics of any of A6-A9.

A11. A liquid pharmaceutical composition comprising dupilumab and a surfactant, wherein the composition has a pH of 5.6-6.2, and the composition is free of at least one of histidine, acetate, arginine, and sucrose; wherein the composition may be further limited by the characteristics of any of A6-A10.

A12. A liquid pharmaceutical composition comprising dupilumab, a non-ionic polyol, and a surfactant, wherein the composition has a pH of 5.6-6.2, and the composition is free of at least one of histidine, acetate, arginine, and sucrose; wherein the composition may be further limited by the characteristics of any of A6-A10.

A13. A liquid pharmaceutical composition comprising dupilumab, an organic acid salt, and a surfactant, wherein the composition has a pH of 5.6-6.2, and the composition is free of at least one of histidine, acetate, arginine, and sucrose; wherein the composition may be further limited by the characteristics of any of A6-A10.

A14. A liquid pharmaceutical composition comprising dupilumab, a basic amino acid, and a surfactant, wherein the composition has a pH of 5.6-6.2, and the composition is free of at least one of histidine, acetate, arginine, and sucrose; wherein the composition may be further limited by the characteristics of any of A6-A10.

A15. A liquid pharmaceutical composition comprising dupilumab, an inorganic salt, and a surfactant, wherein the composition has a pH of 5.6-6.2, and the composition is free of at least one of histidine, acetate, arginine, and sucrose; wherein the composition may be further limited by the characteristics of any of A6-A10.

A16. A liquid pharmaceutical composition comprising dupilumab, an organic acid salt, a basic amino acid, and a surfactant, wherein the composition has a pH of 5.6-6.2, and the composition is free of at least one of histidine, acetate, arginine, and sucrose; wherein the composition may be further limited by the characteristics of any of A6-A10.

A17. A liquid pharmaceutical composition comprising dupilumab, a non-ionic polyol, an organic acid salt, and a surfactant, wherein the composition has a pH of 5.6-6.2, and the composition is free of at least one of histidine, acetate, arginine, and sucrose; wherein the composition may be further limited by the characteristics of any of A6-A10.

A18. A liquid pharmaceutical composition comprising dupilumab, a non-ionic polyol, a basic amino acid, and a surfactant, wherein the composition has a pH of 5.6-6.2, and the composition is free of at least one of histidine, acetate, arginine, and sucrose; wherein the composition may be further limited by the characteristics of any of A6-A10.

A19. A liquid pharmaceutical composition comprising dupilumab, a non-ionic polyol, an inorganic salt, and a surfactant, wherein the composition has a pH of 5.6-6.2, and the composition is free of at least one of histidine, acetate, arginine, and sucrose; wherein the composition may be further limited by the characteristics of any of A6-A10.

A20. A liquid pharmaceutical composition comprising dupilumab, a non-ionic polyol, an organic acid salt, a basic amino acid, and a surfactant, wherein the composition has a pH of 5.6-6.2, and the composition is free of at least one of histidine, acetate, arginine, and sucrose; wherein the composition may be further limited by the characteristics of any of A6-A10.

A21. A liquid pharmaceutical composition comprising dupilumab and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A22. A liquid pharmaceutical composition comprising dupilumab, a non-ionic polyol, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A23. A liquid pharmaceutical composition comprising dupilumab, an organic acid salt, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A24. A liquid pharmaceutical composition comprising dupilumab, a basic amino acid, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A25. A liquid pharmaceutical composition comprising dupilumab, an inorganic salt, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A26. A liquid pharmaceutical composition comprising dupilumab, an organic acid salt, a basic amino acid, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A27. A liquid pharmaceutical composition comprising dupilumab, a non-ionic polyol, an organic acid salt, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A28. A liquid pharmaceutical composition comprising dupilumab, a non-ionic polyol, a basic amino acid, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A29. A liquid pharmaceutical composition comprising dupilumab, a non-ionic polyol, an inorganic salt, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A30. A liquid pharmaceutical composition comprising dupilumab, a non-ionic polyol, an organic acid salt, a basic amino acid, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A31. A liquid pharmaceutical composition comprising 90-210 mg/mL dupilumab and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A32. A liquid pharmaceutical composition comprising 90-210 mg/mL dupilumab, 70-230 mM non-ionic polyol, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A33. A liquid pharmaceutical composition comprising 90-210 mg/mL dupilumab, 5-25 mM organic acid salt, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A34. A liquid pharmaceutical composition comprising 90-210 mg/mL dupilumab, 15-150 mM basic amino acid, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A35. A liquid pharmaceutical composition comprising 90-210 mg/mL dupilumab, 15-150 mM inorganic salt, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A36. A liquid pharmaceutical composition comprising 90-210 mg/mL dupilumab, 5-25 mM organic acid salt, 15-150 mM basic amino acid, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A37. A liquid pharmaceutical composition comprising 90-210 mg/mL dupilumab, 70-230 mM non-ionic polyol, 5-25 mM organic acid salt, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A38. A liquid pharmaceutical composition comprising 90-210 mg/mL dupilumab, 70-230 mM non-ionic polyol, 15-150 mM basic amino acid, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A39. A liquid pharmaceutical composition comprising 90-210 mg/mL dupilumab, 70-230 mM non-ionic polyol, 15-150 mM inorganic salt, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A40. A liquid pharmaceutical composition comprising 90-210 mg/mL dupilumab, 70-230 mM non-ionic polyol, 5-25 mM organic acid salt, 15-150 mM basic amino acid, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A10.

A41. A liquid pharmaceutical composition comprising 90-210 mg/mL dupilumab and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A42. A liquid pharmaceutical composition comprising 90-210 mg/mL dupilumab, 70-230 mM non-ionic polyol, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A43. A liquid pharmaceutical composition comprising 90-210 mg/mL dupilumab, 5-25 mM organic acid salt, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A44. A liquid pharmaceutical composition comprising 90-210 mg/mL dupilumab, 15-150 mM basic amino acid, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A45. A liquid pharmaceutical composition comprising 90-210 mg/mL dupilumab, 15-150 mM inorganic salt, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A46. A liquid pharmaceutical composition comprising 90-210 mg/mL dupilumab, 5-25 mM organic acid salt, 15-150 mM basic amino acid, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A47. A liquid pharmaceutical composition comprising 90-210 mg/mL dupilumab, 70-230 mM non-ionic polyol, 5-25 mM organic acid salt, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A48. A liquid pharmaceutical composition comprising 90-210 mg/mL dupilumab, 70-230 mM non-ionic polyol, 15-150 mM basic amino acid, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A49. A liquid pharmaceutical composition comprising 90-210 mg/mL dupilumab, 70-230 mM non-ionic polyol, 15-150 mM inorganic salt, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A50. A liquid pharmaceutical composition comprising 90-210 mg/mL dupilumab, 70-230 mM non-ionic polyol, 5-25 mM organic acid salt, 15-150 mM basic amino acid, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A10.

A51. A liquid pharmaceutical composition comprising dupilumab, a buffer system, and a surfactant, wherein the composition has a pH of 5.6-6.2, and the composition is free of at least one of histidine, acetate, arginine, and sucrose; wherein the composition may be further limited by the characteristics of any of A6-A9.

A52. A liquid pharmaceutical composition comprising dupilumab, a buffer system, a non-ionic polyol, and a surfactant, wherein the composition has a pH of 5.6-6.2, and the composition is free of at least one of histidine, acetate, arginine, and sucrose; wherein the composition may be further limited by the characteristics of any of A6-A9.

A53. A liquid pharmaceutical composition comprising dupilumab, a buffer system, an organic acid salt, and a surfactant, wherein the composition has a pH of 5.6-6.2, and the composition is free of at least one of histidine, acetate, arginine, and sucrose; wherein the composition may be further limited by the characteristics of any of A6-A9.

A54. A liquid pharmaceutical composition comprising dupilumab, a buffer system, a basic amino acid, and a surfactant, wherein the composition has a pH of 5.6-6.2, and the composition is free of at least one of histidine, acetate, arginine, and sucrose; wherein the composition may be further limited by the characteristics of any of A6-A9.

A55. A liquid pharmaceutical composition comprising dupilumab, a buffer system, an inorganic salt, and a surfactant, wherein the composition has a pH of 5.6-6.2, and the composition is free of at least one of histidine, acetate, arginine, and sucrose; wherein the composition may be further limited by the characteristics of any of A6-A9.

A56. A liquid pharmaceutical composition comprising dupilumab, a buffer system, an organic acid salt, a basic amino acid, and a surfactant, wherein the composition has a pH of 5.6-6.2, and the composition is free of at least one of histidine, acetate, arginine, and sucrose; wherein the composition may be further limited by the characteristics of any of A6-A9.

A57. A liquid pharmaceutical composition comprising dupilumab, a buffer system, a non-ionic polyol, an organic acid salt, and a surfactant, wherein the composition has a pH of 5.6-6.2, and the composition is free of at least one of histidine, acetate, arginine, and sucrose; wherein the composition may be further limited by the characteristics of any of A6-A9.

A58. A liquid pharmaceutical composition comprising dupilumab, a buffer system, a non-ionic polyol, a basic amino acid, and a surfactant, wherein the composition has a pH of 5.6-6.2, and the composition is free of at least one of histidine, acetate, arginine, and sucrose; wherein the composition may be further limited by the characteristics of any of A6-A9.

A59. A liquid pharmaceutical composition comprising dupilumab, a buffer system, a non-ionic polyol, an inorganic salt, and a surfactant, wherein the composition has a pH of 5.6-6.2, and the composition is free of at least one of histidine, acetate, arginine, and sucrose; wherein the composition may be further limited by the characteristics of any of A6-A9.

A60. A liquid pharmaceutical composition comprising dupilumab, a buffer system, a non-ionic polyol, an organic acid salt, a basic amino acid, and a surfactant, wherein the composition has a pH of 5.6-6.2, and the composition is free of at least one of histidine, acetate, arginine, and sucrose; wherein the composition may be further limited by the characteristics of any of A6-A9.

A61. A liquid pharmaceutical composition comprising dupilumab, a buffer system, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be

7

further limited by the characteristics of any of A6-A9.

A62. A liquid pharmaceutical composition comprising dupilumab, a buffer system, a non-ionic polyol, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A63. A liquid pharmaceutical composition comprising dupilumab, a buffer system, an organic acid salt, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A64. A liquid pharmaceutical composition comprising dupilumab, a buffer system, a basic amino acid, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A65. A liquid pharmaceutical composition comprising dupilumab, a buffer system, an inorganic salt, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A66. A liquid pharmaceutical composition comprising dupilumab, a buffer system, an organic acid salt, a basic amino acid, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A67. A liquid pharmaceutical composition comprising dupilumab, a buffer system, a non-ionic polyol, an organic acid salt, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A68. A liquid pharmaceutical composition comprising dupilumab, a buffer system, a non-ionic polyol, a basic amino acid, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A69. A liquid pharmaceutical composition comprising dupilumab, a buffer system, a non-ionic polyol, an inorganic salt, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A70. A liquid pharmaceutical composition comprising dupilumab, a buffer system, a non-ionic polyol, an organic acid salt, a basic amino acid, and a surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A71. A liquid pharmaceutical composition comprising 90-210 mg/mL dupilumab, 3-30 mM buffer system, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A72. A liquid pharmaceutical composition comprising 90-210 mg/mL dupilumab, 3-30 mM buffer system, 70-230 mM non-ionic polyol, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A73. A liquid pharmaceutical composition comprising 90-210 mg/mL dupilumab, 3-30 mM buffer system, 5-25 mM organic acid salt, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A74. A liquid pharmaceutical composition comprising 90-210 mg/mL dupilumab, 3-30 mM buffer system, 15-150 mM basic amino acid, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A75. A liquid pharmaceutical composition comprising 90-210 mg/mL dupilumab, 3-30 mM buffer system, 15-150 mM inorganic salt, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A76. A liquid pharmaceutical composition comprising 90-210 mg/mL dupilumab, 3-30 mM buffer system, 5-25 mM organic acid salt, 15-150 mM basic amino acid, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A77. A liquid pharmaceutical composition comprising 90-210 mg/mL dupilumab, 3-30 mM buffer system, 70-230 mM non-ionic polyol, 5-25 mM organic acid salt, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A78. A liquid pharmaceutical composition comprising 90-210 mg/mL dupilumab, 3-30 mM buffer system, 70-230 mM non-ionic polyol, 15-150 mM basic amino acid, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A79. A liquid pharmaceutical composition comprising 90-210 mg/mL dupilumab, 3-30 mM buffer system, 70-230 mM non-ionic polyol, 15-150 mM inorganic salt, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A80. A liquid pharmaceutical composition comprising 90-210 mg/mL dupilumab, 3-30 mM buffer system, 70-230 mM non-ionic polyol, 5-25 mM organic acid salt, 15-150 mM basic amino acid, and 0.1-3 mg/mL surfactant, wherein the composition has a pH of 5.6-6.2, and the composition is free of at least one of histidine, acetate, arginine, and sucrose; wherein the composition may be further limited by the characteristics of any of A6-A9.

A81. A liquid pharmaceutical composition comprising 90-210 mg/mL dupilumab, 3-30 mM buffer system, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A82. A liquid pharmaceutical composition comprising 90-210 mg/mL dupilumab, 3-30 mM buffer system, 70-230 mM non-ionic polyol, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A83. A liquid pharmaceutical composition comprising 90-210 mg/mL dupilumab, 3-30 mM buffer system, 5-25 mM organic acid salt, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A84. A liquid pharmaceutical composition comprising 90-210 mg/mL dupilumab, 3-30 mM buffer system, 15-150 mM basic amino acid, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A85. A liquid pharmaceutical composition comprising 90-210 mg/mL dupilumab, 3-30 mM buffer system, 15-150 mM inorganic salt, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A86. A liquid pharmaceutical composition comprising 90-210 mg/mL dupilumab, 3-30 mM buffer system, 5-25 mM organic acid salt, 15-150 mM basic amino acid, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A87. A liquid pharmaceutical composition comprising 90-210 mg/mL dupilumab, 3-30 mM buffer system, 70-230 mM non-ionic polyol, 5-25 mM organic acid salt, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A88. A liquid pharmaceutical composition comprising 90-210 mg/mL dupilumab, 3-30 mM buffer system, 70-230 mM non-ionic polyol, 15-150 mM basic amino acid, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A89. A liquid pharmaceutical composition comprising 90-210 mg/mL dupilumab, 3-30 mM buffer system, 70-230 mM non-ionic polyol, 15-150 mM inorganic salt, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A90. A liquid pharmaceutical composition comprising 90-210 mg/mL dupilumab, 3-30 mM buffer system, 70-230 mM non-ionic polyol, 5-25 mM organic acid salt, 15-150 mM basic amino acid, and 0.1-3 mg/mL surfactant, wherein the composition has a pH of 5.6-6.2 and an ionic strength between 10-200 mM (preferably between 25-100 mM), and the composition is free of at least one of histidine, acetate, arginine, and sucrose; wherein the composition may be further limited by the characteristics of any of A6-A9.

A91. A liquid pharmaceutical composition comprising dupilumab, and surfactant in a respective molar ratio of 1 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A92. A liquid pharmaceutical composition comprising dupilumab, a basic amino acid, and surfactant in a respective molar ratio of 1 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A93. A liquid pharmaceutical composition comprising dupilumab, an inorganic salt, and surfactant in a respective molar ratio of 1 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose.

A94. A liquid pharmaceutical composition comprising dupilumab, a basic amino acid, an inorganic salt, and surfactant in a respective molar ratio of 1 : 5-300 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A95. A liquid pharmaceutical composition comprising dupilumab, a non-ionic polyol, and surfactant in a respective molar ratio of 1 : 20-500 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A96. A liquid pharmaceutical composition comprising dupilumab, a non-ionic polyol, a basic amino acid, and surfactant in a respective molar ratio of 1 : 20-500 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A97. A liquid pharmaceutical composition comprising dupilumab, a non-ionic polyol, an inorganic salt, and surfactant in a respective molar ratio of 1 : 20-500 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A98. A liquid pharmaceutical composition comprising dupilumab, a non-ionic polyol, a basic amino acid, an inorganic salt, and surfactant in a respective molar ratio of 1 : 20-500 : 5-300 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A99. A liquid pharmaceutical composition comprising dupilumab, an organic acid salt, and surfactant in a respective molar ratio of 1 : 2-50 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A100. A liquid pharmaceutical composition comprising dupilumab, an organic acid salt, a basic amino acid, and surfactant in a respective molar ratio of 1 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A101. A liquid pharmaceutical composition comprising dupilumab, an organic acid salt, an inorganic salt, and surfactant in a respective molar ratio of 1 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A102. A liquid pharmaceutical composition comprising dupilumab, an organic acid salt, a basic amino acid, an inorganic salt, and surfactant in a respective molar ratio of 1 : 2-50 : 5-300 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A103. A liquid pharmaceutical composition comprising dupilumab, a non-ionic polyol, an organic acid salt, and surfactant in a respective molar ratio of 1 : 20-500 : 2-50 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A104. A liquid pharmaceutical composition comprising dupilumab, a non-ionic polyol, an organic acid salt, a basic amino acid, and surfactant in a respective molar ratio of 1 : 20-500 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A105. A liquid pharmaceutical composition comprising dupilumab, a non-ionic polyol, an organic acid salt, an inorganic salt, and surfactant in a respective molar ratio of 1 : 20-500 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A106. A liquid pharmaceutical composition comprising dupilumab, a non-ionic polyol, an organic acid salt, a basic amino acid, an inorganic salt, and surfactant in a respective molar ratio of 1 : 20-500 : 2-50 : 5-300 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A107. A liquid pharmaceutical composition comprising dupilumab, a buffer system, and surfactant in a respective molar ratio of 1 : 2-50 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A108. A liquid pharmaceutical composition comprising dupilumab, a buffer system, a basic amino acid, and surfactant in a respective molar ratio of 1 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A109. A liquid pharmaceutical composition comprising dupilumab, a buffer system, an inorganic salt, and surfactant in a respective molar ratio of 1 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A110. A liquid pharmaceutical composition comprising dupilumab, a buffer system, a basic amino acid, an inorganic salt, and surfactant in a respective molar ratio of 1 : 2-50 : 5-300 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A111. A liquid pharmaceutical composition comprising dupilumab, a buffer system, a non-ionic polyol, and surfactant in a respective molar ratio of 1 : 2-50 : 20-500 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A1 12. A liquid pharmaceutical composition comprising dupilumab, a buffer system, a non-ionic polyol, a basic amino acid, and surfactant in a respective molar ratio of 1 : 2-50 : 20-500 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A1 13. A liquid pharmaceutical composition comprising dupilumab, a buffer system, a non-ionic polyol, an inorganic salt, and surfactant in a respective molar ratio of 1 : 2-50 : 20-500 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A114. A liquid pharmaceutical composition comprising dupilumab, a buffer system, a non-ionic polyol, a basic amino acid, an inorganic salt, and surfactant in a respective molar ratio of 1 : 2-50 : 20-500 : 5-300 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A1 15. A liquid pharmaceutical composition comprising dupilumab, a buffer system, an organic acid salt, and surfactant in a respective molar ratio of 1 : 2-50 : 2-50 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A116. A liquid pharmaceutical composition comprising dupilumab, a buffer system, an organic acid salt, a basic amino acid, and surfactant in a respective molar ratio of 1 : 2-50 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A117. A liquid pharmaceutical composition comprising dupilumab, a buffer system, an organic acid salt, an inorganic salt, and surfactant in a respective molar ratio of 1 : 2-50 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A1 18. A liquid pharmaceutical composition comprising dupilumab, a buffer system, an organic acid salt, a basic amino acid, an inorganic salt, and surfactant in a respective molar ratio of 1 : 2-50 : 2-50 : 5-300 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A1 19. A liquid pharmaceutical composition comprising dupilumab, a buffer system, a non-ionic polyol, an organic acid salt, and surfactant in a respective molar ratio of 1 : 2-50 : 20-500 : 2-50 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A120. A liquid pharmaceutical composition comprising dupilumab, a buffer system, a non-ionic polyol, an organic acid salt, a basic amino acid, and surfactant in a respective molar ratio of 1 : 2-50 : 20-500 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A121. A liquid pharmaceutical composition comprising dupilumab, a buffer system, a non-ionic polyol, an organic acid salt, an inorganic salt, and surfactant in a respective molar ratio of 1 : 2-50 : 20-500 : 2-50 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A122. A liquid pharmaceutical composition comprising dupilumab, a buffer system, a non-ionic polyol, an organic acid salt, a basic amino acid, an inorganic salt, and surfactant in a respective molar ratio of 1 : 2-50 : 20-500 : 2-50 : 5-300 : 5-300 : 0.01-3, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A123. A liquid pharmaceutical composition comprising dupilumab, and surfactant in a respective molar ratio of 1 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A124. A liquid pharmaceutical composition comprising dupilumab, a basic amino acid, and surfactant in a respective molar ratio of 1 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A125. A liquid pharmaceutical composition comprising dupilumab, an inorganic salt, and surfactant in a respective molar ratio of 1 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose.

A126. A liquid pharmaceutical composition comprising dupilumab, a basic amino acid, an inorganic salt, and surfactant in a respective molar ratio of 1 : 10-200 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A127. A liquid pharmaceutical composition comprising dupilumab, a non-ionic polyol, and surfactant in a respective molar ratio of 1 : 50-250 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A128. A liquid pharmaceutical composition comprising dupilumab, a non-ionic polyol, a basic amino acid, and surfactant in a respective molar ratio of 1 : 50-250 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A129. A liquid pharmaceutical composition comprising dupilumab, a non-ionic polyol, an inorganic salt, and surfactant in a respective molar ratio of 1 : 50-250 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A130. A liquid pharmaceutical composition comprising dupilumab, a non-ionic polyol, a basic amino acid, an inorganic salt, and surfactant in a respective molar ratio of 1 : 50-250 : 10-200 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A131. A liquid pharmaceutical composition comprising dupilumab, an organic acid salt, and surfactant in a respective molar ratio of 1 : 5-40 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A132. A liquid pharmaceutical composition comprising dupilumab, an organic acid salt, a basic amino acid, and surfactant in a respective molar ratio of 1 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A133. A liquid pharmaceutical composition comprising dupilumab, an organic acid salt, an inorganic salt, and surfactant in a respective molar ratio of 1 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A134. A liquid pharmaceutical composition comprising dupilumab, an organic acid salt, a basic amino acid, an inorganic salt, and surfactant in a respective molar ratio of 1 : 5-40 : 10-200 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A135. A liquid pharmaceutical composition comprising dupilumab, a non-ionic polyol, an organic acid salt, and surfactant in a respective molar ratio of 1 : 50-250 : 5-40 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A136. A liquid pharmaceutical composition comprising dupilumab, a non-ionic polyol, an organic acid salt, a basic amino acid, and surfactant in a respective molar ratio of 1 : 50-250 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A137. A liquid pharmaceutical composition comprising dupilumab, a non-ionic polyol, an organic acid salt, an inorganic salt, and surfactant in a respective molar ratio of 1 : 50-250 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A138. A liquid pharmaceutical composition comprising dupilumab, a non-ionic polyol, an organic acid salt, a basic amino acid, an inorganic salt, and surfactant in a respective molar ratio of 1 : 50-250 : 5-40 : 10-200 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A139. A liquid pharmaceutical composition comprising dupilumab, a buffer system, and surfactant in a respective molar ratio of 1 : 5-40 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A140. A liquid pharmaceutical composition comprising dupilumab, a buffer system, a basic amino acid, and surfactant in a respective molar ratio of 1 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A141. A liquid pharmaceutical composition comprising dupilumab, a buffer system, an inorganic salt, and surfactant in a respective molar ratio of 1 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A142. A liquid pharmaceutical composition comprising dupilumab, a buffer system, a basic amino acid, an inorganic salt, and surfactant in a respective molar ratio of 1 : 5-40 : 10-200 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A143. A liquid pharmaceutical composition comprising dupilumab, a buffer system, a non-ionic polyol, and surfactant in a respective molar ratio of 1 : 5-40 : 50-250 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A144. A liquid pharmaceutical composition comprising dupilumab, a buffer system, a non-ionic polyol, a basic amino acid, and surfactant in a respective molar ratio of 1 : 5-40 : 50-250 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A145. A liquid pharmaceutical composition comprising dupilumab, a buffer system, a non-ionic polyol, an inorganic salt, and surfactant in a respective molar ratio of 1 : 5-40 : 50-250 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A146. A liquid pharmaceutical composition comprising dupilumab, a buffer system, a non-ionic polyol, a basic amino acid, an inorganic salt, and surfactant in a respective molar ratio of 1 : 5-40 : 50-250 : 10-200 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A147. A liquid pharmaceutical composition comprising dupilumab, a buffer system, an organic acid salt, and surfactant in a respective molar ratio of 1 : 5-40 : 5-40 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A148. A liquid pharmaceutical composition comprising dupilumab, a buffer system, an organic acid salt, a basic amino acid, and surfactant in a respective molar ratio of 1 : 5-40 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A149. A liquid pharmaceutical composition comprising dupilumab, a buffer system, an organic acid salt, an inorganic salt, and surfactant in a respective molar ratio of 1 : 5-40 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A150. A liquid pharmaceutical composition comprising dupilumab, a buffer system, an organic acid salt, a basic amino acid, an inorganic salt, and surfactant in a respective molar ratio of 1 : 5-40 : 5-40 : 10-200 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A151. A liquid pharmaceutical composition comprising dupilumab, a buffer system, a non-ionic polyol, an organic acid salt, and surfactant in a respective molar ratio of 1 : 5-40 : 50-250 : 5-40 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A152. A liquid pharmaceutical composition comprising dupilumab, a buffer system, a non-ionic polyol, an organic acid salt, a basic amino acid, and surfactant in a respective molar ratio of 1 : 5-40 : 50-250 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A153. A liquid pharmaceutical composition comprising dupilumab, a buffer system, a non-ionic polyol, an organic acid salt, an inorganic salt, and surfactant in a respective molar ratio of 1 : 5-40 : 50-250 : 5-40 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A154. A liquid pharmaceutical composition comprising dupilumab, a buffer system, a non-ionic polyol, an organic acid salt, a basic amino acid, an inorganic salt, and surfactant in a respective molar ratio of 1 : 5-40 : 50-250 : 5-40 : 10-200 : 10-200 : 0.1-2, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A155. A liquid pharmaceutical composition comprising dupilumab, and surfactant in a respective molar ratio of 1 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A156. A liquid pharmaceutical composition comprising dupilumab, a basic amino acid, and surfactant in a respective molar ratio of 1 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A157. A liquid pharmaceutical composition comprising dupilumab, an inorganic salt, and surfactant in a respective molar ratio of 1 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose.

A158. A liquid pharmaceutical composition comprising dupilumab, a basic amino acid, an inorganic salt, and surfactant in a respective molar ratio of 1 : 25-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A159. A liquid pharmaceutical composition comprising dupilumab, a non-ionic polyol, and surfactant in a respective molar ratio of 1 : 100-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A160. A liquid pharmaceutical composition comprising dupilumab, a non-ionic polyol, a basic amino acid, and surfactant in a respective molar ratio of 1 : 100-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A161. A liquid pharmaceutical composition comprising dupilumab, a non-ionic polyol, an inorganic salt, and surfactant in a respective molar ratio of 1 : 100-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A162. A liquid pharmaceutical composition comprising dupilumab, a non-ionic polyol, a basic amino acid, an inorganic salt, and surfactant in a respective molar ratio of 1 : 100-150 : 25-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A163. A liquid pharmaceutical composition comprising dupilumab, an organic acid salt, and surfactant in a respective molar ratio of 1 : 8-25 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A164. A liquid pharmaceutical composition comprising dupilumab, an organic acid salt, a basic amino acid, and surfactant in a respective molar ratio of 1 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A165. A liquid pharmaceutical composition comprising dupilumab, an organic acid salt, an inorganic salt, and surfactant in a respective molar ratio of 1 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A166. A liquid pharmaceutical composition comprising dupilumab, an organic acid salt, a basic amino acid, an inorganic salt, and surfactant in a respective molar ratio of 1 : 8-25 : 25-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A167. A liquid pharmaceutical composition comprising dupilumab, a non-ionic polyol, an organic acid salt, and surfactant in a respective molar ratio of 1 : 100-150 : 8-25 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A168. A liquid pharmaceutical composition comprising dupilumab, a non-ionic polyol, an organic acid salt, a basic amino acid, and surfactant in a respective molar ratio of 1 : 100-150 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A169. A liquid pharmaceutical composition comprising dupilumab, a non-ionic polyol, an organic acid salt, an inorganic salt, and surfactant in a respective molar ratio of 1 : 100-150 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A170. A liquid pharmaceutical composition comprising dupilumab, a non-ionic polyol, an organic acid salt, a basic amino acid, an inorganic salt, and surfactant in a respective molar ratio of 1 : 100-150 : 8-25 : 25-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A10.

A171. A liquid pharmaceutical composition comprising dupilumab, a buffer system, and surfactant in a respective molar ratio of 1 : 8-25 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A172. A liquid pharmaceutical composition comprising dupilumab, a buffer system, a basic amino acid, and surfactant in a respective molar ratio of 1 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A173. A liquid pharmaceutical composition comprising dupilumab, a buffer system, an inorganic salt, and surfactant in a respective molar ratio of 1 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A174. A liquid pharmaceutical composition comprising dupilumab, a buffer system, a basic amino acid, an inorganic salt, and surfactant in a respective molar ratio of 1 : 8-25 : 25-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A175. A liquid pharmaceutical composition comprising dupilumab, a buffer system, a non-ionic polyol, and surfactant in a respective molar ratio of 1 : 8-25 : 100-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A176. A liquid pharmaceutical composition comprising dupilumab, a buffer system, a non-ionic polyol, a basic amino acid, and surfactant in a respective molar ratio of 1 : 8-25 : 100-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A177. A liquid pharmaceutical composition comprising dupilumab, a buffer system, a non-ionic polyol, an inorganic salt, and surfactant in a respective molar ratio of 1 : 8-25 : 100-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A178. A liquid pharmaceutical composition comprising dupilumab, a buffer system, a non-ionic polyol, a basic amino acid, an inorganic salt, and surfactant in a respective molar ratio of 1 : 8-25 : 100-150 : 25-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A179. A liquid pharmaceutical composition comprising dupilumab, a buffer system, an organic acid salt, and surfactant in a respective molar ratio of 1 : 8-25 : 8-25 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A180. A liquid pharmaceutical composition comprising dupilumab, a buffer system, an organic acid salt, a basic amino acid, and surfactant in a respective molar ratio of 1 : 8-25 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A181. A liquid pharmaceutical composition comprising dupilumab, a buffer system, an organic acid salt, an inorganic salt, and surfactant in a respective molar ratio of 1 : 8-25 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A182. A liquid pharmaceutical composition comprising dupilumab, a buffer system, an organic acid salt, a basic amino acid, an inorganic salt, and surfactant in a respective molar ratio of 1 : 8-25 : 8-25 : 25-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A183. A liquid pharmaceutical composition comprising dupilumab, a buffer system, a non-ionic polyol, an organic acid salt, and surfactant in a respective molar ratio of 1 : 8-25 : 100-150 : 8-25 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A184. A liquid pharmaceutical composition comprising dupilumab, a buffer system, a non-ionic polyol, an organic acid salt, a basic amino acid, and surfactant in a respective molar ratio of 1 : 8-25 : 100-150 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A185. A liquid pharmaceutical composition comprising dupilumab, a buffer system, a non-ionic polyol, an organic acid salt, an inorganic salt, and surfactant in a respective molar ratio of 1 : 8-25 : 100-150 : 8-25 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

A186. A liquid pharmaceutical composition comprising dupilumab, a buffer system, a non-ionic polyol, an organic acid salt, a basic amino acid, an inorganic salt, and surfactant in a respective molar ratio of 1 : 8-25 : 100-150 : 8-25 : 25-150 : 25-150 : 1-1.7, wherein the composition has a pH of 5.6-6.2 and preferably an ionic strength between 10-200 mM (more preferably between 25-100 mM), and the composition is free of at least one of histidine, acetate, arginine, and sucrose; wherein the composition may be further limited by the characteristics of any of A6-A9.

Preferably, one or more (more preferably two or more, most preferably three or more) of the components (buffer system, non-ionic polyol, organic acid salt, basic amino acid, inorganic salt, surfactant), to the extent they are recited (and/or not absent, e.g. the buffer may be absent), of each of the liquid pharmaceutical compositions defined in aspects A1-A186 are further sub-defined as follows:

- the buffer system comprises one or more buffers (preferably just one buffer), each of which has a conjugate acid form with a pKa within +/-1 unit of the pH of the composition;

- the non-ionic polyol that is a sugar component (i.e. a sugar or sugar alcohol, preferably a non-reducing sugar component);

- the organic acid salt is a monocarboxylate salt (i.e. a molecule which has only one carboxylic moiety that is salted/saltable with a base, such as sodium hydroxide, which thereby excludes zwitterionic amine-carboxylates pairs - i.e. glutamate or aspartate fulfils such a definition as well as acetate, lactate, propanoate, and the like);

- the basic amino acid is amino acid with a basic side chain comprising a basic group (e.g. amine, guanidine) whose conjugate acid has a pKa greater than or equal to 7 (preferably greater than or equal to 9);

- the inorganic salt is an alkali or alkaline earth metal halide; AND/OR

- the surfactant is a non-ionic surfactant.

Preferably, the components (buffer system, non-ionic polyol, organic acid salt, basic amino acid, inorganic salt, surfactant), to the extent they are recited (and/or not absent, e.g. the buffer may be absent), of each of the liquid pharmaceutical compositions defined in aspects A1-A186 are all further sub-defined as follows:

- the buffer system comprises one or more buffers (preferably just one buffer), each of which has a conjugate acid form with a pKa within +/-1 unit of the pH of the composition;

- the non-ionic polyol that is a sugar component (i.e. a sugar or sugar alcohol, preferably a non-reducing sugar component);

- the organic acid salt is a monocarboxylate salt (i.e. a molecule which has only one carboxylic moiety that is salted/saltable with a base, such as sodium hydroxide, which thereby excludes zwitterionic amine-carboxylates pairs - i.e. glutamate or aspartate fulfils such a definition as well as acetate, lactate, propanoate, and the like);

- the basic amino acid is amino acid with a basic side chain comprising a basic group (e.g. amine, guanidine) whose conjugate acid has a pKa greater than or equal to 7 (preferably greater than or equal to 9);

- the inorganic salt is an alkali or alkaline earth metal halide; AND

- the surfactant is a non-ionic surfactant.

Preferably, one or more (more preferably two or more, most preferably three or more) of the components (buffer system, non-ionic polyol, organic acid salt, basic amino acid, inorganic salt, surfactant), to the extent they are recited (and/or not absent, e.g. the buffer may be absent), of each of the liquid pharmaceutical compositions defined in aspects A1-A186 are further sub-defined as follows:

- the buffer system comprises a buffer selected from a histidine buffer, a succinate buffer, a phosphate buffer, an MES buffer, or a combination of any two thereof;

- the non-ionic polyol is selected from trehalose, sucrose, mannitol, or sorbitol;

- the organic acid salt is selected from acetate, lactate, or glutamate;

- the basic amino acid is selected from arginine or lysine;

- the inorganic salt is selected from NaCl, KCl, $MgCl_2$, or $NH_4Cl$; AND/OR

- the surfactant is selected from a polysorbate or a poloxamer.

Preferably, one or more (more preferably two or more, most preferably three or more) of the components (buffer system, non-ionic polyol, organic acid salt, basic amino acid, inorganic salt, surfactant), to the extent they are recited (and/or not absent, e.g. the buffer may be absent), of each of the liquid pharmaceutical compositions defined in aspects A1-A186 are further sub-defined as follows:

- the buffer system is either a succinate buffer alone, or a phosphate buffer alone, most preferably a succinate buffer alone;

- the non-ionic polyol is trehalose;

- the organic acid salt is lactate;

- the basic amino acid is lysine;

- the inorganic salt is NaCl; AND/OR

- the surfactant is polysorbate 80.

Preferably, the components (buffer system, non-ionic polyol, organic acid salt, basic amino acid, inorganic salt, surfactant), to the extent they are recited (and/or not absent, e.g. the buffer may be absent), of each of the liquid pharmaceutical compositions defined in aspects A1-A186 are all further sub-defined as follows:

- the buffer system comprises a buffer selected from a histidine buffer, a succinate buffer, a phosphate buffer, an MES buffer, or a combination of any two thereof;

- the non-ionic polyol is selected from trehalose, sucrose, mannitol, or sorbitol;

- the organic acid salt is selected from acetate, lactate, or glutamate;

- the basic amino acid is selected from arginine or lysine;

- the inorganic salt is selected from NaCl, KCl, MgCl$_2$, or NH$_4$Cl; AND

- the surfactant is selected from a polysorbate or a poloxamer.

More preferably, the components (buffer system, non-ionic polyol, organic acid salt, basic amino acid, inorganic salt, surfactant), to the extent they are recited (and/or not absent, e.g. the buffer may be absent), of each of the liquid pharmaceutical compositions defined in aspects A1-A186 are all further sub-defined as follows:

- the buffer system is either a succinate buffer alone, or a phosphate buffer alone, most preferably a succinate buffer alone;

- the non-ionic polyol is trehalose;

- the organic acid salt is lactate;

- the basic amino acid is lysine;

- the inorganic salt is NaCl; AND

- the surfactant is polysorbate 80.

Preferably, each of the liquid pharmaceutical compositions defined in aspects A1-A186 comprise water (i.e. water for injection). Suitably, any of the liquid pharmaceutical compositions defined in aspects A1-A186 may consist of the stipulated components along with water.

According to a further aspect of the present invention there is provided a method of manufacturing a liquid pharmaceutical composition as defined in any of aspects A1-A186, the method comprising mixing together (or otherwise combining) the components included in the liquid pharmaceutical composition, most suitably in a diluent (preferably water for injection), optionally in any amounts, concentrations, and/or forms defined herein; and optionally adjusting any one or more parameters stipulated herein in relation to the liquid pharmaceutical composition (e.g. adjusting pH to achieve the desired or stipulated pH).

According to a further aspect of the present invention there is provided a liquid pharmaceutical composition obtainable by, obtained by, or directly obtained by a method of manufacturing a pharmaceutical composition as defined herein.

According to a further aspect of the present invention there is provided a drug delivery device (e.g. vial, ampoule, syringe, pre-filled syringe, injection pen (e.g. essentially incorporating a syringe), autoinjector, or intravenous bag) comprising a liquid pharmaceutical composition as defined in any of aspects A1-A186.

According to a further aspect of the present invention there is provided a kit of parts comprising a drug delivery device (e.g. vial, ampoule, syringe, pre-filled syringe, injection pen (e.g. essentially incorporating a syringe), autoinjector, or intravenous bag), a liquid pharmaceutical composition as defined in any of aspects A1-A186 (optionally contained in a container, suitably as defined herein, which container suitably is, is associated with, or is a part of the drug delivery device), and optionally a set of instructions with directions regarding the administration (e.g. sub-cutaneous) of the liquid pharmaceutical composition.

According to a further aspect of the present invention there is provided a method of treating a disease or medical disorder in a patient in need of such treatment, said method comprising administering to said patient a therapeutically effective amount of a liquid pharmaceutical composition as defined in any of aspects A1-A186. Administering most preferably comprises subcutaneously-administering a therapeutically effective amount of the pharmaceutical composition, suitably *via* subcutaneous injection.

According to a further aspect of the present invention there is provided a liquid pharmaceutical composition, as defined in any of aspects A1-A186, for use in treating a disease or medical disorder in a patient in need of such treatment.

According to a further aspect of the present invention there is provided a use of a liquid pharmaceutical composition, as defined in any of aspects A1-A186, in the manufacture of a medicament for the treatment of a disease or disorder.

According to a further aspect of the present invention, there is provided (suitably as defined herein) a method of treating a disease or medical disorder, a liquid pharmaceutical composition for use in treating a disease or medical disorder, or a use of a liquid pharmaceutical composition in the manufacture of a medicament for a treatment of a disease or disorder, wherein the disease or medical disorder is selected from the group consisting of atopic dermatitis (suitably moderate-to-severe), asthma (suitably moderate-to-severe), chronic rhinosinusitis with nasal polyposis (CRSwNP), and eosinophilic esophagitis.

According to a further aspect of the present invention, there is provided (suitably as defined herein) a method of treating a disease or medical disorder, a pharmaceutical composition for use in treating a disease or medical disorder, or a use of a pharmaceutical composition in the manufacture of a medicament for a treatment of a disease or disorder, wherein the disease or medical disorder is selected from the group consisting of atopic dermatitis (suitably moderate-to-severe), asthma (suitably moderate-to-severe), chronic rhinosinusitis with nasal polyposis (CRSwNP), and eosinophilic esophagitis.

All of the aforesaid methods of treatment, composition(s) for use, and use of composition(s) in the manufacture of a medicament, are equally applicable to the relevant packages, containers, pharmaceutical products, drug-delivery devices, and kits incorporating said composition(s).

Any features, including optional, suitable, and preferred features, described in relation to any particular aspect of the invention may also be features, including optional, suitable and preferred features, of any other aspect of the present invention. Moreover, any features, including optional, suitable, and preferred features, described in any of the aforesaid aspects, may be further defined as set forth elsewhere herein (e.g. in the description that follows, and/or the appended claims).

## BRIEF DESCRIPTION OF THE DRAWINGS

For a better understanding of the invention, and to show how embodiments of the same are put into effect, reference is now made, by way of example, to the following figures, in which:

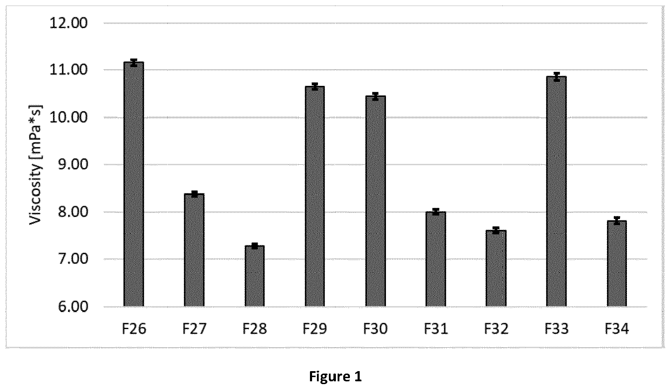

Figure 1 is bar chart showing the viscosities (at 25C) of formulations F26-F34 at Time 0.

## DETAILED DESCRIPTION OF THE INVENTION

### DEFINITIONS

Unless otherwise stated, the following terms used in the specification and claims have the following meanings set out below. Many of the terms used throughout the present application are terms of art that a person skilled in the art of antibody formulation science would readily understand.

Unless otherwise stated, wherever a dependent claim/paragraph depends upon a stipulated claim/paragraph "... *or any other claims/paragraphs dependent thereon"* (or similar such language, e.g. "the composition as defined in paragraph A1 or any preceding paragraphs dependent thereon"), such "other claims/paragraphs" suitably include any claims/paragraphs that depend directly or indirectly (i.e. *via* one or more other dependencies) on the stipulated claim/paragraph. In such a manner, numbered paragraphs, much like patent claims, may be multiply dependent.

References herein to a pharmaceutical or pharmaceutical active, including dupilumab, include the originator drug substance, whether as commercially available, as described in a patent document, or as described elsewhere in the art, and also biosimilars thereof. Such references may also include biobetters (or biosuperiors) of an originator drug substance.

Dupilumab (formulated by the originator as commercially available DUPIXENT®), with the CAS# 1190264-60-8, is an interleukin-4 receptor alpha antagonist, a human monoclonal antibody of the IgG4 subclass that binds to the IL-4R$\alpha$ subunit and inhibits IL-4 and IL-13 signalling. Dupilumab has an approximate molecular weight of 147 kDa (146896.9522 in Kegg Database, entry number D10354), and for the purpose of molar calculations herein the molecular weight is taken as 147 kDa (e.g. 150 mg/mL and 175 mg/mL dupilumab respectively equates to 1.02 mM and 1.19 mM). Dupilumab is well known, as is its structure and method of production (the DUPIXENT FDA Label, revised September 2023, explains in Section 11, "Description", that "Dupilumab is produced by recombinant DNA technology in Chinese Hamster Ovary cell suspension culture"), and further details thereof are available in US 7,608,693, US 8,735,095, WO2012047954A1, and WO2016077675A1, all filed by Regeneron Pharmaceuticals Inc. References herein to dupilumab (abbrev "dupi") encompass both originator and biosimilars thereof.

Unless stated otherwise, references herein to a "pKa" should be construed as a pKa value in water at standard ambient temperature and pressure (SATP), suitably of the conjugate acid of the relevant species.

Herein, an "osmolality" of a formulation may be measured by a variety of methods (and with a variety of equipment, such as an osmometer) well known in the art. The osmolality of a formulation may be measured using methods (and equipment) as mentioned herein, such as in the Example section. Alternatively, the osmolality of a formulation may be calculated as well-known in the art, noting the osmolality is generally the same as osmolarity in aqueous formulations with a density of approximately 1 g/mL (which, in general, may be presumed for compositions of the invention). Osmolality calculations, however, exclude any contributions thereto by the antibody. For instance, the osmolarity of a solution, in osmoles per liter (osmol/L) may be calculated as follows:

$$Osmolarity = \sum_i \varphi_i n_i C_i$$

where each index i represents the identity of a given solute, $\varphi$ is the osmotic coefficient relevant to each said solute (and is preferably approximated to 1 for all solutes for the purposes of the invention), $n$ is the number of particles (e.g. ions) into which each said solute molecule dissociates (for sorbitol $n$ is 1, for NaCl $n$ is 2, for $MgCl_2$ $n$ is 3), and $C$ is the molar concentration of each said solute. Since osmotic coefficients may be ignored (i.e. presumed to be 1 for each solute), the osmolarity (and thus osmolality, since formulations of the invention may be presumed to have a density of approximately 1 g/mL) calculated may be simplified thus:

$$Osmolarity = \sum_i n_i C_i$$

Herein, an "ionic strength provider" is a compound, typically a salt, that imparts (or increases) ionic strength in a solution, generally an aqueous solution. Unless otherwise stated, herein ionic strength is measured as a molar ionic strength of a solution (I), which is a function of the concentration of all ions present in said solution. Molar ionic strength (a well known term of art) may be expressed thus:

$$I = \frac{1}{2} \cdot \sum_{i=1}^{n} c_i z_i^2$$

where I is the molar ionic strength, $c_i$ is the molar concentration of ion $i$ (preferably as a molarity, such as mM), $z_i$ is the charge number of that ion, and the sum is taken over all ions in solution. Calculated ionic strengths generally exclude any contributions from an antibody. Ionic strengths may be defined by way of a range, especially where buffers are present, because the ionic strength of buffers is generally pH dependent. However, it is straightforward to calculate ionic strength ranges for a buffer (from one extreme to another, between relevant key protonation states of the relevant buffering species).

The term "buffer" (or "buffer system") is well known in the art. Herein, the terms "buffer" and "buffer system" may be used interchangeably. However, a buffer system may contain multiple buffers or a single buffer. Herein, a "buffer system" comprises one or more buffering agent(s) and/or an acid/base conjugate(s) thereof. One may view a buffer as a combination of conjugate acid and conjugate base forms thereof, or alternatively as a combination of a buffering agent and conjugate acid or conjugate base form thereof. Concentrations of the different buffering species (i.e. conjugate acid/base forms), and/or pH, may be calculated (e.g. *via* the well known "Henderson-Hasselbalch equation") or experimentally determined. A buffer preferably refers to a system that has a reasonable or good buffering capacity at a prevailing pH. Preferably, the term "buffer" is used to denote a system where the conjugate acid form of the buffer (or of a buffering pair) has a pK$_a$ within +/-1 units of the prevailing (or target) pH, more preferably within +/- 0.5 units of the prevailing (or target) pH; the relevant conjugate acid form being the conjugate acid form whose pKa is closest (compared to other buffer forms) to the prevailing pH. As such, where the aforementioned limitations apply, neither acetate (whose conjugate acid, acetic acid, has a pKa of 4.76), lactate (whose conjugate acid, lactic acid, has a pKa of 3.86), nor glutamate (whose conjugate acid, glutamic acid, has a pKa of 4.3) would constitute a buffer at pH 5.9.

The term "organic acid salt" is a well-known term of art and includes a salt form of any organic compound comprising an acid moiety (e.g. carboxylate, sulphate, phosphate, etc.). The organic acid salt is preferably not a buffer, in the sense that its free acid form (e.g. carboxylic acid where the salt is a carboxylate) preferably has a pKa that is outside +/- 1 (preferably outside +/- 0.5) units of the prevailing (or target) pH. Examples may include acetate, lactate, glutamate, aspartate, and the like.

The term "non-ionic polyol" is well understood in the art and encompasses any compound with a plurality of alcohol moieties that is net neutral in terms of charge. Preferably said non-ionic polyols are non-ionisable and do not exhibit zwitterionic forms. Non-ionic polyols in their broadest sense include, for example, short C2-C3 polyols (e.g. propylene glycol, glycerol), sugar alcohols (e.g. sorbitol, mannitol), and sugars (e.g. trehalose, sucrose). The term "sugar component" includes both sugar alcohols and sugars. Preferably, the non-ionic polyol is a "non-reducing sugar component", which means a sugar or sugar alcohol without any aldehyde moieties or without the capability of forming an aldehyde moiety (e.g. through isomerism or tautomerisation).

Unless stated otherwise, references herein to an "amino acid" or "amino acids", whether specific (e.g. arginine, lysine, histidine) or general (e.g. "basic amino acid", "non-basic/non-acidic amino acid"), in the context of their presence or otherwise within compositions of the invention, relate to the corresponding <u>free amino acid(s)</u> (regardless of its/their protonation state and/or salt form, though for consistency amounts may be calculated by reference to the free amino acid *per se*), and not to <u>amino acid residue(s)</u> within an antibody. Unless stated otherwise, references herein to any one or more "amino acids", whether specific or general, suitably relate to the L- stereoisomers or a racemate thereof, most suitably L-amino acids. Most preferably and such amino acids are natural amino acids.

The term "basic amino acid" refers to an amino acid with a basic side chain (i.e. a side chain that will generally be, or at least partially be, in a protonated or salt form at pH 7). Examples of natural amino acids fulfilling such a definition include arginine, lysine, and to a lesser extent histidine. References herein to a "basic amino acid" preferably includes free (zwitterionic) and salt forms (e.g. hydrochloride salts) thereof. Generally, such basic amino acids contribute to ionic strength at prevailing pHs (e.g. pH 5.6-6.2) because a significant amount of said basic amino acid will be in a salt (i.e. protonated) form.

The term "non-basic/non-acidic amino acid" refers to an amino acid whose side chain is both non-basic and non-acidic. Such an amino acid is uncharged (i.e. neutral) at pH 7, though it may of course exist in zwitterionic form. For instance, this excludes basic amino acids (e.g. arginine, lysine, histidine), and acidic amino acids (e.g. aspartic acid, glutamic acid). Examples of non-basic/non-acid amino acids includes glycine, proline, and the like.

Herein, an "antioxidant" is a compound that mitigates oxidation of groups within dupilumabthat might otherwise be vulnerable to oxidation. The amino acids, methionine and cysteine are antioxidants in accordance with the invention. However, a "chelator" may also serve as an antioxidant through being capable of complexing, preferably in a multidentate manner, with various groups, molecules, atoms, or ions, that may otherwise impart oxidative stress. EDTA (and salts thereof) is an example of a chelator antioxidant.

The term "inorganic salt" is well known in the art and includes the likes of sodium chloride. An inorganic salt preferably has no organic cations or anions.

The term "surfactant" is well known in the art. Examples include polysorbates, poloxamers, kolliphors, and the like.

Herein, a "tonicity modifier" or "tonicifier" refers to a compound whose inclusion within a composition contributes to (or increases) the overall osmolality and osmolarity of the composition.

Herein, the term "pharmaceutical composition" refers to a formulation of a (bio)pharmaceutical active which renders the biological activity of the active ingredient therapeutically effective, but which does not include other ingredients which are obviously toxic to a subject to which the formulation is intended to be administered.

Herein, the term "component" is often used interchangeably with the term "ingredient", especially in the context of compositions. A component/ingredient may be a specific component/ingredient, such as sorbitol, or a general component/ingredient (e.g. ingredient class) such as a sugar component, where a specific example of the sugar component is sorbitol.

An ingredient (or component) may in itself be composed of (or comprise) a plurality of ingredients (e.g. sub-ingredients). For example, a buffer system may include a plurality of buffers. As such, any general principles defined herein in respect of a composition (e.g. amounts; the notion of a composition consisting essentially of, or consisting of one or more components - this may equally apply to a component with sub-components/sub-ingredients) may also be applicable to a component capable itself of comprising a plurality of sub-components.

Herein, amounts stipulated for components and ingredients, whether specified in terms of "parts", ppm (parts per million), percentages (%, e.g. wt%), or ratios, are intended to be by weight, unless stated otherwise.

Where the quantity or concentration of a particular component of a given composition is specified as a weight percentage (wt% or %w/w), said weight percentage refers to the percentage of said component by weight relative to the total weight of the composition as a whole. It will be understood by those skilled in the art that the sum of weight percentages of all components of a composition (whether or not specified) will total 100 wt%. However, where not all components are listed (e.g. where compositions are said to "comprise" one or more particular components), the weight percentage balance may optionally be made up to 100 wt% by unspecified ingredients (e.g. a diluent, such as water, or other non-essentially but suitable additives).

Where a composition is said to comprise a plurality of stipulated ingredients (optionally in stipulated amounts, concentrations, relative molar ratios, relative weight ratios, etc.), said composition may include additional ingredients other than those stipulated. However, in certain embodiments (especially embodiments and aspects that contain a plurality of ingredients/components, especially where amounts are stipulated, especially where a diluent, e.g. water, is also present), a composition said to comprise a plurality of stipulated ingredients may in fact consist essentially of or consist of all the stipulated ingredients (optionally along with water for injection wherever a diluent is not specified).

Herein, molar ratios (or parts by moles) between respective components may be expressed in a number of ways, as well known in the art. For example, molar ratios between Component X, Component Y, and Component Z may be expressed in terms of absolute values (e.g. $x_1 : y_1 : z_1$) or ranges (e.g. $x_1$-$x_2 : y_1$-$y_2 : z_1$-$z_2$ or $x_1 : y_1$-$y_2 : z_1$-$z_2$).

Where a composition is said to "consists essentially of" particular components/ingredients, said composition suitably comprises at least 70 wt% of said components/ingredients, suitably at least 90 wt% thereof, suitably at least 95 wt% thereof, most suitably at least 99 wt% thereof. Suitably, a composition said to "consist essentially of" particular components/ingredients consists of said components/ingredients save for one or more trace (suitably *de minimis*) impurities.

Where a composition is said to "consist of" a plurality of components, whether defined generically or specifically, this preferably includes associated species, such as counterions (e..g. Na$^+$), acids (e.g. HCl), solvates (e.g. H$_2$O), that are not necessarily recited. For instance, a composition that consist of, *inter alia,* a basic amino acid, includes a basic amino acid that is salted (e.g. with HCl or such like) and thus includes additional species that contribute to said salt. In the case of organic acid salt, it includes amounts of organic acid the co-exists in equilibrium with the organic acid salt.

Preferably, and/or unless stated otherwise, where multiple components/ingredients are stipulated, each individual component/ingredient is a distinct and separate from any others regardless of whether definitions therefor overlap. For instance, if both a buffer and inorganic salt are stipulated, this indicates that a buffer is required (which may happen to be or comprise an inorganic salt) and an additional inorganic salt.

Wherever a composition is said to be "free of [*a particular component*]" or "characterised by an absence of [*a particular component*]", this suitably means that the composition in question is either substantially free or entirely free of said component.

When a composition is "substantially free" of a given component, said composition suitably comprises no more than 0.1 wt% of said component, suitably no more than 0.01 wt% of said component, suitably no more than 0.001 wt% of said component, suitably no more than 0.0001 wt% of said component, suitably no more than 0.00001 wt%, suitably no more than 0.000001 wt%, suitably no more than 0.0000001 wt% thereof, most suitably no more than 0.0001 parts per billion (by weight).

Herein, unless otherwise stated, the term "viscosity" refers to dynamic viscosity at 25°C measured by a rheometer at a constant shear rate of 200 s$^{-1}$.

Herein, unless otherwise stated, the term "opalescence" (sometimes referred to as "turbidity" , "haziness", "cloudiness") generally refers to opalescence typically determined by UV spectroscopy (e.g. light scatter measured by absorbance at OD350nm and OD550nm) and/or Nephelometry (e.g. using a turbidimeter operating at 400nm to 600nm and detecting at a 90° angle), the latter of which usually reports results in Nephelometric Turbidity Units (NTU)..

Suitably, unless stated otherwise, where reference is made to a parameter (e.g. pH, pKa, etc.) or state of a material (e.g. liquid, gas, etc.) which may depend on pressure and/or temperature, suitably in the absence of further clarification such a reference refers to said parameter at standard ambient temperature and pressure (SATP). SATP is a temperature of 298.15 K (25 °C, 77 °F) and an absolute pressure of 100 kPa (14.504 psi, 0.987 atm).

Herein, unless incompatible in a given context, wherever a component is stipulated which is capable of ionization (e.g. protonation or deprotonation), the definition of said component suitably includes any suitable salts thereof, suitably pharmaceutically acceptable salts thereof. For example, references herein to the succinic acid suitably includes succinate salts, unless the context dictates otherwise. Likewise, unless incompatible in a given context, wherever a component is stipulated which is capable of neutralisation, the definition of said component suitably includes neutralised forms thereof. For instance, references herein to succinates may suitably include succinic acid.

## GENERAL POINTS AND ADVANTAGES REGARDING THE INVENTION

Many of the advantages of the present invention, and indeed the challenges involved in its conception and development, will be self-evident. The inventive endeavours elucidated in this disclosure represent a significant contribution to the art, a contribution to which the present invention is commensurate in scope.

The present invention provides alternative (particularly a viable alternative) dupilumab formulations to those of the prior art, and in particular an alternative (particularly a viable alternative) dupilumab formulation to the originator DUPIXENT® formulations. Suitably, such alternative dupilumab formulations provide comparable or improved stability (especially with respect to antibody stability as measured before and after stress testing) compared to those of the prior art, especially compared to the originator DUPIXENT® formulations. Indeed, dupilumab formulations of the invention generally withstand various stresses (e.g. agitation, heat, light, oxidation) to which drug products may be exposed during manufacture, transport, and storage.

Typically, such alternatives use a different combination of components and/or relative concentrations thereof, and in some cases use fewer components.

Many of the formulations of the invention alleviate or eliminate one or more problems associated with prior art formulations, especially those of the originator DUPIXENT® formulations and other such formulations explored in WO2012047954A1 (Regeneron Pharmaceuticals) and U.S. Patent No. 7,608,693 (Regeneron Pharmaceuticals).

Advantageously, many dupilumab formulations of the invention may be readily diluted without detriment and, if desirable, respiked with the diluted excipients to restore their original concentrations whilst retaining the lower (diluted) concentration of dupilumab. This is helpful in terms of manufacturing different dosage forms with different dupilumab concentrations.

Advantageously, many dupilumab formulations of the invention exhibit comparable or reduced viscosity and/or opalescence/turbidity compared to the originator DUPIXENT® formulations. Suitably, formulations of the invention exhibit comparable or improved syringeability compared to the originator DUPIXENT® formulations.

## PHARMACEUTICAL COMPOSITION

The present invention provides a liquid pharmaceutical composition comprising dupilumab. The liquid pharmaceutical composition may be any of the liquid pharmaceutical compositions defined in aspects A1-A186.

The liquid pharmaceutical composition preferably comprises a diluent, which most preferably is water (for injection). This preference is applicable to all of aspects A1-A186.

The liquid pharmaceutical composition suitably comprises dupilumab, and one or more components selected from the group consisting of an organic acid salt, a non-ionic polyol, a basic amino acid, an inorganic salt, a non-basic/non-acidic amino acid, an antioxidant, and a surfactant; wherein the composition is free of at least one of histidine, acetate, arginine, and sucrose.

The liquid pharmaceutical composition suitably comprises dupilumab, and two or more components selected from the group consisting of an organic acid salt, a non-ionic polyol, a basic amino acid, an inorganic salt, a non-basic/non-acidic amino acid, an antioxidant, and a surfactant; wherein the composition is free of at least two of histidine, acetate, arginine, and sucrose.

The liquid pharmaceutical composition suitably comprises dupilumab, and three or more components selected from the group consisting of an organic acid salt, a non-ionic polyol, a basic amino acid, an inorganic salt, a non-basic/non-acidic amino acid, an antioxidant, and a surfactant; wherein the composition is free of at least three of histidine, acetate, arginine, and sucrose.

The liquid pharmaceutical composition suitably comprises dupilumab, and four or more components selected from the group consisting of an organic acid salt, a non-ionic polyol, a basic amino acid, an inorganic salt, a non-basic/non-acidic amino acid, an antioxidant, and a surfactant; wherein the composition is free of all of histidine, acetate, arginine, and sucrose.

The liquid pharmaceutical composition suitably comprises dupilumab, and three or more components selected from the group consisting of a buffer system, an organic acid salt, a non-ionic polyol, a basic amino acid, an inorganic salt, a non-basic/non-acidic amino acid, an antioxidant, and a surfactant; wherein the composition is free of at least three of histidine, acetate, arginine, and sucrose.

The liquid pharmaceutical composition suitably comprises dupilumab, and four or more components selected from the group consisting of a buffer system, an organic acid salt, a non-ionic polyol, a basic amino acid, an inorganic salt, a non-basic/non-acidic amino acid, an antioxidant, and a surfactant; wherein the composition is free of all of histidine, acetate, arginine, and sucrose.

The liquid pharmaceutical composition suitably comprises dupilumab, an organic acid salt, a non-ionic polyol, either a basic a basic amino acidor an inorganic salt, and a surfactant; wherein the composition is free of at least two of histidine, acetate, arginine, and sucrose.

The liquid pharmaceutical composition may be buffer-free. For example, the liquid pharmaceutical compositions according to aspects A1-A10, A11-A50, A91-A1 06, A123-A138, and A155-A170, may be essentially buffer free in that they are free of any buffers whose conjugate acid has a pKa within +/-1 units of the pH.

The liquid pharmaceutical composition may, however, comprise a buffer system. For instance, the liquid pharmaceutical compositions according to aspects A1-A9, A51-A88, A107-A122, A139-A154, and A171-A186, may comprise a buffer system. Whist the buffer system may comprise or consist of two buffers, most preferably, where a buffer system is present, the buffer system consists of only a single buffer. Under such circumstances, preferably no other components (save for the antibody itself to the extent it may exhibit inherant buffering capacity) constitute a buffer in the sense of having a conjugate acid form that exhibits a pKa within +/- 1 units of the pH.

The liquid pharmaceutical composition suitably has a pH between 5.6 and 6.2. Preferably, however, the pH of the liquid pharmaceutical composition is between 5.8 and 6.0.

The liquid pharmaceutical composition preferably comprises an organic acid salt.

The liquid pharmaceutical composition preferably comprises a non-ionic polyol.

The liquid pharmaceutical composition preferably comprises a basic amino acid (though most suitably it is a salt or partial salt thereof).

The liquid pharmaceutical composition may suitably comprise an inorganic salt.

Preferably, where a basic amino acid is present within the pharmaceutical composition, an inorganic salt is absent, and *vice versa.* Both may be considered (additional) ionic strength providers, but preferably a composition contains one of but not both of a basic amino acid (which is preferably a salt or partial salt thereof) and an inorganic salt.

The liquid pharmaceutical composition may suitably comprise a non-basic/non-acidic amino acid.

The liquid pharmaceutical composition may comprise an antioxidant.

The liquid pharmaceutical composition preferably comprises a surfactant, preferably a non-ionic surfactant.

The liquid pharmaceutical composition preferably has an ionic strength between 10-200 mM (more preferably between 25-100 mM). The ionic strength ignores any contribution from the dupilumab. Contributors to the ionic strength preferably include one or more of a buffer system (wherever a buffer system is included, though it may be absent), an organic acid salt, a basic amino acid (since it is most preferably in a salt or partial salt form), and an inorganic salt. Most preferably only an organic acid salt and a basic amino acid, and optionally also a buffer system (where present), contribute to the ionic strength of the liquid pharmaceutical composition.

The liquid pharmaceutical composition preferably has an osmolality between 200-400 mOsm/kg (more preferably between 250-350 mOsm/kg). Suitably the liquid pharmaceutical composition is (substantially) isotonic with human blood.

The pharmaceutical composition may comprise an additional tonicifier, for instance, to "top up" the osmolality and/or ionic strength (where said tonicifier is a salt) of the composition.

Preferably, the viscosity of the liquid pharmaceutical composition is less than or equal to 14 mPa.s., more preferably less than or equal to 12 mPa.s.

Preferably, the opalescence of the liquid pharmaceutical composition is less than or equal to 34 NTU, more preferably less than or equal to 28 NTU.

Herein, where a liquid pharmaceutical composition is said to comprise, consist essentially of, or consist of components/ingredients, this preferably means that the liquid pharmaceutical composition is formed by mixing together (in no/any particular order) said components/ingredients, preferably in water (for injection). As such, the defined components/ingredients are suitably defined inputs. A person skilled in the art appreciates that ions of ionic input components/ingredients and ionisable input components/ingredients may dissociate in solution. As such, liquid compositions are more appropriately defined by reference to input components/ingredients.

**Key Pointer:** This section on "PHARMACEUTICAL COMPOSITION", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Dupilumab*

The liquid pharmaceutical composition, according to the invention, comprises dupilumab, preferably in a concentration as further described in this section. Such may include any of the liquid pharmaceutical compositions defined in aspects A1-A186, or any sub-definitions thereof directly and unambiguously derivable from this specification.

Dupilumab is a well-known therapeutic antibody. Dupilumab is described in US 7,608,693, and it may be purified from any originator DUPIXENT® formulation or prepared as a biosimilar.

Dupilumab is suitably present within the liquid pharmaceutical composition at a concentration of 50-300 mg/mL (i.e. 0.340-2.041 mM). Preferably, dupilumab is present within the liquid pharmaceutical composition at a concentration of 90-210 mg/mL (i.e. 0.612-1.43 mM). More preferably, dupilumab is present within the liquid pharmaceutical composition at a concentration of 145-180 mg/mL (i.e. 0.986-1.224 mM). Most preferably dupilumab is present within the liquid pharmaceutical composition at a concentration of 150 mg/mL (1.02 mM) or 175 mg/mL (1.19 mM) - either of these specific dupilumab concentrations may be applied to any of the liquid pharmaceutical compositions defined in aspects A1-A186, or any sub-definitions thereof directly and unambiguously derivable from this specification. As such:

- In a preferred embodiment, the dupilumab is present within the liquid pharmaceutical composition (as defined herein, including any of the liquid pharmaceutical compositions defined in aspects A1-A186, or any sub-definitions thereof directly and unambiguously derivable from this specification) at a concentration of 150 mg/mL (i.e. 1.02 mM); or

- In a preferred embodiment, the dupilumab is present within the liquid pharmaceutical composition (as defined herein, including any of the liquid pharmaceutical compositions defined in aspects A1-A186, or any sub-definitions thereof directly and unambiguously derivable from this specification) at a concentration of 175 mg/mL (i.e. 1.19 mM).

Most preferably, the liquid pharmaceutical composition contains the same excipients and amounts thereof (especially absolute concentrations, be them molar concentrations or weight concentrations), regardless of the concentration of dupilumab, which is most preferably present at a concentration of either 150 mg/mL or 175 mg/mL. Indeed, an advantage of the invention may be that, despite different concentrations of dupilumab, the excipients and concentrations thereof are otherwise the same.

When defining pharmaceutical compositions in terms of molar ratios, preferably dupilumab is defined as 1 and any other components are indexed thereto.

**Key Pointer:** This section on "Dupilumab", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Buffer System (or absence thereof) and pH*

The liquid pharmaceutical composition of the invention (including, where compatible, of any of aspects of A1-A186, or any sub-definitions thereof directly and unambiguously derivable from this specification), may suitably comprise a buffer system.

The buffer system is suitably buffering and tonicifying and is preferably also stabilising (or at least not destabilising). The buffer system suitably also contributes to ionic strength.

The buffer system may comprise one or more buffers, though suitably at most two buffers. Applicable dual buffer systems may include, *inter alia,* phosphate-citrate, histidine-citrate, histidine-phosphate, histidine-succinate, and phosphate-succinate.

Most preferably, however, the buffer system comprises only a single buffer.

Preferably, to be a "buffer" the buffer has a conjugate acid form with a pKa that is within +/-1 of the target (or prevailing) pH - this may be referred to as a "pKa-pH limitation". Preferably, to be a "buffer" the buffer has a conjugate acid form with a pKa that is within +/-1 of some or all of a stipulated target (or prevailing) pH range. Preferably, to be a "buffer" the buffer has a conjugate acid form with a pKa that is within +/-1 of all of a stipulated target (or prevailing) pH range. Preferably the "pH range" mentioned above is pH 5.6-6.2, more preferably pH 5.8-6.0. Examples of buffers complying with such a limitation, at pHs of 5.6-6.2, include:

- A histidine buffer, because histidine has a conjugate acid form (imidazolium histidine) having a pKa of 6.0, which is within +/-1 of all pHs in the range of 5.6-6.2. At such pHs, the histidine buffer is primarily a mixture of free histidine and imidazolium histidine.

- A succinate buffer, because succinate has a conjugate acid form (the monoanionic succinate, as per monosodium succinate) having a pKa (second pKa of succinic acid) of 5.6, which is within +/-1 of all pHs in the range of 5.6-6.2. At such pHs, a succinate buffer is primarily a mixture of monoanionic and dianionic succinate.

- An adipate buffer, because adipate has a conjugate acid form (monoanionic adipate), having a pKa of 5.4.

- A citrate buffer, because citrate has a conjugate acid form (dianionic citrate) having a pKa (third pKa of citric acid) of 6.4, which is within +/-1 of all pHs in the range of 5.6-6.2. Citrate also has a conjugate acid form (monoanionic citrate) having a pKa (second pKa of citric acid) of 4.76, which is within +/-1 of some of the pHs in the lower part of the 5.6-6.2 range.

- An MES (2-(*N*-morpholino)ethanesulfonic acid) buffer, because MES has a conjugate acid form (zwitterionic MES) having a pKa of 6.15.

- A phosphate buffer, because phosphate has a conjugate acid form (monoanionic phosphate, e.g. as per monosodium phosphate) with a pKa of 7.2 - however, this is only within +/-1 of the top of the 5.6-6.2 pH range. Nonetheless, phosphate may still constitute a buffer, even at pH 5.9, in the event that phosphate is specifically stipulated as a/the buffer. Alternatively, phosphate may still be contained within the formulation, but may instead constitute an "additional ingredient" (e.g. a tonicifier, and/or stabiliser).

Even more preferably, to qualify as a buffer said buffer has a pKa that is within +/- 0.5 of the target (or prevailing) pH - such a definition would exclude phosphate buffer, but still encompasses the others listed above. Preferably, to be a "buffer" the buffer has a conjugate acid form with a pKa that is within +/- 0.5 of some or all of a stipulated target (or prevailing) pH range. Preferably, to be a "buffer" the buffer has a conjugate acid form with a pKa that is within +/- 0.5 of all of a stipulated target (or prevailing) pH range.

At a pH of 5.9 (one of the preferred pHs of compositions of the invention), acetate/acetic acid would not constitute a buffer where the definition of a buffer is constrained by one of the aforementioned pKa-pH limitations, because acetic acid has a pKa of 4.76. Instead, at such a pH, acetate may be present as an organic acid salt. However, a skilled person will appreciate that, even at pH 5.9, acetate may still exhibit some (albeit limited) buffering capacity even where it is not defined as a buffer as such.

The liquid pharmaceutical composition may still comprise one or more ingredients that are ostensibly buffers, at certain pHs, but which fall outside the aforementioned preferable pKa-pH limitations - such ingredient(s) may fall into one of the other component categories, such as organic acid salt, basic amino acid, additional ingredient (e.g. additional tonicifier).

The buffer system may suitably comprise one or more (preferably at most two, but most preferably at most one) buffers selected from the group consisting of a succinate buffer, an adipate buffer, a citrate buffer, a maleate buffer, a malate buffer, a phosphate buffer, and a histidine buffer.

Preferably, wherever a buffer system is present, said buffer system only comprises anionic buffers (i.e. is suitably free of cationic buffers). An anionic buffer is a buffer whose core buffering molecule (i.e. the molecule that protonates and/or deprotonates during buffering) ionises to yield an anionic form of said core buffering molecule (i.e. the core buffering molecule deprotonates to form an anion, or further anion in the case of polyprotic buffers) - examples are succinate, adipate, citrate, maleate, malate, and phosphate. A cationic buffer is a buffer whose core buffering molecule ionises to yield a cationic form of said core buffering molecule (i.e. the core buffering molecule protonates to form a cation) - examples include histidine.

Where a buffer system is present, preferably the buffer system comprises one or more (preferably at most two, but most preferably at most one) buffers selected from the group consisting of a succinate buffer, an adipate buffer, a citrate buffer, a maleate buffer, a malate buffer, and a phosphate buffer. Where a buffer system is present, more preferably the buffer system comprises one or more (preferably at most two, but most preferably at most one) buffers selected from the group consisting of a succinate buffer, an adipate buffer, a citrate buffer, a maleate buffer, and a malate buffer. Where a buffer system is present, most preferably the buffer system comprises one or more (preferably at most two, but most preferably at most one) buffers selected from the group consisting of a succinate buffer, a phosphate buffer, or a histidine buffer, most preferably succinate.

Where a buffer system is present, said buffer system suitably consists of a phosphate buffer alone. Where a buffer system is present, said buffer system preferably consists of a succinate buffer alone.

As a skilled person will readily appreciate, a buffer may be formed by mixing together two forms of the same buffer (i.e. as input ingredients) - said two forms still constitute a single buffer component.

As per some of the aspects of the invention detailed above, liquid pharmaceutical compositions of the invention may be free of any buffers, or at least free of any buffer having a conjugate acid whose pKa is within +/-1 (or within +/-0.5) of the target or prevailing pH. This, however, does not exclude other components, defined as being present in the liquid pharmaceutical composition (including dupilumab itself), which may have some buffering capacity. For instance, as a skilled person will readily appreciate, antibodies, especially at high concentrations, may have inherent buffering capacity. This fact can indeed facilitate self-buffering compositions (i.e. without any need for the addition of a buffer system).

Where a buffer system is present, the liquid pharmaceutical composition may comprise 2-50 mM buffer system. Preferably, however, the liquid pharmaceutical composition comprises 3-30 mM buffer system. More preferably the liquid pharmaceutical composition comprises 15-25 mM buffer system. In a preferred embodiment, the liquid pharmaceutical composition comprises 20 mM buffer system. Whilst these molar quantities of buffer system may constitute collective molarities of one or more buffers (preferably selected from those above), preferably such buffer systems comprise only a single buffer, preferably selected from those above, more preferably selected from either succinate buffer or phosphate buffer, most preferably succinate buffer.

Preferably the liquid pharmaceutical composition is free of histidine buffer.

**Key Pointer:** This section on "Buffer System (or absence thereof) and pH", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Organic Acid Salt (or Partial Salt thereof)*

The liquid pharmaceutical composition of the invention (including, where compatible, of any of aspects of A1-A186, or any sub-definitions thereof directly and unambiguously derivable from this specification), suitably comprises an organic acid salt.

The organic acid salt is suitably tonicifying and is preferably also stabilising (or at least not destabilising). The organic acid salt suitably also contributes to ionic strength.

An organic acid salt is a salt of an organic acid. The organic acid salt is preferably a water-soluble salt of an organic acid.

Preferably, the organic acid (or the organic acid salt) has a pKa outside of +/- 1 units of the target (or prevailing) pH. Preferably, the organic acid (of the organic acid salt) has a pKa outside of +/- 1 units of some or all of a target (or prevailing) pH range. Preferably, the organic acid (of the organic acid salt) has a pKa outside of +/-1 units of all of a target (or prevailing) pH range.

Preferably, the organic acid (or the organic acid salt) has a pKa outside of +/- 0.5 units of the target (or prevailing) pH. Preferably, the organic acid (of the organic acid salt) has a pKa outside of +/- 0.5 units of some or all of a target (or prevailing) pH range. Preferably, the organic acid (of the organic acid salt) has a pKa outside of +/- 0.5 units of all of a target (or prevailing) pH range.

Quite reasonably, the "buffer" may be defined more broadly than the organic acid salt. For example, suitably:

- the buffer may have a conjugate acid form with a pKa that is within +/- 1.5 of the target (or prevailing) pH, whereas the organic acid (or the organic acid salt) has a pKa outside of +/- 0.5 units of the target (or prevailing) pH;

- the buffer may have a conjugate acid form with a pKa that is within +/-1 of the target (or prevailing) pH, whereas the organic acid (or the organic acid salt) has a pKa outside of +/- 0.5 units of the target (or prevailing) pH;

- the buffer has a conjugate acid form with a pKa that is within +/- 1 of some or all of a stipulated target (or prevailing) pH range, whereas the organic acid (of the organic acid salt) has a pKa outside of +/-1 units of all of a target (or prevailing) pH range.

- the buffer has a conjugate acid form with a pKa that is within +/- 1 of some or all of a stipulated target (or prevailing) pH range, whereas the organic acid (of the organic acid salt) has a pKa outside of +/- 0.5 units of all of a target (or prevailing) pH range.

Preferably the "pH range" mentioned above is pH 5.6-6.2, more preferably pH 5.8-6.0.

The organic acid salt may be a salt of any organic acid, including but not limited to an organic carboxylic acid, an organic sulphonic acid, an organic phosphoric acid, and even an organic compound with an acidic hydroxy moiety (i.e. acidic alcohol, diol, phenol, and such like, but only where the hydroxy in question has a suitably low pKa value, suitably within 2 units, more preferably within 1 unit of the prevailing or target pH). The organic acid may comprise any number of other functional groups in addition to an acid moiety (e.g. a hydroxyl group in addition to a carboxylic group). Preferably the organic acid salt is a salt of an organic carboxylic acid. Preferably the organic acid salt is a salt of a monoacid (i.e. the organic acid has only a single acidic moiety, where moieties such as hydroxyl are ignored as acid groups if their pKa is much higher than the prevailing or target pH, i.e. the pKa is at least 2, preferably at least 3, units higher than the target or prevailing pH). Most preferably the organic acid salt is a salt of a monocarboxylic acid - i.e. it has only a single carboxylic acid. Preferably the organic acid salt is a water-soluble salt of a monocarboxylic acid. Examples of preferred organic acids are acetic acid (though preferably this is excluded), lactic acid, glu formic acid, ascorbic acid. Most preferably the organic acid salt is a salt of acetic acid or lactic acid, most preferably of lactic acid. However, glutamate and aspartate salts may also be suitable.

The organic acid salt may, for instance, be formed by reacting the organic acid with a base. The base may be an organic base (e.g. arginine, lysine, histidine, imidazole) or an inorganic base (e.g. sodium hydroxide).

The organic acid salt preferably comprises, or consists of, an anionic (deprotonated) form of the (corresponding) organic acid (i.e. organic acid anion) and an associated cation. The associated cation may be an organic cation (e.g. a protonated form of an organic base, an organic base with a basic nitrogen, e.g. an argininium, lysinium, or histidinium) or an inorganic cation (e.g. a metal or ammonium cation, e.g. an alkali metal or alkaline earth metal cation, e.g. $Na^+$). An argininium cation is a protonated (positively charged) ionic form of arginine. A lysinium cation is a protonated (positively charged) ionic form of lysine. A histidinium cation is a protonated (positively charged) ionic form of histidine.

As will be apparent, since liquid pharmaceutical compositions of the invention may contain both an organic acid salt and a basic amino acid, both such components may be delivered by a single compound that is the product of the reaction between the corresponding organic acid and basic amino acid. Examples would include argininium acetate (the arginine salt of acetic acid), lysininium lactate (the lysine salt of lactic acid). Whilst such compounds may constitute both an organic acid salt and a basic amino acid, they may also constitute either an organic acid salt or a basic amino acid for liquid pharmaceutical compositions defined as comprising one of either an organic acid salt or a basic amino acid.

Preferably (especially in terms of input ingredients), the organic acid salt is an inorganic (e.g. metal and/or ammonium) salt or the corresponding organic acid. Most preferably the organic acid salt is a metal salt of the corresponding organic acid, preferably an alkali metal salt of the corresponding organic acid, most preferably a sodium salt of the corresponding organic acid.

Preferably the organic acid salt is a metal carboxylate, most suitably a metal monocarboxylate. Particularly preferred organic acid salts are metal acetate, metal glutamate, and/or metal lactate, more preferably metal lactate or metal glutamate. A metal glutamate is preferably a metal monoglutamate.

Preferably the organic acid salt is glutamate or lactate.

In a most preferred embodiment, the organic acid salt is sodium lactate or sodium glutamate. Sodium lactate is a particularly preferred organic acid salt. Sodium glutamate (preferably sodium monoglutamate) is a particularly preferred organic acid salt.

The organic acid salt may be a full or partial salt (either in terms of input ingredients or resultant composition). By way of example, the input ingredients used to form the organic acid salt component of the liquid pharmaceutical composition of the invention, may comprise a mixture of:

> i. The organic acid and the organic acid salt - e.g. a mixture of acetic acid and sodium acetate, or a mixture of lactic acid and sodium lactate;

> ii. The organic acid and a base - e.g. a mixture of acetic acid and sodium hydroxide (but not enough to completely deprotonate the acetic acid), or a mixture of lactic acid and sodium hydroxide (but not enough to completely deprotonate lactic acid); and/or

> iii. The organic acid salt and an acid - e.g. a mixture of sodium acetate and hydrochloric acid (but not enough to completely protonate acetate), or a mixture or sodium lactate and hydrochloride acid (but not enough to completely protonate lactate).

but any of i-iii, especially example I, still constitutes a single organic acid salt component (unless the relevant base or cationic part of the organic acid salt constitutes another stipulated component, such as a basic amino acid salt). For example, a mixture of lactic acid and sodium lactate still constitutes a single component (organic acid salt, or lactic acid salt) even though it is ostensibly a partial salt (because some will subsist as lactate and some as lactic acid). The two are different forms of the same component.

An organic acid salt may be differentiated from a buffer, within the same liquid pharmaceutical composition, by reference to the pKa of their respective conjugate acids - the buffer will be that which comprises a conjugate acid with a pKa closer to the prevailing (or target) pH than that of the organic acid of the organic acid salt.

Preferably the organic acid salt is a full salt (i.e. -100%, or >90%, preferably >95% salted, most preferably >98% salted).

Where an organic acid salt is present, the liquid pharmaceutical composition may comprise 2-50 mM organic acid salt. Preferably, however, the liquid pharmaceutical composition comprises 5-25 mM organic acid salt. More preferably the liquid pharmaceutical composition comprises 10-22 mM organic acid salt. In a particularly preferred embodiment, the liquid pharmaceutical composition comprises 12.5 mM (or 12-13 mM) organic acid salt. In an alternate embodiment, the liquid pharmaceutical composition comprises 20 mM organic acid salt. These molar quantities of organic acid salt suitably includes all forms thereof - e.g. a partial salt may, for example, exhibit both carboxylic acid and carboxylate salt forms, so the molarities of both are included. Preferably the organic acid salts to which such molar quantities apply are selected from those above, most preferably a lactate salt. Where a buffer system is present, suitably the molar concentration of the organic acid salt is lower than that of the buffer system.

Where lactate is present as the organic acid salt, preferably the composition comprises 5-25 mM lactate (i.e. lactate salt), more preferably 10-22 mM lactate, most preferably 12.5 mM lactate (though there may also be 20 mM lactate).

Where glutamate is present as the organic acid salt, preferably the composition comprises 5-25 mM glutamate (i.e. glutamate salt), more preferably 10-22 mM glutamate, most preferably 20 mM glutamate.

Preferably the liquid pharmaceutical composition is free of acetate.

**Key Pointer:** This section on "Organic Acid Salt (or Partial Salt thereof)", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

## *Non-ionic Polyol*

The liquid pharmaceutical composition of the invention (including, where compatible, of any of aspects of A1-A186, or any sub-definitions thereof directly and unambiguously derivable from this specification), preferably comprises a non-ionic polyol. A non-ionic polyol is a compound that is non-ionic, and suitably non-ionisable (i.e. cannot even be a zwitterion), component comprising a plurality of alcohol moieties.

The non-ionic polyol is generally tonicifying and is preferably also stabilising (or at least not destabilising). The non-ionic polyol suitably does not contribute to ionic strength.

The non-ionic polyol is preferably either a C2-C3 polyol (e.g. propylene glycol, glycerol) or a sugar component (e.g. sugar alcohol or sugar). Most preferably the non-ionic polyol is a sugar component and, indeed, any references herein to a "non-ionic polyol", especially in aspects of A1-A186 and sub-definitions thereof derivable from this specification, may be replaced by the term "sugar component".

The sugar component is suitably either a sugar alcohol or a sugar. Preferably the sugar component is a non-reducing sugar component.

The sugar component is preferably selected from the group consisting of sugar alcohols, monosaccharides, and disaccharides (all of the aforementioned being preferably non-reducing).

Preferred sugar alcohols, for use as the sugar component, are selected from the group consisting of mannitol, sorbitol, erythritol, meso-erythritol, xylitol, arabitol, erythritol, lactitol, maltitol, inositol, threitol, and glycerol. Sorbitol and mannitol are more preferred, with sorbitol being the most preferred sugar alcohol.

Preferred sugars (monosaccharides and disaccharides), for use as the sugar component, are selected from the group consisting of trehalose, sucrose, maltose, lactose, glucose, mannose, galactose, ribose, and xylose. Raffinose (a trisaccharide) is also an option. Trehalose and sucrose are more preferred, with trehalose being the most preferred sugar.

The sugar component (and thus non-ionic polyol) is most preferably trehalose.

Preferably, the liquid pharmaceutical composition comprises at most one sugar component, which is preferably a sugar component selected from above, most preferably trehalose.

The liquid pharmaceutical composition, including any relevant compositions according to aspects A1-A186 (or any sub-definitions thereof directly and unambiguously derivable from this specification), suitably comprises 50-400 mM non-ionic polyol. Preferably, however, the liquid pharmaceutical composition comprises 70-230 mM non-ionic polyol. Preferably, the liquid pharmaceutical composition comprises 90-210 mM non-ionic polyol. The liquid pharmaceutical composition may suitably comprise 125-175 mM non-ionic polyol. Most preferably the liquid pharmaceutical composition comprises 140-150 mM non-ionic polyol (preferably 146 mM thereof). However, a non-ionic polyol concentration of 90-110 mM may also be preferred, especially when a basic amino acid is present at a concentration greater than or equal to 55 mM (most preferably when the basic amino acid is present at a concentration greater than or equal to 70 mM). The non-ionic polyol to which the aforesaid molar concentrations relate is suitably a sugar component, preferably a sugar component selected from those above, most preferably trehalose.

Preferably the liquid pharmaceutical composition is free of sucrose.

Surprisingly, the inventors have found that dupilumab can be effectively formulated without a disaccharide (such as sucrose or trehalose), without any sugar or sugar alcohol, or indeed without any non-ionic polyol. This is particularly the case where two or more, but most preferably all, of a buffer (e.g. succinate or phosphate), an organic acid salt (e.g. lactate or glutamate), and a basic amino acid (e.g. lysine or arginine, suitably in salt form such as hydrochloride salt form) are present, preferably also alongside a surfactant (e.g. PS80 or Px188). This may also be particularly the case where the composition contains a combined concentration of greater than or equal to 80 mM of one or more, most preferably two or more, of a buffer (e.g. succinate or phosphate), an organic acid salt (e.g. lactate or glutamate), and a basic amino acid (e.g. lysine or arginine, suitably in salt form such as hydrochloride salt form), more preferably greater than or equal to 110 mM. As such, the liquid pharmaceutical composition, especially a composition compatibly defined in any of aspects A1-A186 or any embodiments thereof defined in numbered paragraphs B1-B26, C1-C31, D1-D41, E1-E41, and F1-F26 or indeed in other specific embodiments thereof, is suitably (substantially or, more preferably, entirely) free of a disaccharide (such as sucrose or trehalose), more suitably (substantially or, more preferably, entirely) free of any sugars or sugar alcohols, and most preferably (substantially or, more preferably, entirely) free of any non-ionic polyols (suitably as defined herein).

**Key Pointer:** This section on "Non-ionic Polyol", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Basic Amino Acid (Salt or Partial Salt thereof)*

The liquid pharmaceutical composition of the invention (including, where compatible, of any of aspects of A1-A186, or any sub-definitions thereof directly and unambiguously derivable from this specification), suitably comprises a basic amino acid. The definition of "basic amino acid" is explained in the above "DEFINITIONS" section. The "basic amino acid" may also be called "an amino acid with a basic side chain". The basic amino acid may be provided, as an input ingredient, as a salt (e.g. arginine.HCl, lysine.HCl, and the like) or partial salt (e.g. a mixture of arginine and arginine.HCl, or a mixture of lysine and lysine.HCl), or may even be provided as the free (zwitterionic) amino acid (e.g. arginine or lysine), but at prevailing/target pHs such as pH 5.6-6.2 such an amino acid will invariably be in a protonated state in the final liquid pharmaceutical composition.

The basic amino acid is generally tonicifying and is preferably also stabilising (or at least not destabilising). The basic amino acid suitably contributes to ionic strength, not least because a significant amount thereof will generally be protonated at prevailing/target pHs such as pH 5.6-6.2.

The basic amino acid is preferably selected from arginine, lysine, and histidine. In any of these cases, said basic amino acid may be provided (as an input ingredient) as a free base (i.e. free zwitterionic amino acid), salt (e.g hydrochloride salt), or a partial salt (i.e. a mixture of the free base and salt). Examples for each of these three basic amino acids include:

- Arginine may be provided, as an input ingredient, as either: free arginine (Arg), arginine hydrochloride (Arg.HCl), or as an arginine partial salt constituted by a mixture of free arginine and arginine hydrochloride (Arg/Arg.HCl).

- Lysine may be provided, as an input ingredient, as either: free lysine (Lys), lysine hydrochloride (Lys.HCl), or as a lysine partial salt constituted by a mixture of free lysine and lysine hydrochloride (Lys/Lys.HCl).

- Histidine may be provided, as an input ingredient, as either: free histidine (His), histidine hydrochloride (His.HCl), or as a histidine partial salt constituted by a mixture of free histidine and histidine hydrochloride (His/His.HCl).

Of course, acid salts other than hydrochloride salts may be employed.

The basic amino acid is preferably selected from arginine and lysine, but is most preferably lysine. Lysine is preferably provided, as an input ingredient, as lysine hydrochloride, or a mixture of free lysine and lysine hydrochloride.

Where a basic amino acid is present, the liquid pharmaceutical composition, including any relevant compositions according to aspects A1-A186 (or any sub-definitions thereof directly and unambiguously derivable from this specification), suitably comprises 5-200 mM basic amino acid. Preferably, however, the liquid pharmaceutical composition comprises 15-150 mM basic amino acid. The liquid pharmaceutical composition may suitably comprise 20-80 mM basic amino acid. The liquid pharmaceutical composition may suitably comprise 70-130 mM basic amino acid. The liquid pharmaceutical composition may specifically comprise 25 mM, 50 mM, 75 mM, 125 mM, 135 mM, or 150 mM basic amino acid, though more preferably the liquid pharmaceutical composition specifically comprises 50 mM, 75 mM, 125 mM, or 135 mM basic amino acid. These molar concentrations of basic amino acid includes all forms thereof - e.g. a partial salt may, for example, exhibit both free base and salt forms, so the molarities of both are included. Preferably the basic amino acid to which such molar concentrations apply are selected from those above, most preferably lysine.

Preferably, where a basic amino acid is present, the liquid pharmaceutical composition is either free of an inorganic salt (as defined herein) or contains less than or equal to 10 mM (preferably less than or equal to 5 mM, preferably less than or equal to 1 mM) inorganic salt. This limitation excludes any inorganic salts that may happen to form in solution as a result of ion exchanges following mixing of input ingredients.

Preferably the liquid pharmaceutical composition is free of arginine.

**Key Pointer:** This section on "Basic Amino Acid (Salt or Partial Salt thereof)", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

## *Inorganic Salt*

The liquid pharmaceutical composition of the invention (including, where compatible, of any of aspects of A1-A186, or any sub-definitions thereof directly and unambiguously derivable from this specification), may suitably comprise an inorganic salt. The definition of "inorganic salt" is explained in the above "DEFINITIONS" section.

The inorganic salt is suitably tonicifying and is preferably also stabilising (or at least not destabilising). The inorganic salt suitably also contributes to ionic strength. Preferably, the inorganic salt is non-buffering (i.e. imparts substantially no buffering effect), especially at the prevailing pH. Preferably, the inorganic salt is neither acidic nor basic.

The inorganic salt may be a metal salt or non-metal salt.

An example of a suitable non-metal salt is ammonium chloride.

Preferably, however, the inorganic salt is a metal salt (i.e. an inorganic metal salt). Preferably the metal salt comprises one type of metal cation (e.g. a single metal type, preferably at one oxidation state) and one type of inorganic anion (though the anion may comprise more than one type of atom).

The inorganic metal salt is preferably a metal halide, most preferably selected from the group consisting of sodium chloride, potassium chloride, magnesium chloride, and calcium chloride.

Most preferably, where present, the inorganic salt is sodium chloride.

Where an inorganic salt is present, the liquid pharmaceutical composition, including any relevant compositions according to aspects A1-A186 (or any sub-definitions thereof directly and unambiguously derivable from this specification), suitably comprises 5-200 mM inorganic salt. Preferably, however, the liquid pharmaceutical composition comprises 15-150 mM inorganic salt. The liquid pharmaceutical composition may suitably comprise 20-80 mM inorganic salt, more preferably 40-60 mM inorganic salt. The liquid pharmaceutical composition may suitably comprise 70-130 mM inorganic salt, more preferably 125 mM inorganic salt. The liquid pharmaceutical composition may specifically comprise 50 mM or 125 mM inorganic salt, though most preferably the liquid pharmaceutical composition specifically comprises 50 mM inorganic salt. Preferably the inorganic salt to which such molar concentrations apply are selected from those above, most preferably sodium chloride.

Preferably, where an inorganic salt is present, the liquid pharmaceutical composition is either free of a basic amino acid (as defined herein) or contains less than or equal to 10 mM (preferably less than or equal to 5 mM, preferably less than or equal to 1 mM) basic amino acid.

Where an inorganic salt is stipulated as present within the liquid pharmaceutical composition, this refers to said inorganic salt as an input component/ingredient, not to inorganic salts that may happen to form in solution as a result of ion-exchanges or pH adjustments. The inorganic salt is therefore preferably over and above any inorganic salts that may happen to form in solution as result of ion-exchanges or pH adjustments.

**Key Pointer:** This section on "Inorganic Salt", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Non-basic/non-acidic Amino Acid*

The liquid pharmaceutical composition of the invention (including, where compatible, of any of aspects of A1-A186, or any sub-definitions thereof directly and unambiguously derivable from this specification), may suitably comprise a non-basic/non-acidic amino acid. The definition of "non-basic/non-acidic amino acid" is explained in the above "DEFINITIONS" section.

The non-basic/non-acidic amino acid is suitably tonicifying and is preferably also stabilising (or at least not destabilising). The non-basic/non-acidic amino acid suitably does not contribute to ionic strength.

Preferably, the non-basic/non-acidic amino acid is non-buffering (i.e. imparts substantially no buffering effect), especially at the prevailing pH. Preferably, the non-basic/non-acidic amino acid is neither acidic nor basic. As such, preferably the non-basic/non-acidic amino acid is neutral (even if zwitterionic).

The non-basic/non-acidic amino acid is preferably selected from glycine, proline, alanine, valine, leucine, isoleucine, phenylalanine, tryptophan, serine, threonine, glutamine, and asparagine. Methionine and cysteine are also non-basic/non-acidic amino acids but, herein, they are categorised as antioxidants.

Most preferably the non-basic/non-acidic amino acid is selected from glycine or proline, most preferably glycine.

Where a non-basic/non-acidic amino acid is present, the liquid pharmaceutical composition, including any relevant compositions according to aspects A1-A186 (or any sub-definitions thereof directly and unambiguously derivable from this specification), may suitably comprise 5-200 mM non-basic/non-acidic amino acid. Preferably, however, the liquid pharmaceutical composition comprises 15-160 mM non-basic/non-acidic amino acid. The liquid pharmaceutical composition may suitably comprise 20-80 mM non-basic/non-acidic amino acid. The liquid pharmaceutical composition may suitably comprise 70-160 mM non-basic/non-acidic amino acid. The liquid pharmaceutical composition may specifically comprise 25 mM, 50 mM, 75 mM, 125 mM, 135 mM, or 150 mM non-basic/non-acidic amino acid, though more preferably the liquid pharmaceutical composition specifically comprises 150 mM non-basic/non-acidic amino acid. Preferably the non-basic/non-acidic amino acid to which such molar concentrations apply are selected from those above, most preferably glycine.

**Key Pointer:** This section on "Non-basic/non-acidic Amino Acid", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Antioxidant*

The liquid pharmaceutical composition of the invention (including, where compatible, of any of aspects of A1-A186, or any sub-definitions thereof directly and unambiguously derivable from this specification), may suitably comprise an antioxidant. The definition of "antioxidant" is explained in the above "DEFINITIONS" section.

The antioxidant is suitably tonicifying and is preferably also stabilising (or at least not destabilising), preferably stabilising against oxidation of dupilumab. The antioxidant suitably does not contribute to ionic strength.

The antioxidant may be selected from methionine, cysteine, glutathione, ascorbic acid, a metal metabisulphite, a metal thiosulphate, alpha-tocopherol, EDTA, DTPA, or pharmaceutically acceptable salt(s) of any of the aforementioned. Most preferably, the antioxidant is an antioxidant amino acid, preferably selected from methionine, cysteine, or glutathione. Most preferably, the antioxidant is methionine.

Where an antioxidant is present, the liquid pharmaceutical composition may comprise 2-50 mM antioxidant. Preferably, however, the liquid pharmaceutical composition comprises 5-25 mM antioxidant. More preferably, the liquid pharmaceutical composition comprises 15-25 mM antioxidant. In a particular embodiment, the liquid pharmaceutical composition comprises 20 mM antioxidant. Preferably the antioxidant to which such molar concentrations apply are selected from those above, most preferably methionine.

**Key Pointer:** This section on "Antioxidant", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

## *Surfactant*

The liquid pharmaceutical composition of the invention (including, where compatible, of any of aspects of A1-A186, or any sub-definitions thereof directly and unambiguously derivable from this specification), preferably comprises a surfactant.

The surfactant is preferably a non-ionic surfactant. Whilst the surfactant may contribute to osmolality, its contribution is suitably substantially negligible, not least due to the relatively low concentration of said surfactant. The surfactant preferably does not contribute to ionic strength.

Suitably, the surfactant is a surfactant selected from the group consisting of a fatty alcohol, a fatty alcohol ether, a fatty acid ester, a fatty acid amide, a polyoxyalkylene alkyl ether, a polyoxyethylene alkyl ether, a non-ionic block copolymer, alpha-tocopherol, and any combination thereof. Suitably, the surfactant is a surfactant selected from the group consisting of polysorbates, spans, poloxamers, kolliphors, and any combination thereof. Suitably, the surfactant is a surfactant selected from the group consisting of a sorbitan ester (e.g. Span), an ethoxylated sorbitan ester (e.g. polysorbate), and any combination thereof. Suitably, the surfactant is a surfactant selected from the group consisting of sorbitan monolaurate, sorbitan monostearate, sorbitan tristearate, and any combination thereof.

Suitably, the surfactant is a polysorbate surfactant. Suitably, the surfactant is a surfactant selected from the group consisting of polysorbate 20, polysorbate 40, polysorbate 60, polysorbate 80, and any combination thereof.

Suitably, the surfactant is a surfactant selected from the group consisting of glycerol monostearate, glycerol monolaurate, polyoxylglycerides (e.g. lauroyl polyoxylglycerides), and any combination thereof.

Suitably, the surfactant is a surfactant selected from Macrogol 15 hydroxystearate, macrogol cetostearyl ether, macrogol stearyl ether, polyoxyethylene castor oil derivatives, polyoxyethylene stearates, and any combination thereof.

Suitably, the surfactant is a surfactant that is or comprises a block alkoxylate. Suitably, the surfactant is a surfactant selected from the group consisting of poloxamers, poloxamer 182, poloxamer 188 (Pluronic F68), poloxamer 407 (Pluronic F127), Synperonics, Kolliphors, and any combination thereof. Suitably, the surfactant is a poloxamer. Suitably, the surfactant is Poloxamer 188 (e.g. Pluronic F68). Suitably, the surfactant is Poloxamer 407 (e.g. Pluronic F127).

Suitably, the surfactant is kolliphor hs-15.

Suitably, the surfactant is a surfactant selected from the group consisting of polysorbate 20, polysorbate 80, poloxamer 182, poloxamer 188, poloxamer 407, kolliphor hs-15, and any combination thereof.

The surfactant is most preferably selected from a polysorbate, a poloxamer, or a kolliphor. More preferably the surfactant is a polysorbate or a poloxamer, such as PS20, PS80, or Px188. More preferably still, the surfactant is a polysorbate, preferably a polysorbate selected from polysorbate 20 and polysorbate 80 (preferably not a combination thereof).

Most preferably, the surfactant is polysorbate 80 (PS80).

Where the composition is free of at least one of histidine, acetate, sucrose, and arginine, the composition preferably comprises a surfactant that is either a polysorbate or a poloxamer, most preferably PS80 or Px188. In such scenarios, the surfactant is advantageously poloxamer 188, though it may be polysorbate 80.

Where the composition is free of at least two of (more preferably free of at least three of, and most preferably free of all of) histidine, acetate, sucrose, and arginine, the composition preferably comprises a surfactant that is either a polysorbate or a poloxamer, most preferably PS80 or Px188. In such scenarios, the surfactant is advantageously poloxamer 188, though it may still be polysorbate 80.

Where the composition does contain two or more of (and especially where it contains three or more of) histidine, acetate, sucrose, and arginine, the composition is preferably free of any poloxamer surfactants (especially poloxamer 188) and free of any surfactants comprising a polyethylene glycol (especially PEG3350).

The liquid pharmaceutical composition suitably comprises 0.01-5 mg/mL surfactant. Preferably, however, the liquid pharmaceutical composition comprises 0.1-3 mg/mL surfactant. More preferably the liquid pharmaceutical composition comprises 0.5-2.5 mg/mL surfactant. Most preferably, the liquid pharmaceutical composition comprises 2 mg/mL (i.e. 0.2 wt%) surfactant. Preferably the surfactant to which such molar concentrations apply are selected from those above, preferably PS80 or Px188, most preferably polysorbate 80.

**Key Pointer:** This section on "Surfactant", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Diluents*

The liquid pharmaceutical composition preferably comprises a diluent. The diluent suitably constitutes the balance of ingredients in the liquid pharmaceutical composition so that the weight percentages of all ingredients/components total 100 wt%. Preferably, the diluent is present wherever the composition is said to consist of specified ingredients/components. Preferably, the diluent dissolves all ingredients/components of the composition. The diluent is preferably an aqueous diluent. Most preferably, the diluent is water (e.g. water for injection, WFI).

**Key Pointer:** This section on "Diluents", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Additional Ingredients*

The liquid pharmaceutical composition of the invention (including, where compatible, of any of aspects of A1-A186, or any sub-definitions thereof directly and unambiguously derivable from this specification), may comprise one or more additional ingredients above and beyond those stipulated above (or stipulated in aspects of A1-A186, or any sub-definitions thereof directly and unambiguously derivable from this specification). Additional ingredients may, for instance, including additional tonicifier(s), preservative(s), and/or additional stabiliser(s).

### *Additional Tonicifier (or Tonicity Agent)*

The liquid pharmaceutical composition may comprise an additional tonicifier (or additional tonicity agent, or additional tonicity modifier, all of which have the same meaning), suitably in addition to any other stipulated ingredients/components, where such other stipulated ingredients/components may also contribute to osmolality and/or tonicity.

The additional tonicifier contributes to (or increases) overall osmolality and osmolarity. The additional tonicifier suitably contributes at least 25 mOsmol/kg to the overall osmolality of the composition, preferably at least 40 mOsmol/kg. The additional tonicifier suitably contributes at most 200 mOsmol/kg to the overall osmolality of the composition, preferably at most 150 mOsmol/kg. As such, the additional tonicifier suitably contributes 25-200 mOsmol/kg to overall osmolality, preferably 40-150 mOsmol/kg.

Whilst the additional tonicifier may be non-ionic, it may suitably contribute to the overall ionic strength of the composition.

The additional tonicifier is preferably a single tonicifier though, depending on the nature of the tonicifier, this may include more than one form of the same compound/component.

Suitably, the additional tonicifier is selected from the group consisting of a metal salt tonicifier (e.g. NaCl), a non-metal salt tonicifier (e.g. NH$_4$Cl, histidine hydrochloride), a polyol tonicifier (e.g. a sugar or sugar alcohol, including any of those sugars or sugar alcohols defined herein in relation to a sugar component), and an amino acid tonicifier (e.g. glycine, proline, arginine, lysine, histidine, aspartic acid, glutamic acid).

Where the additional tonicifier is an amino acid tonicifier, it is suitably selected from the group consisting of arginine, glycine, histidine, lysine, aspartic acid, glutamic acid, and any combination thereof. Some amino acid tonicifiers may still be a single tonicifier and yet exist or be provided in more than one form thereof- histidine and histidine hydrochloride is one such example. The additional tonicifier may be histidine, and this would include all forms (e.g. zwitterionic and imidazolium forms) thereof.

Where the additional tonicifier is a polyol tonicifier, it is suitably selected from the group consisting of mannitol, sorbitol, glycerol, maltose, glucose, lactose, and any combination thereof.

Where the additional tonicifier is a non-metal salt tonicifier, it is suitably selected from the group consisting of ammonium chloride, ammonium formate, ammonium acetate, histidine hydrochloride, and any combination thereof.

Where the additional tonicifier is a metal salt tonicifier, it is suitably selected from the group consisting of sodium chloride, potassium chloride, magnesium chloride, calcium chloride, sodium acetate, sodium citrate, monosodium glutamate, sodium phosphate, potassium phosphate, and any combination thereof.

Whilst the additional tonicifier may be (or contribute to) buffering at the prevailing pH (e.g. histidine, metal phosphate, metal acetate), preferably the additional tonicifier is a non-buffering tonicifier at the prevailing pH (i.e. it imparts little to no buffering effect at the prevailing pH).

Most preferably, where an additional tonicifier is present, the additional tonicifier is sodium chloride.

Suitably, the additional tonicifier is present within the composition at a concentration of 5-300 mM tonicifier. Preferably, the additional tonicifier is present within the composition at a concentration of 10-200 mM tonicifier. More preferably, the additional tonicifier is present within the composition at a concentration of 25-150 mM tonicifier. Most preferably, the additional tonicifier is present within the composition at a concentration of 40-110 mM tonicifier.

Preferably, there is no additional tonicifier present within the composition where either or both organic acid salt and/or basic amino acid are present. Preferably there is no additional tonicifier beyond the other stipulated ingredients/-components.

**Key Pointer:** This section on "Additional Ingredients", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Absent Ingredients*

The liquid pharmaceutical composition, of the invention (including, where compatible, of any of aspects of A1-A186, or any sub-definitions thereof directly and unambiguously derivable from this specification), may be characterised by an absence of one or more ingredients/components.

Preferably the liquid pharmaceutical composition is free of histidine, acetate, sucrose, and/or arginine.

Preferably the liquid pharmaceutical composition is free of histidine.

Preferably the liquid pharmaceutical composition is free of acetate.

Preferably the liquid pharmaceutical composition is free of sucrose.

Preferably the liquid pharmaceutical composition is free of arginine.

More preferably, the liquid pharmaceutical composition is free of two or more of histidine, acetate, sucrose, and arginine.

Even more preferably, the liquid pharmaceutical composition is free of three or more of histidine, acetate, sucrose, and arginine.

Most preferably, the liquid pharmaceutical composition is free of all of histidine, acetate, and sucrose, and is suitably further free of arginine.

**Key Pointer:** This section on "Absent Ingredients", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Ionic Strength and Osmolality*

The liquid pharmaceutical composition, of the invention (including, where compatible, of any of aspects of A1-A186, or any sub-definitions thereof directly and unambiguously derivable from this specification), may be characterised by ionic strength, the definition for which is set forth in the "DEFINITIONS" section above. The ionic strength is preferably a theoretical (or calculated) ionic strength that ignores any contribution from the dupilumab.

The liquid pharmaceutical composition preferably has an ionic strength between 10-200 mM (more preferably between 25-100 mM, or between 50-140 mM). Contributors to the ionic strength preferably include one or more of a buffer system (wherever a buffer system is included, though it may be absent), an organic acid salt, a basic amino acid (since it is most preferably in a salt or partial salt form), an inorganic salt, an additional tonicifier. Most preferably only an organic acid salt and a basic amino acid, and optionally also a buffer system (where present), contribute to the ionic strength of the liquid pharmaceutical composition.

The liquid pharmaceutical composition, of the invention (including, where compatible, of any of aspects of A1-A186, or any sub-definitions thereof directly and unambiguously derivable from this specification), may be characterised by osmolality, the definition for which is set forth in the "DEFINITIONS" section above. Osmolality may be measured (or measurable), for instance, by freezing-point depression methods. However, preferably, the osmolality is a theoretical (or calculated) osmolality that ignores any contribution from the dupilumab.

The liquid pharmaceutical composition preferably has an osmolality between 200-400 mOsm/kg (more preferably between 250-350 mOsm/kg). Suitably the liquid pharmaceutical composition is (substantially) isotonic with human blood.

**Key Pointer:** This section on "Ionic Strength and Osmolality", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

### *Viscosity and Turbidity*

The liquid pharmaceutical composition, of the invention (including, where compatible, of any of aspects of A1-A186, or any sub-definitions thereof directly and unambiguously derivable from this specification), may be characterised by viscosity and/or turbidity (or opalescence), the definition for which are set forth in the "DEFINITIONS" section above.

The liquid pharmaceutical composition of the invention may suitably have a viscosity that is the same or less than that of the originator formulations, especially for comparable dupilumab concentrations.

Preferably, the viscosity of the liquid pharmaceutical composition is less than or equal to 14 mPa.s., more preferably less than or equal to 12 mPa.s, most preferably less than or equal to 10 mPa.s.

Preferably, the opalescence of the liquid pharmaceutical composition is less than or equal to 34 NTU, more preferably less than or equal to 28 NTU, most preferably less than 24 NTU.

**Key Pointer:** This section on "Viscosity and Turbidity", or any part thereof, may be applied to any of aspects A1-A186 or any embodiments thereof defined in numbered paragraphs B1-B26, C1-C31, D1-D41, E1-E41, and F1-F26 or indeed in other specific embodiments thereof, preferably in combination with any, some, or all Key Pointers set forth elsewhere herein.

*Particular Embodiments*

In a particular embodiment, the liquid pharmaceutical composition comprises or consists of:

- dupilumab;

- a surfactant (preferably selected from polysorbate 80 or poloxamer 188);

- water (for injection); and

two to five (preferably two to four) of any of:

- a buffer (preferably selected from a succinate buffer, an MES buffer, or a phosphate buffer, and possibly, though preferably not, a histidine buffer, though most preferably a succinate buffer if a buffer is present, though the composition may be free of buffers);

- an organic acid salt (preferably selected from a lactate salt or a glutamate salt, and possibly, though preferably not, an acetate salt, and most preferably a lactate salt);

- a sugar component (preferably selected from trehalose or sucrose, though most preferably not sucrose);

- a basic amino acid (preferably selected from lysine or arginine, though most preferably not arginine); and

- an inorganic salt (preferably, where present, sodium chloride);

on the proviso that at least one of histidine, acetate, sucrose, and arginine is absent from the composition;

wherein the composition has a pH of 5.6-6.2 and is optionally further characterised by one or more (preferably by two or more, more preferably all) of:

- an ionic strength of 20-200 mM;
- an osmolality of 200-400 mOsmol/kg;
- a viscosity of less than or equal to 18 mPa.s; and
- a turbidity of less than or equal to 34 NTU.

In a particular embodiment, the liquid pharmaceutical composition comprises or consists of:

- 90-210 mg/mL dupilumab;
- 0.1-3 mg/mL surfactant (preferably selected from polysorbate 80 or poloxamer 188);
- water (for injection); and

two to five (preferably two to four, more preferably three or four) of any of:

- 3-30 mM buffer (preferably selected from a succinate buffer, an MES buffer, or a phosphate buffer, and possibly, though preferably not, a histidine buffer, though most preferably a succinate buffer if a buffer is present, though the composition may be free of buffers);
- 5-25 mM organic acid salt (preferably selected from a lactate salt or a glutamate salt, and possibly, though preferably not, an acetate salt, and most preferably a lactate salt);
- 100-300 mM sugar component (preferably selected from trehalose or sucrose, though most preferably not sucrose);
- 15-150 mM basic amino acid (preferably selected from lysine or arginine, though most preferably not arginine); and
- 15-150 mM inorganic salt (preferably, where present, sodium chloride);

on the proviso that at least one of histidine, acetate, sucrose, and arginine is absent from the composition;
wherein the composition has a pH of 5.6-6.2 and is further characterised by one or more (preferably by two or more) of:

- an ionic strength of 50-200 mM;
- an osmolality of 250-350 mOsmol/kg;
- a viscosity of less than or equal to 14 mPa.s; and
- a turbidity of less than or equal to 28 NTU.

In a particular embodiment, the liquid pharmaceutical composition comprises or consists of:

- 140-180 mg/mL dupilumab;
- 1-2.5 mg/mL surfactant selected from polysorbate 80 or poloxamer 188;
- water (for injection); and

two to five (preferably two to four, more preferably three or four) of any of:

- 5-25 mM buffer selected from a succinate buffer, an MES buffer, a phosphate buffer, or a histidine buffer;
- 10-25 mM organic acid salt selected from a lactate salt, a glutamate salt, or an acetate salt;
- 130-160 mM sugar component selected from trehalose or sucrose;
- 15-150 mM basic amino acid selected from lysine or arginine; and
- 20-130 mM inorganic salt selected from sodium chloride or potassium chloride;

on the proviso that at least one of histidine, acetate, sucrose, and arginine is absent from the composition;
wherein the composition has a pH of 5.6-6.2 and is further characterised by:

- an ionic strength of 50-200 mM;
- an osmolality of 250-350 mOsmol/kg;
- a viscosity of less than or equal to 14 mPa.s; and
- a turbidity of less than or equal to 28 NTU.

The following particular embodiments pertain to a liquid pharmaceutical composition comprising or consisting of: dupilumab (preferably 90-210 mg/mL dupilumab; most preferably 140-180 mg/mL dupilumab), a surfactant (preferably 0.1-3 mg/mL surfactant, polysorbate 80, or 1-2.5 mg/mL polysorbate 80); water; and two or more of:

- a buffer (preferably 3-30 mM buffer, a succinate buffer, 5-25 mM succinate buffer, a MES buffer, 5-25 mM MES buffer, a phosphate buffer, 5-25 mM phosphate buffer, a histidine buffer, or 5-25 mM histidine buffer);
- an organic acid salt (preferably 5-25 mM organic acid salt, a lactate salt, 10-25 mM lactate salt, a glutamate salt, 10-25 mM glutamate salt, an acetate salt, or 10-25 mM acetate salt);
- a sugar component (preferably 100-300 mM sugar component, trehalose, 100-200 mM trehalose, sucrose, or 100-200 mM sucrose);
- a basic amino acid (preferably 15-150 mM basic amino acid, lysine, 15-150 mM lysine, arginine, or 15-150 mM arginine);
- an inorganic salt (preferably 15-150 mM inorganic salt, sodium chloride, or 15-150 mM sodium chloride);

on the proviso that at least one (preferably at least two) of histidine, acetate, sucrose, and arginine is absent from the composition; wherein the composition has a pH of 5.6-6.2;
wherein the composition is optionally further characterised by one or more (preferably by two or more, most preferably all) of: an ionic strength of 50-200 mM, an osmolality of 250-350 mOsmol/kg, a viscosity of less than or equal to 14 mPa.s, and a turbidity of less than or equal to 28 NTU.

*Embodiments with Generic Components*

B) The following numbered paragraphs, B1-B26, relate to particular embodiments of a liquid pharmaceutical composition, all of which have a pH of 5.6-6.2, all of which are free of at least one (preferably at least two) of histidine, acetate, sucrose, and arginine, and all of which are optionally further characterised by one or more (preferably by two or more, most preferably all) of an ionic strength of 50-200 mM, an osmolality of 250-350 mOsmol/kg, a viscosity of less than or equal to 14 mPa.s, and a turbidity of less than or equal to 28 NTU; wherein each composition comprises or consists of: dupilumab; a surfactant; water; and also:

B1. a buffer; and an organic acid salt.

B2. a buffer; and a sugar component.

B3. a buffer; and a basic amino acid.

B4. a buffer; and an inorganic salt.

B5. an organic acid salt; and a sugar component.

B6. an organic acid salt; and a basic amino acid.

B7. an organic acid salt; and an inorganic salt.

B8. a sugar component; and a basic amino acid.

B9. a sugar component; and an inorganic salt.

B10. a basic amino acid; and an inorganic salt.

B11. a buffer; an organic acid salt; and a sugar component.

B12. a buffer; an organic acid salt; and a basic amino acid.

B13. a buffer; an organic acid salt; and an inorganic salt.

B14. a buffer; a sugar component; and a basic amino acid.

B15. a buffer; a sugar component; and an inorganic salt.

B16. a buffer; a basic amino acid; and an inorganic salt.

B17. an organic acid salt; a sugar component; and a basic amino acid.

B18. an organic acid salt; a sugar component; and an inorganic salt.

B19. an organic acid salt; a basic amino acid; and an inorganic salt.

B20. a sugar component; a basic amino acid; and an inorganic salt.

B21. a buffer; an organic acid salt; a sugar component; and a basic amino acid.

B22. a buffer; an organic acid salt; a sugar component; and an inorganic salt.

B23. a buffer; an organic acid salt; a basic amino acid; and an inorganic salt.

B24. a buffer; a sugar component; a basic amino acid; and an inorganic salt.

B25. an organic acid salt; a sugar component; a basic amino acid; and an inorganic salt.

B26. a buffer; an organic acid salt; a sugar component; a basic amino acid; and an inorganic salt.

*Embodiments with Generic Components and Concentration Ranges*

C) The following numbered paragraphs, C1-C31, relate to particular embodiments of a liquid pharmaceutical composition, all of which have a pH of 5.6-6.2, all of which are free of at least one (preferably at least two) of histidine, acetate, sucrose, and arginine, and all of which are optionally further characterised by one or more (preferably by two or more, most preferably all) of an ionic strength of 50-200 mM, an osmolality of 250-350 mOsmol/kg, a viscosity of less than or equal to 14 mPa.s, and a turbidity of less than or equal to 28 NTU; wherein each composition comprises or consists of: 90-210 mg/mL dupilumab; 0.1-3 mg/mL surfactant; water; and also:

C1. 3-30 mM buffer.

C2. 5-25 mM organic acid salt.

C3. 50-300 mM sugar component.

C4. 15-150 mM basic amino acid.

C5. 15-150 mM inorganic salt.

C6. 3-30 mM buffer; and 5-25 mM organic acid salt.

C7. 3-30 mM buffer; and 50-300 mM sugar component.

C8. 3-30 mM buffer; and 15-150 mM basic amino acid.

C9. 3-30 mM buffer; and 15-150 mM inorganic salt.

C10. 5-25 mM organic acid salt; and 50-300 mM sugar component.

C11. 5-25 mM organic acid salt; and 15-150 mM basic amino acid.

C12. 5-25 mM organic acid salt; and 15-150 mM inorganic salt.

C13. 50-300 mM sugar component; and 15-150 mM basic amino acid.

C14. 50-300 mM sugar component; and 15-150 mM inorganic salt.

C15. 15-150 mM basic amino acid; and 15-150 mM inorganic salt.

C16. 3-30 mM buffer; 5-25 mM organic acid salt; and 50-300 mM sugar component.

C17. 3-30 mM buffer; 5-25 mM organic acid salt; and 15-150 mM basic amino acid.

C18. 3-30 mM buffer; 5-25 mM organic acid salt; and 15-150 mM inorganic salt.

C19. 3-30 mM buffer; 50-300 mM sugar component; and 15-150 mM basic amino acid.

C20. 3-30 mM buffer; 50-300 mM sugar component; and 15-150 mM inorganic salt.

C21. 3-30 mM buffer; 15-150 mM basic amino acid; and 15-150 mM inorganic salt.

C22. 5-25 mM organic acid salt; 50-300 mM sugar component; and 15-150 mM basic amino acid.

C23. 5-25 mM organic acid salt; 50-300 mM sugar component; and 15-150 mM inorganic salt.

C24. 5-25 mM organic acid salt; 15-150 mM basic amino acid; and 15-150 mM inorganic salt.

C25. 50-300 mM sugar component; 15-150 mM basic amino acid; and 15-150 mM inorganic salt.

C26. 3-30 mM buffer; 5-25 mM organic acid salt; 50-300 mM sugar component; and 15-150 mM basic amino acid.

C27. 3-30 mM buffer; 5-25 mM organic acid salt; 50-300 mM sugar component; and 15-150 mM inorganic salt.

C28. 3-30 mM buffer; 5-25 mM organic acid salt; 15-150 mM basic amino acid; and 15-150 mM inorganic salt.

C29. 3-30 mM buffer; 50-300 mM sugar component; 15-150 mM basic amino acid; and 15-150 mM inorganic salt.

C30. 5-25 mM organic acid salt; 50-300 mM sugar component; 15-150 mM basic amino acid; and 15-150 mM inorganic salt.

C31. 3-30 mM buffer; 5-25 mM organic acid salt; 50-300 mM sugar component; 15-150 mM basic amino acid; and 15-150 mM inorganic salt.

_Embodiments with Specific Components And Concentration Ranges_

D) The following numbered paragraphs, D1-D41, relate to particular embodiments of a liquid pharmaceutical composition, all of which have a pH of 5.6-6.2, all of which are free of at least one (preferably at least two) of histidine, acetate, sucrose, and arginine, and all of which are optionally further characterised by one or more (preferably by two or more, most preferably all) of an ionic strength of 50-200 mM, an osmolality of 250-350 mOsmol/kg, a viscosity of less than or equal to 14 mPa.s, and a turbidity of less than or equal to 28 NTU; wherein each composition comprises or, more preferably, consists of: 90-210 mg/mL dupilumab; 0.5-2.5 mg/mL surfactant (preferably a polysorbate surfactant, most preferably polysorbate 80); water; and also:

D1. 5-25 mM succinate buffer; and 10-25 mM lactate salt.

D2. 5-25 mM phosphate buffer; and 10-25 mM lactate salt.

D3. 5-25 mM succinate buffer; and 70-230 mM trehalose.

D4. 5-25 mM phosphate buffer; and 70-230 mM trehalose.

D5. 5-25 mM succinate buffer; and 15-150 mM lysine or arginine (preferably lysine).

D6. 5-25 mM phosphate buffer; and 15-150 mM lysine or arginine (preferably lysine).

D7. 5-25 mM succinate buffer; and 15-150 mM sodium chloride.

D8. 5-25 mM phosphate buffer; and 15-150 mM sodium chloride.

D9. 10-25 mM lactate salt; and 70-230 mM trehalose.

D10. 10-25 mM lactate salt; and 15-150 mM lysine or arginine (preferably lysine).

D11. 10-25 mM lactate salt; and 15-150 mM sodium chloride.

D12. 70-230 mM trehalose; and 15-150 mM lysine or arginine (preferably lysine).

D13. 70-230 mM trehalose; and 15-150 mM sodium chloride.

D14. 15-150 mM lysine or arginine (preferably lysine); and 15-150 mM sodium chloride.

D15. 5-25 mM succinate buffer; 10-25 mM lactate salt; and 70-230 mM trehalose.

D16. 5-25 mM phosphate buffer; 10-25 mM lactate salt; and 70-230 mM trehalose.

D17. 5-25 mM succinate buffer; 10-25 mM lactate salt; and 15-150 mM lysine or arginine (preferably lysine).

D18. 5-25 mM phosphate buffer; 10-25 mM lactate salt; and 15-150 mM lysine or arginine (preferably lysine).

D19. 5-25 mM succinate buffer; 10-25 mM lactate salt; and 15-150 mM sodium chloride.

D20. 5-25 mM phosphate buffer; 10-25 mM lactate salt; and 15-150 mM sodium chloride.

D21. 5-25 mM succinate buffer; 70-230 mM trehalose; and 15-150 mM lysine or arginine (preferably lysine).

D22. 5-25 mM phosphate buffer; 70-230 mM trehalose; and 15-150 mM lysine or arginine (preferably lysine).

D23. 5-25 mM succinate buffer; 70-230 mM trehalose; and 15-150 mM sodium chloride.

D24. 5-25 mM phosphate buffer; 70-230 mM trehalose; and 15-150 mM sodium chloride.

D25. 5-25 mM succinate buffer; 15-150 mM lysine or arginine (preferably lysine); and 15-150 mM sodium chloride.

D26. 5-25 mM phosphate buffer; 15-150 mM lysine or arginine (preferably lysine); and 15-150 mM sodium chloride.

D27. 10-25 mM lactate salt; 70-230 mM trehalose; and 15-150 mM lysine or arginine (preferably lysine).

D28. 10-25 mM lactate salt; 70-230 mM trehalose; and 15-150 mM sodium chloride.

D29. 10-25 mM lactate salt; 15-150 mM lysine or arginine (preferably lysine); and 15-150 mM sodium chloride.

D30. 70-230 mM trehalose; 15-150 mM lysine or arginine (preferably lysine); and 15-150 mM sodium chloride.

D31. 5-25 mM succinate buffer; 10-25 mM lactate salt; 70-230 mM trehalose; and 15-150 mM lysine or arginine (preferably lysine).

D32. 5-25 mM phosphate buffer; 10-25 mM lactate salt; 70-230 mM trehalose; and 15-150 mM lysine or arginine (preferably lysine).

D33. 5-25 mM succinate buffer; 10-25 mM lactate salt; 70-230 mM trehalose; and 15-150 mM sodium chloride.

D34. 5-25 mM phosphate buffer; 10-25 mM lactate salt; 70-230 mM trehalose; and 15-150 mM sodium chloride.

D35. 5-25 mM succinate buffer; 10-25 mM lactate salt; 15-150 mM lysine or arginine (preferably lysine); and 15-150 mM sodium chloride.

D36. 5-25 mM phosphate buffer; 10-25 mM lactate salt; 15-150 mM lysine or arginine (preferably lysine); and 15-150 mM sodium chloride.

D37. 5-25 mM succinate buffer; 70-230 mM trehalose; 15-150 mM lysine or arginine (preferably lysine); and 15-150 mM sodium chloride.

D38. 5-25 mM phosphate buffer; 70-230 mM trehalose; 15-150 mM lysine or arginine (preferably lysine); and 15-150 mM sodium chloride.

D39. 10-25 mM lactate salt; 70-230 mM trehalose; 15-150 mM lysine or arginine (preferably lysine); and 15-150 mM sodium chloride.

D40. 5-25 mM succinate buffer; 10-25 mM lactate salt; 70-230 mM trehalose; 15-150 mM lysine or arginine (preferably lysine); and 15-150 mM sodium chloride.

D41. 5-25 mM phosphate buffer; 10-25 mM lactate salt; 70-230 mM trehalose; 15-150 mM lysine or arginine (preferably lysine); and 15-150 mM sodium chloride.

*Embodiments with Specific Components And Specific Concentrations*

E) The following numbered paragraphs, E1-E41, relate to particular embodiments of a liquid pharmaceutical composition, all of which have a pH of 5.6-6.2, all of which are free of at least one (preferably at least two) of histidine, acetate, sucrose, and arginine, and all of which are optionally further characterised by one or more (preferably by two or more, most preferably all) of an ionic strength of 50-200 mM, an osmolality of 250-350 mOsmol/kg, a viscosity of less than or equal to 14 mPa.s, and a turbidity of less than or equal to 28 NTU; wherein each composition comprises or, more preferably, consists of: 150 mg/mL or 175 mg/mL dupilumab; 2 mg/mL polysorbate 80 or poloxamer 188 (preferably polysorbate 80); water; and also:

E1. 20 mM buffer selected from a succinate, MES, phosphate, or histidine buffer (preferably 20 mM succinate buffer); and 12.5 mM organic salt selected from a lactate or acetate salt (most preferably 20 mM lactate).

E2. 20 mM buffer selected from a succinate, MES, phosphate, or histidine buffer (preferably 20 mM succinate buffer); and 20 mM glutamate salt.

E3. 20 mM buffer selected from a succinate, MES, phosphate, or histidine buffer (preferably 20 mM succinate buffer); and 146 mM (though optionally 100 mM) sugar component selected from trehalose or sucrose (most preferably trehalose).

E4. 20 mM buffer selected from a succinate, MES, phosphate, or histidine buffer (preferably 20 mM succinate buffer); and a basic amino acid selected from lysine or arginine (most preferably lysine) at a concentration of 50 mM, 75 mM, 125 mM, or 135 mM.

E5. 20 mM buffer selected from a succinate, MES, phosphate, or histidine buffer (preferably 20 mM succinate buffer); and 125 mM sodium chloride.

E6. 12.5 mM organic salt selected from a lactate or acetate salt (most preferably 20 mM lactate); and 146 mM (though optionally 100 mM) sugar component selected from trehalose or sucrose (most preferably trehalose).

E7. 20 mM glutamate salt; and 146 mM (though optionally 100 mM) sugar component selected from trehalose or sucrose (most preferably trehalose).

E8. 12.5 mM organic salt selected from a lactate or acetate salt (most preferably 20 mM lactate); and a basic amino acid selected from lysine or arginine (most preferably lysine) at a concentration of 50 mM, 75 mM, 125 mM, or 135 mM.

E9. 20 mM glutamate salt; and a basic amino acid selected from lysine or arginine (most preferably lysine) at a concentration of 50 mM, 75 mM, 125 mM, or 135 mM.

E10. 12.5 mM organic salt selected from a lactate or acetate salt (most preferably 20 mM lactate); and 125 mM sodium chloride.

E11. 20 mM glutamate salt; and 125 mM sodium chloride.

E12. 146 mM (though optionally 100 mM) sugar component selected from trehalose or sucrose (most preferably trehalose); and a basic amino acid selected from lysine or arginine (most preferably lysine) at a concentration of 50 mM, 75 mM, 125 mM, or 135 mM.

E13. 146 mM (though optionally 100 mM) sugar component selected from trehalose or sucrose (most preferably trehalose); and 125 mM sodium chloride.

E14. a basic amino acid selected from lysine or arginine (most preferably lysine) at a concentration of 50 mM, 75 mM, 125 mM, or 135 mM; and 125 mM sodium chloride.

E15. 20 mM buffer selected from a succinate, MES, phosphate, or histidine buffer (preferably 20 mM succinate buffer); 12.5 mM organic salt selected from a lactate or acetate salt (most preferably 20 mM lactate); and 146 mM (though optionally 100 mM) sugar component selected from trehalose or sucrose (most preferably trehalose).

E16. 20 mM buffer selected from a succinate, MES, phosphate, or histidine buffer (preferably 20 mM succinate buffer); 20 mM glutamate salt; and 146 mM (though optionally 100 mM) sugar component selected from trehalose or sucrose (most preferably trehalose).

E17. 20 mM buffer selected from a succinate, MES, phosphate, or histidine buffer (preferably 20 mM succinate buffer); 12.5 mM organic salt selected from a lactate or acetate salt (most preferably 20 mM lactate); and a basic amino acid selected from lysine or arginine (most preferably lysine) at a concentration of 50 mM, 75 mM, 125 mM, or 135 mM.

E18. 20 mM buffer selected from a succinate, MES, phosphate, or histidine buffer (preferably 20 mM succinate buffer); 20 mM glutamate salt; and a basic amino acid selected from lysine or arginine (most preferably lysine) at a concentration of 50 mM, 75 mM, 125 mM, or 135 mM.

E19. 20 mM buffer selected from a succinate, MES, phosphate, or histidine buffer (preferably 20 mM succinate buffer); 12.5 mM organic salt selected from a lactate or acetate salt (most preferably 20 mM lactate); and 125 mM sodium chloride.

E20. 20 mM buffer selected from a succinate, MES, phosphate, or histidine buffer (preferably 20 mM succinate buffer); 20 mM glutamate salt; and 125 mM sodium chloride.

E21. 20 mM buffer selected from a succinate, MES, phosphate, or histidine buffer (preferably 20 mM succinate buffer); 146 mM (though optionally 100 mM) sugar component selected from trehalose or sucrose (most preferably trehalose); and a basic amino acid selected from lysine or arginine (most preferably lysine) at a concentration of 50 mM, 75 mM, 125 mM, or 135 mM.

E22. 20 mM buffer selected from a succinate, MES, phosphate, or histidine buffer (preferably 20 mM succinate buffer); 146 mM (though optionally 100 mM) sugar component selected from trehalose or sucrose (most preferably trehalose); and 125 mM sodium chloride.

E23. 20 mM buffer selected from a succinate, MES, phosphate, or histidine buffer (preferably 20 mM succinate buffer); a basic amino acid selected from lysine or arginine (most preferably lysine) at a concentration of 50 mM, 75 mM, 125 mM, or 135 mM; and 125 mM sodium chloride.

E24. 12.5 mM organic salt selected from a lactate or acetate salt (most preferably 20 mM lactate); 146 mM (though optionally 100 mM) sugar component selected from trehalose or sucrose (most preferably trehalose); and a basic amino acid selected from lysine or arginine (most preferably lysine) at a concentration of 50 mM, 75 mM, 125 mM, or 135 mM.

E25. 20 mM glutamate salt; 146 mM (though optionally 100 mM) sugar component selected from trehalose or sucrose (most preferably trehalose); and a basic amino acid selected from lysine or arginine (most preferably lysine) at a concentration of 50 mM, 75 mM, 125 mM, or 135 mM.

E26. 12.5 mM organic salt selected from a lactate or acetate salt (most preferably 20 mM lactate); 146 mM (though optionally 100 mM) sugar component selected from trehalose or sucrose (most preferably trehalose); and 125 mM sodium chloride.

E27. 20 mM glutamate salt; 146 mM (though optionally 100 mM) sugar component selected from trehalose or sucrose (most preferably trehalose); and 125 mM sodium chloride.

E28. 12.5 mM organic salt selected from a lactate or acetate salt (most preferably 20 mM lactate); a basic amino acid selected from lysine or arginine (most preferably lysine) at a concentration of 50 mM, 75 mM, 125 mM, or 135 mM; and 125 mM sodium chloride.

E29. 20 mM glutamate salt; a basic amino acid selected from lysine or arginine (most preferably lysine) at a concentration of 50 mM, 75 mM, 125 mM, or 135 mM; and 125 mM sodium chloride.

E30. 146 mM (though optionally 100 mM) sugar component selected from trehalose or sucrose (most preferably trehalose); a basic amino acid selected from lysine or arginine (most preferably lysine) at a concentration of 50 mM, 75 mM, 125 mM, or 135 mM; and 125 mM sodium chloride.

E31. 20 mM buffer selected from a succinate, MES, phosphate, or histidine buffer (preferably 20 mM succinate buffer); 12.5 mM organic salt selected from a lactate or acetate salt (most preferably 20 mM lactate); 146 mM (though optionally 100 mM) sugar component selected from trehalose or sucrose (most preferably trehalose); and a basic amino acid selected from lysine or arginine (most preferably lysine) at a concentration of 50 mM, 75 mM, 125 mM, or 135 mM.

E32. 20 mM buffer selected from a succinate, MES, phosphate, or histidine buffer (preferably 20 mM succinate buffer); 20 mM glutamate salt; 146 mM (though optionally 100 mM) sugar component selected from trehalose or sucrose (most preferably trehalose); and a basic amino acid selected from lysine or arginine (most preferably lysine) at a concentration of 50 mM, 75 mM, 125 mM, or 135 mM.

E33. 20 mM buffer selected from a succinate, MES, phosphate, or histidine buffer (preferably 20 mM succinate buffer); 12.5 mM organic salt selected from a lactate or acetate salt (most preferably 20 mM lactate); 146 mM (though optionally 100 mM) sugar component selected from trehalose or sucrose (most preferably trehalose); and 125 mM sodium chloride.

E34. 20 mM buffer selected from a succinate, MES, phosphate, or histidine buffer (preferably 20 mM succinate buffer); 20 mM glutamate salt; 146 mM (though optionally 100 mM) sugar component selected from trehalose or sucrose (most preferably trehalose); and 125 mM sodium chloride.

E35. 20 mM buffer selected from a succinate, MES, phosphate, or histidine buffer (preferably 20 mM succinate buffer); 12.5 mM organic salt selected from a lactate or acetate salt (most preferably 20 mM lactate); a basic amino acid selected from lysine or arginine (most preferably lysine) at a concentration of 50 mM, 75 mM, 125 mM, or 135 mM; and 125 mM sodium chloride.

E36. 20 mM buffer selected from a succinate, MES, phosphate, or histidine buffer (preferably 20 mM succinate buffer); 20 mM glutamate salt; a basic amino acid selected from lysine or arginine (most preferably lysine) at a concentration of 50 mM, 75 mM, 125 mM, or 135 mM; and 125 mM sodium chloride.

E37. 20 mM buffer selected from a succinate, MES, phosphate, or histidine buffer (preferably 20 mM succinate buffer); 146 mM (though optionally 100 mM) sugar component selected from trehalose or sucrose (most preferably trehalose); a basic amino acid selected from lysine or arginine (most preferably lysine) at a concentration of 50 mM, 75 mM, 125 mM, or 135 mM; and 125 mM sodium chloride.

E38. 12.5 mM organic salt selected from a lactate or acetate salt (most preferably 20 mM lactate); 146 mM (though optionally 100 mM) sugar component selected from trehalose or sucrose (most preferably trehalose); a basic amino acid selected from lysine or arginine (most preferably lysine) at a concentration of 50 mM, 75 mM, 125 mM, or 135 mM; and 125 mM sodium chloride.

E39. 20 mM glutamate salt; 146 mM (though optionally 100 mM) sugar component selected from trehalose or sucrose (most preferably trehalose); a basic amino acid selected from lysine or arginine (most preferably lysine) at a concentration of 50 mM, 75 mM, 125 mM, or 135 mM; and 125 mM sodium chloride.

E40. 20 mM buffer selected from a succinate, MES, phosphate, or histidine buffer (preferably 20 mM succinate buffer); 12.5 mM organic salt selected from a lactate or acetate salt (most preferably 20 mM lactate); 146 mM (though optionally 100 mM) sugar component selected from trehalose or sucrose (most preferably trehalose); a basic amino acid selected from lysine or arginine (most preferably lysine) at a concentration of 50 mM, 75 mM, 125 mM, or 135 mM; and 125 mM sodium chloride.

E41. 20 mM buffer selected from a succinate, MES, phosphate, or histidine buffer (preferably 20 mM succinate buffer); 20 mM glutamate salt; 146 mM (though optionally 100 mM) sugar component selected from trehalose or sucrose (most preferably trehalose); a basic amino acid selected from lysine or arginine (most preferably lysine) at a concentration of 50 mM, 75 mM, 125 mM, or 135 mM; and 125 mM sodium chloride.

*Embodiments with Specific Components (and their associated molar ratios)*

F) The following numbered paragraphs, F1-F26, relate to particular embodiments of a liquid pharmaceutical composition (whose components are preferably present in the relevant molar ratios stipulated in aspects A91-A186), all of which have a pH of 5.6-6.2, all of which are free of at least one (preferably at least two) of histidine, acetate, sucrose, and arginine, and all of which are optionally further characterised by one or more (preferably by two or more, most preferably all) of an ionic strength of 50-200 mM, an osmolality of 250-350 mOsmol/kg, a viscosity of less than or equal to 14 mPa.s, and a turbidity of less than or equal to 28 NTU; wherein each composition comprises or, more preferably, consists of: dupilumab; a surfactant selected from a polysorbate surfactant or a poloxamer surfactant (preferably polysorbate 80); water; and also:

F1. a buffer selected from a succinate, MES, phosphate, or histidine buffer (preferably succinate); and an organic acid salt selected from a lactate, glutamate, or acetate salt (preferably lactate).

F2. a buffer selected from a succinate, MES, phosphate, or histidine buffer (preferably succinate); and a non-ionic polyol selected from sucrose or trehalose (preferably trehalose).

F3. a buffer selected from a succinate, MES, phosphate, or histidine buffer (preferably succinate); and a basic amino acid selected from lysine or arginine (preferably lysine).

F4. a buffer selected from a succinate, MES, phosphate, or histidine buffer (preferably succinate); and an inorganic salt selected from NaCl or KCl (preferably NaCl).

F5. an organic acid salt selected from a lactate, glutamate, or acetate salt (preferably lactate); and a non-ionic polyol selected from sucrose or trehalose (preferably trehalose).

F6. an organic acid salt selected from a lactate, glutamate, or acetate salt (preferably lactate); and a basic amino acid selected from lysine or arginine (preferably lysine).

F7. an organic acid salt selected from a lactate, glutamate, or acetate salt (preferably lactate); and an inorganic salt selected from NaCl or KCl (preferably NaCl).

F8. a non-ionic polyol selected from sucrose or trehalose (preferably trehalose); and a basic amino acid selected from lysine or arginine (preferably lysine).

F9. a non-ionic polyol selected from sucrose or trehalose (preferably trehalose); and an inorganic salt selected from NaCl or KCl (preferably NaCl).

F10. a basic amino acid selected from lysine or arginine (preferably lysine); and an inorganic salt selected from NaCl or KCl (preferably NaCl).

F11. a buffer selected from a succinate, MES, phosphate, or histidine buffer (preferably succinate); an organic acid salt selected from a lactate, glutamate, or acetate salt (preferably lactate); and a non-ionic polyol selected from sucrose or trehalose (preferably trehalose).

F12. a buffer selected from a succinate, MES, phosphate, or histidine buffer (preferably succinate); an organic acid salt selected from a lactate, glutamate, or acetate salt (preferably lactate); and a basic amino acid selected from lysine or arginine (preferably lysine).

F13. a buffer selected from a succinate, MES, phosphate, or histidine buffer (preferably succinate); an organic acid salt selected from a lactate, glutamate, or acetate salt (preferably lactate); and an inorganic salt selected from NaCl or KCl (preferably NaCl).

F14. a buffer selected from a succinate, MES, phosphate, or histidine buffer (preferably succinate); a non-ionic polyol selected from sucrose or trehalose (preferably trehalose); and a basic amino acid selected from lysine or arginine (preferably lysine).

F15. a buffer selected from a succinate, MES, phosphate, or histidine buffer (preferably succinate); a non-ionic polyol selected from sucrose or trehalose (preferably trehalose); and an inorganic salt selected from NaCl or KCl (preferably NaCl).

F16. a buffer selected from a succinate, MES, phosphate, or histidine buffer (preferably succinate); a basic amino acid selected from lysine or arginine (preferably lysine); and an inorganic salt selected from NaCl or KCl (preferably NaCl).

F17. an organic acid salt selected from a lactate, glutamate, or acetate salt (preferably lactate); a non-ionic polyol selected from sucrose or trehalose (preferably trehalose); and a basic amino acid selected from lysine or arginine (preferably lysine).

F18. an organic acid salt selected from a lactate, glutamate, or acetate salt (preferably lactate); a non-ionic polyol selected from sucrose or trehalose (preferably trehalose); and an inorganic salt selected from NaCl or KCl (preferably NaCl).

F19. an organic acid salt selected from a lactate, glutamate, or acetate salt (preferably lactate); a basic amino acid selected from lysine or arginine (preferably lysine); and an inorganic salt selected from NaCl or KCl (preferably NaCl).

F20. a non-ionic polyol selected from sucrose or trehalose (preferably trehalose); a basic amino acid selected from lysine or arginine (preferably lysine); and an inorganic salt selected from NaCl or KCl (preferably NaCl).

F21. a buffer selected from a succinate, MES, phosphate, or histidine buffer (preferably succinate); an organic acid salt selected from a lactate, glutamate, or acetate salt (preferably lactate); a non-ionic polyol selected from sucrose or trehalose (preferably trehalose); and a basic amino acid selected from lysine or arginine (preferably lysine).

F22. a buffer selected from a succinate, MES, phosphate, or histidine buffer (preferably succinate); an organic acid salt selected from a lactate, glutamate, or acetate salt (preferably lactate); a non-ionic polyol selected from sucrose or trehalose (preferably trehalose); and an inorganic salt selected from NaCl or KCl (preferably NaCl).

F23. a buffer selected from a succinate, MES, phosphate, or histidine buffer (preferably succinate); an organic acid salt selected from a lactate, glutamate, or acetate salt (preferably lactate); a basic amino acid selected from lysine or arginine (preferably lysine); and an inorganic salt selected from NaCl or KCl (preferably NaCl).

F24. a buffer selected from a succinate, MES, phosphate, or histidine buffer (preferably succinate); a non-ionic polyol selected from sucrose or trehalose (preferably trehalose); a basic amino acid selected from lysine or arginine (preferably lysine); and an inorganic salt selected from NaCl or KCl (preferably NaCl).

F25. an organic acid salt selected from a lactate, glutamate, or acetate salt (preferably lactate); a non-ionic polyol selected from sucrose or trehalose (preferably trehalose); a basic amino acid selected from lysine or arginine (preferably lysine); and an inorganic salt selected from NaCl or KCl (preferably NaCl).

F26. a buffer selected from a succinate, MES, phosphate, or histidine buffer (preferably succinate); an organic acid salt selected from a lactate, glutamate, or acetate salt (preferably lactate); a non-ionic polyol selected from sucrose or trehalose (preferably trehalose); a basic amino acid selected from lysine or arginine (preferably lysine); and an inorganic salt selected from NaCl or KCl (preferably NaCl).

The liquid pharmaceutical compositions according to embodiments F1-F26 are preferably further defined by the relevant molar ratios expressed in aspects A91-A186. Relevant molar ratios are those of aspects A91-A186 which self-evidently map onto the corresponding components/ingredients stipulated in embodiments F1-F26 (though F1-F26 defines said components/ingredients more specifically, and thus the relevant molar ratios apply to the more specific components/ingredients defined in F1-F26). In an example:

- F8 is further defined by the molar ratios of A96 to provide a molar ratio of **dupilumab** : **non-ionic polyol** selected from sucrose or trehalose (preferably trehalose) : **basic amino acid** selected from lysine or arginine (preferably lysine) : **surfactant** selected from a polysorbate surfactant or a poloxamer surfactant (preferably polysorbate 80) of 1 : 20-500 : 5-300 : 0.01-3 respectively.

- F8 is defined by the molar ratios of A128 to provide a molar ratio of **dupilumab** : **non-ionic polyol** selected from sucrose or trehalose (preferably trehalose) : **basic amino acid** selected from lysine or arginine (preferably lysine) : **surfactant** selected from a polysorbate surfactant or a poloxamer surfactant (preferably polysorbate 80) of 1 : 50-250 : 10-200 : 0.1-2 respectively.

- F8 is defined by the molar ratios of A160 to provide a molar ratio of **dupilumab** : **non-ionic polyol** selected from sucrose or trehalose (preferably trehalose) : **basic amino acid** selected from lysine or arginine (preferably lysine) : **surfactant** selected from a polysorbate surfactant or a poloxamer surfactant (preferably polysorbate 80) of 1 :

100-150 : 25-150 : 1-1.7 respectively.

The person skilled in the art can readily match each and every embodiment of embodiments F1-F26 with relevant molar ratios of aspects A91-A186.

*More Specific Embodiments*

In a particular embodiment, the liquid pharmaceutical composition comprises or, more preferably, consists of (along with water): dupilumab, a surfactant, and two or more of: an organic acid salt that is either lactate or glutamate; trehalose; and a basic amino acid that is either lysine or arginine.

In a particular embodiment, the liquid pharmaceutical composition comprises or, more preferably, consists of (along with water): dupilumab, a surfactant that is either polysorbate 80 or poloxamer 188, and two or more of: an organic acid salt that is either lactate or glutamate; trehalose; and a basic amino acid that is either lysine or arginine.

In a particular embodiment, the liquid pharmaceutical composition comprises or, more preferably, consists of (along with water): dupilumab, polysorbate 80, and two or more of: an organic acid salt that is either lactate or glutamate; trehalose; and a basic amino acid that is either lysine or arginine.

In a particular embodiment, the liquid pharmaceutical composition comprises or, more preferably, consists of (along with water): dupilumab, poloxamer 188, and two or more of: an organic acid salt that is either lactate or glutamate; trehalose; and a basic amino acid that is either lysine or arginine.

In a particular embodiment, the liquid pharmaceutical composition comprises or, more preferably, consists of (along with water): dupilumab, a surfactant, and two or more of: an organic acid salt that is either lactate or glutamate; trehalose; and lysine.

In a particular embodiment, the liquid pharmaceutical composition comprises or, more preferably, consists of (along with water): dupilumab, a surfactant that is either polysorbate 80 or poloxamer 188, and two or more of: an organic acid salt that is either lactate or glutamate; trehalose; and lysine.

In a particular embodiment, the liquid pharmaceutical composition comprises or, more preferably, consists of (along with water): dupilumab, polysorbate 80, and two or more of: an organic acid salt that is either lactate or glutamate; trehalose; and lysine.

In a particular embodiment, the liquid pharmaceutical composition comprises or, more preferably, consists of (along with water): dupilumab, poloxamer 188, and two or more of: an organic acid salt that is either lactate or glutamate; trehalose; and lysine.

In a particular embodiment, the liquid pharmaceutical composition has a theoretical ionic strength between 50 and 180 mM, a theoretical osmolality between 250 and 340 mOsmol/kg, and comprises or, more preferably, consists of (along with water): dupilumab, a surfactant, and two or more of: an organic acid salt that is either lactate or glutamate; trehalose; and a basic amino acid that is either lysine or arginine.

In a particular embodiment, the liquid pharmaceutical composition has a theoretical ionic strength between 50 and 180 mM, a theoretical osmolality between 250 and 340 mOsmol/kg, and comprises or, more preferably, consists of (along with water): dupilumab, a surfactant that is either polysorbate 80 or poloxamer 188, and two or more of: an organic acid salt that is either lactate or glutamate; trehalose; and a basic amino acid that is either lysine or arginine.

In a particular embodiment, the liquid pharmaceutical composition has a theoretical ionic strength between 50 and 180 mM, a theoretical osmolality between 250 and 340 mOsmol/kg, and comprises or, more preferably, consists of (along with water): dupilumab, polysorbate 80, and two or more of: an organic acid salt that is either lactate or glutamate; trehalose; and a basic amino acid that is either lysine or arginine.

In a particular embodiment, the liquid pharmaceutical composition has a theoretical ionic strength between 50 and 180 mM, a theoretical osmolality between 250 and 340 mOsmol/kg, and comprises or, more preferably, consists of (along with water): dupilumab, poloxamer 188, and two or more of: an organic acid salt that is either lactate or glutamate; trehalose; and a basic amino acid that is either lysine or arginine.

In a particular embodiment, the liquid pharmaceutical composition has a theoretical ionic strength between 50 and 180 mM, a theoretical osmolality between 250 and 340 mOsmol/kg, and comprises or, more preferably, consists of (along with water): dupilumab, a surfactant, and two or more of: an organic acid salt that is either lactate or glutamate; trehalose; and lysine.

In a particular embodiment, the liquid pharmaceutical composition has a theoretical ionic strength between 50 and 180 mM, a theoretical osmolality between 250 and 340 mOsmol/kg, and comprises or, more preferably, consists of (along with water): dupilumab, a surfactant that is either polysorbate 80 or poloxamer 188, and two or more of: an organic acid salt that is either lactate or glutamate; trehalose; and lysine.

In a particular embodiment, the liquid pharmaceutical composition has a theoretical ionic strength between 50 and 180 mM, a theoretical osmolality between 250 and 340 mOsmol/kg, and comprises or, more preferably, consists of (along with water): dupilumab, polysorbate 80, and two or more of: an organic acid salt that is either lactate or glutamate;

trehalose; and lysine.

In a particular embodiment, the liquid pharmaceutical composition has a theoretical ionic strength between 50 and 180 mM, a theoretical osmolality between 250 and 340 mOsmol/kg, and comprises or, more preferably, consists of (along with water): dupilumab, poloxamer 188, and two or more of: an organic acid salt that is either lactate or glutamate; trehalose; and lysine.

Preferably, the components/ingredients stipulated in any of the aforesaid embodiments in this section, are present at concentration(s) and/or in molar ratio(s) set forth herein in relation to the relevant components/ingredients (generic or otherwise), especially at concentration(s) as set forth in any of aspects A21-A50 or A71-A90, and/or molar ratio(s) as set forth in any of aspects A91-A186.

*Specific Embodiments Even More Focussed on the Examples*

**F37:** In a particular embodiment, the liquid pharmaceutical composition comprises or, more preferably, consists of 90-210 mg/mL dupilumab, 3-30 mM buffer (preferably selected from succinate, phosphate, or MES buffer), 70-230 mM sugar component (preferably trehalose), 20-80 mM basic amino acid (preferably lysine), and 0.5-2.5 mg/mL surfactant (preferably polysorbate 80); wherein the composition has a pH of 5.8-6.0.

**F39:** In a particular embodiment, the liquid pharmaceutical composition comprises or, more preferably, consists of 90-210 mg/mL dupilumab, 3-30 mM buffer (preferably selected from succinate, phosphate, or MES buffer), 70-230 mM sugar component (preferably trehalose), 20-80 mM basic amino acid (preferably lysine), and 0.5-2.5 mg/mL surfactant (preferably polysorbate 80); wherein the composition has a pH of 5.6-6.2.

**F41:** In a particular embodiment, the liquid pharmaceutical composition comprises or, more preferably, consists of 90-210 mg/mL dupilumab, 5-30 mM organic acid salt (preferably glutamate), 70-230 mM sugar component (preferably trehalose), 20-80 mM basic amino acid (preferably lysine), and 0.5-2.5 mg/mL surfactant (preferably polysorbate 80); wherein the composition has a pH of 5.6-6.0.

**F44:** In a particular embodiment, the liquid pharmaceutical composition comprises or, more preferably, consists of 90-210 mg/mL dupilumab, 5-25 mM organic acid salt (preferably lactate), 70-230 mM sugar component (preferably trehalose), 20-80 mM basic amino acid (preferably lysine), and 0.5-2.5 mg/mL surfactant (preferably polysorbate 80); wherein the composition has a pH of 5.8-6.0.

**F45:** In a particular embodiment, the liquid pharmaceutical composition comprises or, more preferably, consists of 90-210 mg/mL dupilumab, 5-25 mM organic acid salt (preferably lactate), 70-230 mM sugar component (preferably trehalose), 20-80 mM basic amino acid (preferably lysine), and 0.5-2.5 mg/mL surfactant (preferably poloxamer 188); wherein the composition has a pH of 5.8-6.0.

**F46:** In a particular embodiment, the liquid pharmaceutical composition comprises or, more preferably, consists of 90-210 mg/mL dupilumab, 5-25 organic acid salt (preferably lactate), 50-140 mM basic amino acid (preferably arginine, preferably partial hydrochloride salt thereof), and 0.5-2.5 mg/mL surfactant (preferably polysorbate 80); wherein the composition has a pH of 5.8-6.0.

**F37:** In a particular embodiment, the liquid pharmaceutical composition comprises or, more preferably, consists of 90-210 mg/mL dupilumab, 3-30 mM succinate buffer, 100-200 mM trehalose, 20-80 mM lysine (e.g. hydrochloride salt), and 0.5-2.5 mg/mL polysorbate 80; wherein the composition has a pH of 5.8-6.0.

**F39:** In a particular embodiment, the liquid pharmaceutical composition comprises or, more preferably, consists of 90-210 mg/mL dupilumab, 3-30 mM succinate buffer, 100-200 mM trehalose, 20-80 mM lysine hydrochloride, and 0.5-2.5 mg/mL polysorbate 80; wherein the composition has a pH of 5.6-6.0.

**F41:** In a particular embodiment, the liquid pharmaceutical composition comprises or, more preferably, consists of 90-210 mg/mL dupilumab, 5-30 mM glutamate, 100-200 mM trehalose, 20-80 mM lysine hydrochloride, and 0.5-2.5 mg/mL polysorbate 80; wherein the composition has a pH of 5.6-6.0.

**F44:** In a particular embodiment, the liquid pharmaceutical composition comprises or, more preferably, consists of 90-210 mg/mL dupilumab, 5-25 mM lactate, 100-200 mM trehalose, 20-80 mM lysine hydrochloride, and 0.5-2.5 mg/mL polysorbate 80; wherein the composition has a pH of 5.8-6.0.

**F45:** In a particular embodiment, the liquid pharmaceutical composition comprises or, more preferably, consists of 90-210 mg/mL dupilumab, 5-25 mM lactate, 100-200 mM trehalose, 20-80 mM lysine hydrochloride, and 0.5-2.5 mg/mL poloxamer 188; wherein the composition has a pH of 5.8-6.0.

**F46:** In a particular embodiment, the liquid pharmaceutical composition comprises or, more preferably, consists of 90-210 mg/mL dupilumab, 5-25 lactate, 45-125 mM arginine hydrochloride, 5-15 mM arginine (zwitterionic), and 0.5-2.5 mg/mL polysorbate 80; wherein the composition has a pH of 5.8-6.0.

**F36:** In a particular embodiment, the liquid pharmaceutical composition comprises or, more preferably, consists of 175 mg/mL dupilumab, 20 mM succinate buffer, 125 mM lysine (hydrochloride salt), and 2 mg/mL polysorbate 80; wherein the composition has a pH of 5.7.

**F37:** In a particular embodiment, the liquid pharmaceutical composition comprises or, more preferably, consists of 175 mg/mL dupilumab, 20 mM succinate buffer, 146 mM trehalose, 50 mM lysine (hydrochloride salt), and 2 mg/mL polysorbate 80; wherein the composition has a pH of 5.9.

**F38:** In a particular embodiment, the liquid pharmaceutical composition comprises or, more preferably, consists of 175 mg/mL dupilumab, 20 mM succinate buffer, 125 mM NaCl, and 2 mg/mL polysorbate 80; wherein the composition has a pH of 5.7.

**F39:** In a particular embodiment, the liquid pharmaceutical composition comprises or, more preferably, consists of 175 mg/mL dupilumab, 20 mM succinate buffer, 146 mM trehalose, 50 mM lysine hydrochloride, and 2 mg/mL polysorbate 80; wherein the composition has a pH of 5.7.

**F40:** In a particular embodiment, the liquid pharmaceutical composition comprises or, more preferably, consists of 175 mg/mL dupilumab, 20 mM MES buffer, 12.5 mM lactic acid, 146 mM trehalose, 50 mM lysine hydrochloride, and 2 mg/mL polysorbate 80; wherein the composition has a pH of 5.9.

**F41:** In a particular embodiment, the liquid pharmaceutical composition comprises or, more preferably, consists of 175 mg/mL dupilumab, 20 mM glutamate, 146 mM trehalose, 50 mM lysine hydrochloride, and 2 mg/mL polysorbate 80; wherein the composition has a pH of 5.7.

**F42:** In a particular embodiment, the liquid pharmaceutical composition comprises or, more preferably, consists of 175 mg/mL dupilumab, 20 mM phosphate buffer, 12.5 mM lactate, 125 mM lysine hydrochloride, and 2 mg/mL polysorbate 80; wherein the composition has a pH of 5.9.

**F43:** In a particular embodiment, the liquid pharmaceutical composition comprises or, more preferably, consists of 175 mg/mL dupilumab, 146 mM trehalose, 75 mM lysine hydrochloride, and 2 mg/mL polysorbate 80; wherein the composition has a pH of 5.9.

**F44:** In a particular embodiment, the liquid pharmaceutical composition comprises or, more preferably, consists of 175 mg/mL dupilumab, 12.5 mM lactate, 146 mM trehalose, 50 mM lysine hydrochloride, and 2 mg/mL polysorbate 80; wherein the composition has a pH of 5.9.

**F45:** In a particular embodiment, the liquid pharmaceutical composition comprises or, more preferably, consists of 175 mg/mL dupilumab, 12.5 mM lactate, 146 mM trehalose, 50 mM lysine hydrochloride, and 2 mg/mL poloxamer 188; wherein the composition has a pH of 5.9.

**F46:** In a particular embodiment, the liquid pharmaceutical composition comprises or, more preferably, consists of 175 mg/mL dupilumab, 12.5 mM lactate, 122.7 mM arginine hydrochloride, 12.3 mM arginine (zwitterionic), and 2 mg/mL polysorbate 80; wherein the composition has a pH of 5.9.

**F47:** In a particular embodiment, the liquid pharmaceutical composition comprises or, more preferably, consists of 90-210 mg/mL mg/mL dupilumab, 5-30 mM organic acid salt (preferably lactate), 90-150 mM basic amino acid (preferably arginine), and 0.5-2.5 mg/mL surfactant (preferably polysorbate 80); wherein the composition has a pH of 5.6-6.2.

**F48:** In a particular embodiment, the liquid pharmaceutical composition comprises or, more preferably, consists of 90-210 mg/mL mg/mL dupilumab, 3-30 mM buffer (preferably selected from succinate, phosphate, or MES buffer), 50-230 mM sugar component (preferably trehalose), 50-100 mM basic amino acid (preferably lysine), and 0.5-2.5 mg/mL surfactant (preferably polysorbate 80); wherein the composition has a pH of 5.6-6.2.

**F48a:** In a particular embodiment, the liquid pharmaceutical composition comprises or, more preferably, consists of 90-210 mg/mL mg/mL dupilumab, 3-30 mM buffer (preferably selected from succinate, phosphate, or MES buffer), 90-150 mM basic amino acid (preferably lysine), and 0.5-2.5 mg/mL surfactant (preferably poloxamer 188); wherein the composition has a pH of 5.6-6.2.

**F49:** In a particular embodiment, the liquid pharmaceutical composition comprises or, more preferably, consists of 90-210 mg/mL mg/mL dupilumab, 3-30 mM buffer (preferably selected from succinate, phosphate, or MES buffer), 50-230 mM sugar component (preferably trehalose), 40-80 mM basic amino acid (preferably lysine), and 0.5-2.5 mg/mL surfactant (preferably poloxamer 188); wherein the composition has a pH of 5.6-6.2.

**F50:** In a particular embodiment, the liquid pharmaceutical composition comprises or, more preferably, consists of 90-210 mg/mL mg/mL dupilumab, 5-30 mM organic acid salt (preferably glutamate), 50-230 mM sugar component (preferably trehalose), 40-80 mM basic amino acid (preferably lysine), and 0.5-2.5 mg/mL surfactant (preferably polysorbate 80); wherein the composition has a pH of 5.6-6.2.

**F51:** In a particular embodiment, the liquid pharmaceutical composition comprises or, more preferably, consists of 90-210 mg/mL mg/mL dupilumab, 5-30 mM organic acid salt (preferably lactate), 90-150 mM basic amino acid (preferably arginine), and 0.5-2.5 mg/mL surfactant (preferably poloxamer 188); wherein the composition has a pH of 5.6-6.2.

**F47:** In a particular embodiment, the liquid pharmaceutical composition comprises or, more preferably, consists of 90-210 mg/mL mg/mL dupilumab, 5-30 mM lactate, 90-150 mM arginine hydrochloride, and 0.5-2.5 mg/mL polysorbate 80; wherein the composition has a pH of 5.6-6.0.

**F48:** In a particular embodiment, the liquid pharmaceutical composition comprises or, more preferably, consists of 90-210 mg/mL mg/mL dupilumab, 3-30 mM succinate buffer, 50-200 mM trehalose, 50-100 mM lysine hydrochloride, and 0.5-2.5 mg/mL polysorbate 80; wherein the composition has a pH of 5.6-6.0.

**F48a:** In a particular embodiment, the liquid pharmaceutical composition comprises or, more preferably, consists of 90-210 mg/mL mg/mL dupilumab, 3-30 mM succinate buffer, 90-150 mM lysine hydrochloride, and 0.5-2.5 mg/mL poloxamer 188; wherein the composition has a pH of 5.6-6.0.

**F49:** In a particular embodiment, the liquid pharmaceutical composition comprises or, more preferably, consists of 90-210 mg/mL mg/mL dupilumab, 3-30 mM succinate buffer, 50-200 mM trehalose, 40-80 mM lysine hydrochloride, and 0.5-2.5 mg/mL poloxamer 188; wherein the composition has a pH of 5.6-6.0.

**F50:** In a particular embodiment, the liquid pharmaceutical composition comprises or, more preferably, consists of 90-210 mg/mL mg/mL dupilumab, 5-30 mM glutamate, 50-200 mM trehalose, 40-80 mM lysine hydrochloride, and 0.5-2.5 mg/mL polysorbate 80; wherein the composition has a pH of 5.6-6.0.

**F51:** In a particular embodiment, the liquid pharmaceutical composition comprises or, more preferably, consists of 90-210 mg/mL mg/mL dupilumab, 5-30 mM lactate, 90-150 mM arginine hydrochloride, and 0.5-2.5 mg/mL poloxamer 188; wherein the composition has a pH of 5.6-6.0.

**F47:** In a particular embodiment, the liquid pharmaceutical composition comprises or, more preferably, consists of 175 mg/mL dupilumab, 12.5 mM lactate, 135 mM arginine hydrochloride, and 2 mg/mL polysorbate 80; wherein the composition has a pH of 5.9.

**F48:** In a particular embodiment, the liquid pharmaceutical composition comprises or, more preferably, consists of 175 mg/mL dupilumab, 20 mM succinate buffer, 100 mM trehalose, 75 mM lysine hydrochloride, and 2 mg/mL polysorbate 80; wherein the composition has a pH of 5.9.

**F48a:** In a particular embodiment, the liquid pharmaceutical composition comprises or, more preferably, consists of 175 mg/mL dupilumab, 20 mM succinate buffer, 125 mM lysine hydrochloride, and 2 mg/mL poloxamer 188; wherein the composition has a pH of 5.7.

**F48b:** In a particular embodiment, the liquid pharmaceutical composition comprises or, more preferably, consists of 175 mg/mL dupilumab, 20 mM succinate buffer, 100 mM trehalose, 75 mM lysine hydrochloride, and 2 mg/mL polysorbate 80; wherein the composition has a pH of 5.7.

**F49:** In a particular embodiment, the liquid pharmaceutical composition comprises or, more preferably, consists of 175 mg/mL dupilumab, 20 mM succinate buffer, 146 mM trehalose, 50 mM lysine hydrochloride, and 2 mg/mL poloxamer 188; wherein the composition has a pH of 5.7.

**F50:** In a particular embodiment, the liquid pharmaceutical composition comprises or, more preferably, consists of 175 mg/mL dupilumab, 20 mM glutamate, 146 mM trehalose, 50 mM lysine hydrochloride, and 2 mg/mL polysorbate 80; wherein the composition has a pH of 5.9.

**F51:** In a particular embodiment, the liquid pharmaceutical composition comprises or, more preferably, consists of 175 mg/mL dupilumab, 20 mM lactate, 135 mM arginine hydrochloride, and 2 mg/mL poloxamer 188; wherein the composition has a pH of 5.9.

**CF1:** In a particular embodiment, the liquid pharmaceutical composition comprises or, more preferably, consists of 90-210 mg/mL dupilumab, 5-25 mM buffer (preferably selected from succinate buffer, phosphate, or MES, most preferably succinate), 5-25 mM organic acid salt (preferably selected from lactate or acetate, most preferably lactate), 70-230 mM sugar component (preferably trehalose), 20-100 mM basic amino acid (preferably selected from lysine or arginine, most preferably lysine), 0.1-2.5 mg/mL surfactant (preferably selected from polysorbate 80 or poloxamer 188, preferably polysorbate 80); wherein the composition has a pH of 5.6-6.2.

**CF2:** In a particular embodiment, the liquid pharmaceutical composition comprises or, more preferably, consists of 90-210 mg/mL dupilumab, 5-25 mM buffer (preferably selected from succinate buffer, phosphate, or MES, most preferably succinate), 5-25 mM organic acid salt (preferably selected from lactate or acetate, most preferably lactate), 70-230 mM sugar component (preferably trehalose), 20-100 mM basic amino acid (preferably selected from lysine or arginine, most preferably lysine), 0.1-2.5 mg/mL surfactant (preferably selected from poloxamer 188 or polysorbate 80, preferably poloxamer 188); wherein the composition has a pH of 5.6-6.2.

**CF3:** In a particular embodiment, the liquid pharmaceutical composition comprises or, more preferably, consists of 90-210 mg/mL dupilumab, 5-25 mM buffer (preferably selected from succinate buffer, phosphate, or MES, most preferably phosphate), 5-25 mM organic acid salt (preferably selected from lactate or acetate, most preferably lactate), 70-230 mM sugar component (preferably trehalose), 20-100 mM basic amino acid (preferably selected from lysine or arginine, most preferably lysine), 0.1-2.5 mg/mL surfactant (preferably selected from polysorbate 80 or poloxamer 188, preferably polysorbate 80); wherein the composition has a pH of 5.6-6.2.

**CF4:** In a particular embodiment, the liquid pharmaceutical composition comprises or, more preferably, consists of 90-210 mg/mL dupilumab, 5-25 mM buffer (preferably selected from succinate buffer, phosphate, or MES, most preferably succinate), 5-25 mM organic acid salt (preferably selected from lactate or acetate, most preferably lactate), 70-230 mM sugar component (preferably trehalose), 20-100 mM basic amino acid (preferably selected from arginine or lysine, most preferably arginine), 0.1-2.5 mg/mL surfactant (preferably selected from polysorbate 80 or poloxamer 188, preferably polysorbate 80); wherein the composition has a pH of 5.6-6.2.

**CF5:** In a particular embodiment, the liquid pharmaceutical composition comprises or, more preferably, consists of 90-210 mg/mL dupilumab, 5-25 mM buffer (preferably selected from succinate buffer, phosphate, or MES, most preferably succinate), 5-30 mM organic acid salt (preferably selected from glutamate, lactate, or acetate, most preferably glutamate), 70-230 mM sugar component (preferably trehalose), 20-100 mM basic amino acid (preferably selected from arginine or lysine, most preferably arginine), 0.1-2.5 mg/mL surfactant (preferably selected from polysorbate 80 or poloxamer 188, preferably polysorbate 80); wherein the composition has a pH of 5.6-6.2.

**CF6:** In a particular embodiment, the liquid pharmaceutical composition comprises or, more preferably, consists of 90-210 mg/mL dupilumab, 5-25 mM buffer (preferably selected from succinate buffer, phosphate, or MES, most preferably succinate), 5-30 mM organic acid salt (preferably selected from glutamate, lactate, or acetate, most preferably glutamate), 70-230 mM sugar component (preferably trehalose), 20-100 mM basic amino acid (preferably selected from arginine or lysine, most preferably arginine), 0.1-1.5 mg/mL surfactant (preferably selected from poloxamer 188 or polysorbate 80, preferably poloxamer 188); wherein the composition has a pH of 5.6-6.2.

**CF7:** In a particular embodiment, the liquid pharmaceutical composition comprises or, more preferably, consists of 90-210 mg/mL dupilumab, 5-25 mM organic acid salt (preferably selected from lactate or acetate, most preferably lactate), 70-230 mM sugar component (preferably trehalose), 20-100 mM basic amino acid (preferably selected from arginine or lysine, most preferably arginine), 0.1-2.5 mg/mL surfactant (preferably selected from polysorbate 80 or poloxamer 188, preferably polysorbate 80); wherein the composition has a pH of 5.6-6.2.

**CF8:** In a particular embodiment, the liquid pharmaceutical composition comprises or, more preferably, consists of 90-210 mg/mL dupilumab, 5-25 mM organic acid salt (preferably selected from lactate or acetate, most preferably lactate), 70-230 mM sugar component (preferably trehalose), 20-100 mM basic amino acid (preferably selected from arginine or lysine, most preferably arginine), 0.1-2.5 mg/mL surfactant (preferably selected from poloxamer 188 or polysorbate 80, preferably poloxamer 188); wherein the composition has a pH of 5.6-6.2.

**CF9:** In a particular embodiment, the liquid pharmaceutical composition comprises or, more preferably, consists of 90-210 mg/mL dupilumab, 5-30 mM organic acid salt (preferably selected from glutamate, lactate, or acetate, most preferably glutamate), 70-230 mM sugar component (preferably trehalose), 20-100 mM basic amino acid (preferably selected from lysine or arginine, most preferably lysine), 0.1-2.5 mg/mL surfactant (preferably selected from polysorbate 80 or poloxamer 188, preferably polysorbate 80); wherein the composition has a pH of 5.6-6.2.

**CF10:** In a particular embodiment, the liquid pharmaceutical composition comprises or, more preferably, consists of 90-210 mg/mL dupilumab, 5-30 mM organic acid salt (preferably selected from glutamate, lactate, or acetate, most preferably glutamate), 70-230 mM sugar component (preferably trehalose), 20-100 mM basic amino acid (preferably selected from lysine or arginine, most preferably lysine), 0.1-2.5 mg/mL surfactant (preferably selected from poloxamer 188 or polysorbate 80, preferably poloxamer 188); wherein the composition has a pH of 5.6-6.2.

**CF11:** In a particular embodiment, the liquid pharmaceutical composition comprises or, more preferably, consists of 90-210 mg/mL dupilumab, 5-30 mM organic acid salt (preferably selected from glutamate, lactate, or acetate, most preferably glutamate), 70-230 mM sugar component (preferably trehalose), 20-100 mM basic amino acid partial salt (preferably with 1-30% free base and 70-99% salt, preferably selected from lysine or arginine, most preferably lysine), 0.1-2.5 mg/mL surfactant (preferably selected from polysorbate 80 or poloxamer 188, preferably polysorbate 80); wherein the composition has a pH of 5.6-6.2.

**CF12:** In a particular embodiment, the liquid pharmaceutical composition comprises or, more preferably, consists of 90-210 mg/mL dupilumab, 5-30 mM organic acid salt (preferably selected from glutamate, lactate, or acetate, most preferably glutamate), 70-230 mM sugar component (preferably trehalose), 20-100 mM basic amino acid partial salt (preferably with 1-30% free base and 70-99% salt, preferably selected from lysine or arginine, most preferably lysine), 0.1-2.5 mg/mL surfactant (preferably selected from poloxamer 188 or polysorbate 80, preferably poloxamer 188); wherein the composition has a pH of 5.6-6.2.

**CF13:** In a particular embodiment, the liquid pharmaceutical composition comprises or, more preferably, consists of 90-210 mg/mL dupilumab, 5-25 mM buffer (preferably selected from succinate buffer, phosphate, or MES, most preferably succinate), 5-25 mM organic acid salt (preferably selected from lactate or acetate, most preferably lactate), 70-230 mM sugar component (preferably trehalose), 10-100 mM inorganic salt (preferably sodium chloride), 0.1-2.5 mg/mL surfactant (preferably selected from polysorbate 80 or poloxamer 188, preferably polysorbate 80); wherein the composition has a pH of 5.6-6.2.

**CF14:** In a particular embodiment, the liquid pharmaceutical composition comprises or, more preferably, consists of 90-210 mg/mL dupilumab, 5-30 mM organic acid salt (preferably selected from glutamate, lactate, or acetate, most preferably glutamate), 70-230 mM sugar component (preferably trehalose), 10-100 mM inorganic salt (preferably sodium chloride), 0.1-2.5 mg/mL surfactant (preferably selected from polysorbate 80 or poloxamer 188, preferably polysorbate 80); wherein the composition has a pH of 5.6-6.2.

**CF1:** In a particular embodiment, the liquid pharmaceutical composition comprises or, more preferably, consists of 175 mg/mL dupilumab, 10 mM succinate buffer, 12.5 mM lactate, 146 mM trehalose, 50 mM lysine (hydrochloride salt), 2 mg/mL polysorbate 80; wherein the composition has a pH of 5.9.

CF2: In a particular embodiment, the liquid pharmaceutical composition comprises or, more preferably, consists of 175 mg/mL dupilumab, 10 mM succinate buffer, 12.5 mM lactate, 146 mM trehalose, 50 mM lysine (hydrochloride salt), 2 mg/mL poloxamer 188; wherein the composition has a pH of 5.9.

CF3: In a particular embodiment, the liquid pharmaceutical composition comprises or, more preferably, consists of 175 mg/mL dupilumab, 10 mM phosphate, 12.5 mM lactate, 146 mM trehalose, 50 mM lysine (hydrochloride salt), 2 mg/mL polysorbate 80; wherein the composition has a pH of 5.9.

CF4: In a particular embodiment, the liquid pharmaceutical composition comprises or, more preferably, consists of 175 mg/mL dupilumab, 10 mM succinate buffer, 12.5 mM lactate, 146 mM trehalose, 67.5 mM arginine hydrochloride, 7.5 mM arginine, 2 mg/mL polysorbate 80; wherein the composition has a pH of 5.9.

CF5: In a particular embodiment, the liquid pharmaceutical composition comprises or, more preferably, consists of 175 mg/mL dupilumab, 10 mM succinate buffer, 20 mM glutamate, 146 mM trehalose, 67.5 mM arginine hydrochloride, 7.5 mM arginine, 2 mg/mL polysorbate 80; wherein the composition has a pH of 5.9.

CF6: In a particular embodiment, the liquid pharmaceutical composition comprises or, more preferably, consists of 175 mg/mL dupilumab, 10 mM succinate buffer, 20 mM glutamate, 146 mM trehalose, 67.5 mM arginine hydrochloride, 7.5 mM arginine, 1 mg/mL poloxamer 188; wherein the composition has a pH of 5.9.

CF7: In a particular embodiment, the liquid pharmaceutical composition comprises or, more preferably, consists of 175 mg/mL dupilumab, 12.5 mM lactate, 146 mM trehalose, 67.5 mM arginine hydrochloride, 7.5 mM arginine, 2 mg/mL polysorbate 80; wherein the composition has a pH of 5.9.

CF8: In a particular embodiment, the liquid pharmaceutical composition comprises or, more preferably, consists of 175 mg/mL dupilumab, 12.5 mM lactate, 146 mM trehalose, 67.5 mM arginine hydrochloride, 7.5 mM arginine, 2 mg/mL poloxamer 188; wherein the composition has a pH of 5.9.

CF9: In a particular embodiment, the liquid pharmaceutical composition comprises or, more preferably, consists of 175 mg/mL dupilumab, 20 mM glutamate, 146 mM trehalose, 50 mM lysine hydrochloride, 2 mg/mL polysorbate 80; wherein the composition has a pH of 5.9.

CF10: In a particular embodiment, the liquid pharmaceutical composition comprises or, more preferably, consists of 175 mg/mL dupilumab, 20 mM glutamate, 146 mM trehalose, 50 mM lysine hydrochloride, 2 mg/mL poloxamer 188; wherein the composition has a pH of 5.9.

CF11: In a particular embodiment, the liquid pharmaceutical composition comprises or, more preferably, consists of 175 mg/mL dupilumab, 20 mM glutamate, 146 mM trehalose, 45 mM lysine hydrochloride, 5 mM lysine, 2 mg/mL polysorbate 80; wherein the composition has a pH of 5.9.

CF12: In a particular embodiment, the liquid pharmaceutical composition comprises or, more preferably, consists of 175 mg/mL dupilumab, 20 mM glutamate, 146 mM trehalose, 45 mM lysine hydrochloride, 5 mM lysine, 2 mg/mL poloxamer 188; wherein the composition has a pH of 5.9.

CF13: In a particular embodiment, the liquid pharmaceutical composition comprises or, more preferably, consists of 175 mg/mL dupilumab, 10 mM succinate buffer, 12.5 mM lactate, 146 mM trehalose, 50 mM sodium chloride, 2 mg/mL polysorbate 80; wherein the composition has a pH of 5.9.

CF14: In a particular embodiment, the liquid pharmaceutical composition comprises or, more preferably, consists of 175 mg/mL dupilumab, 20 mM glutamate, 146 mM trehalose, 50 mM sodium chloride, 2 mg/mL polysorbate 80; wherein the composition has a pH of 5.9.

CF15: In a particular embodiment, the liquid pharmaceutical composition comprises or, more preferably, consists of 90-210 mg/mL dupilumab, 5-30 mM buffer (preferably selected from succinate buffer, phosphate, or MES, most preferably succinate), 5-30 mM organic acid salt (preferably selected from lactate or acetate, most preferably lactate), 50-150 mM basic amino acid (preferably selected from lysine or arginine, most preferably lysine), 0.1-2.5 mg/mL surfactant (preferably selected from polysorbate 80 or poloxamer 188, preferably polysorbate 80); wherein the composition has a pH of 5.6-6.2.

CF16: In a particular embodiment, the liquid pharmaceutical composition comprises or, more preferably, consists of 90-210 mg/mL dupilumab, 5-30 mM buffer (preferably selected from succinate buffer, phosphate, or MES, most preferably succinate), 5-30 mM organic acid salt (preferably selected from lactate or acetate, most preferably lactate), 50-150 mM basic amino acid (preferably selected from lysine or arginine, most preferably lysine), 0.1-2.5 mg/mL surfactant (preferably selected from polysorbate 80 or poloxamer 188, preferably poloxamer 188); wherein the composition has a pH of 5.6-6.2.

CF17: In a particular embodiment, the liquid pharmaceutical composition comprises or, more preferably, consists of 90-210 mg/mL dupilumab, 5-30 mM phosphate buffer, 5-30 mM organic acid salt (preferably selected from lactate or acetate, most preferably lactate), 50-150 mM basic amino acid (preferably selected from lysine or arginine, most preferably lysine), 0.1-2.5 mg/mL surfactant (preferably selected from polysorbate 80 or poloxamer 188, preferably polysorbate 80); wherein the composition has a pH of 5.6-6.2.

CF18: In a particular embodiment, the liquid pharmaceutical composition comprises or, more preferably, consists of 90-210 mg/mL dupilumab, 5-30 mM buffer (preferably selected from succinate buffer, phosphate, or MES, most preferably succinate), 5-30 mM organic acid salt (preferably selected from lactate or acetate, most preferably lactate), 50-150 mM basic amino acid (preferably selected from arginine or lysine, most preferably arginine), 0.1-2.5 mg/mL surfactant (preferably selected from polysorbate 80 or poloxamer 188, preferably polysorbate 80); wherein the composition has a pH of 5.6-6.2.

**CF19:** In a particular embodiment, the liquid pharmaceutical composition comprises or, more preferably, consists of 90-210 mg/mL dupilumab, 5-30 mM buffer (preferably selected from succinate buffer, phosphate, or MES, most preferably succinate), 5-30 mM organic acid salt (preferably selected from glutamate, lactate, or acetate, most preferably glutamate), 50-150 mM basic amino acid (preferably selected from arginine or lysine, most preferably arginine), 0.1-2.5 mg/mL surfactant (preferably selected from polysorbate 80 or poloxamer 188, preferably polysorbate 80); wherein the composition has a pH of 5.6-6.2.

**CF20:** In a particular embodiment, the liquid pharmaceutical composition comprises or, more preferably, consists of 90-210 mg/mL dupilumab, 5-30 mM buffer (preferably selected from succinate buffer, phosphate, or MES, most preferably succinate), 5-30 mM organic acid salt (preferably selected from glutamate, lactate, or acetate, most preferably glutamate), 50-150 mM basic amino acid (preferably selected from arginine or lysine, most preferably arginine), 0.1-1.5 mg/mL surfactant (preferably selected from polysorbate 80 or poloxamer 188, preferably poloxamer 188); wherein the composition has a pH of 5.6-6.2.

**CF21:** In a particular embodiment, the liquid pharmaceutical composition comprises or, more preferably, consists of 90-210 mg/mL dupilumab, 5-30 mM organic acid salt (preferably selected from lactate or acetate, most preferably lactate), 90-150 mM basic amino acid (preferably selected from arginine or lysine, most preferably arginine), 0.1-2.5 mg/mL surfactant (preferably selected from polysorbate 80 or poloxamer 188, preferably polysorbate 80); wherein the composition has a pH of 5.6-6.2.

**CF22:** In a particular embodiment, the liquid pharmaceutical composition comprises or, more preferably, consists of 90-210 mg/mL dupilumab, 5-30 mM organic acid salt (preferably selected from lactate or acetate, most preferably lactate), 90-150 mM basic amino acid (preferably selected from arginine or lysine, most preferably arginine), 0.1-2.5 mg/mL surfactant (preferably selected from polysorbate 80 or poloxamer 188, preferably poloxamer 188); wherein the composition has a pH of 5.6-6.2.

**CF23:** In a particular embodiment, the liquid pharmaceutical composition comprises or, more preferably, consists of 90-210 mg/mL dupilumab, 5-30 mM organic acid salt (preferably selected from glutamate, lactate, or acetate, most preferably glutamate), 90-150 basic amino acid (preferably selected from lysine or arginine, most preferably lysine), 0.1-2.5 mg/mL surfactant (preferably selected from polysorbate 80 or poloxamer 188, preferably polysorbate 80); wherein the composition has a pH of 5.6-6.2.

**CF24:** In a particular embodiment, the liquid pharmaceutical composition comprises or, more preferably, consists of 90-210 mg/mL dupilumab, 5-30 mM organic acid salt (preferably selected from glutamate, lactate, or acetate, most preferably glutamate), 90-150 basic amino acid (preferably selected from lysine or arginine, most preferably lysine), 0.1-2.5 mg/mL surfactant (preferably selected from polysorbate 80 or poloxamer 188, preferably poloxamer 188); wherein the composition has a pH of 5.6-6.2.

**CF25:** In a particular embodiment, the liquid pharmaceutical composition comprises or, more preferably, consists of 90-210 mg/mL dupilumab, 5-30 mM buffer (preferably selected from succinate buffer, phosphate, or MES, most preferably succinate), 90-150 basic amino acid (preferably selected from lysine or arginine, most preferably lysine), 0.1-2.5 mg/mL surfactant (preferably selected from polysorbate 80 or poloxamer 188, preferably polysorbate 80); wherein the composition has a pH of 5.6-6.2.

**CF26:** In a particular embodiment, the liquid pharmaceutical composition comprises or, more preferably, consists of 90-210 mg/mL dupilumab, 5-30 mM buffer (preferably selected from succinate buffer, phosphate, or MES, most preferably succinate), 90-150 basic amino acid (preferably selected from lysine or arginine, most preferably lysine), 0.1-2.5 mg/mL surfactant (preferably selected from polysorbate 80 or poloxamer 188, preferably poloxamer 188); wherein the composition has a pH of 5.6-6.2.

**CF27:** In a particular embodiment, the liquid pharmaceutical composition comprises or, more preferably, consists of 90-210 mg/mL dupilumab, 5-30 mM buffer (preferably selected from succinate buffer, phosphate, or MES, most preferably succinate), 5-30 mM organic acid salt (preferably selected from lactate or acetate, most preferably lactate), 50-150 mM inorganic salt (preferably sodium chloride), 0.1-2.5 mg/mL surfactant (preferably selected from polysorbate 80 or poloxamer 188, preferably polysorbate 80); wherein the composition has a pH of 5.6-6.2.

**CF28:** In a particular embodiment, the liquid pharmaceutical composition comprises or, more preferably, consists of 90-210 mg/mL dupilumab, 5-30 mM organic acid salt (preferably selected from glutamate, lactate, or acetate, most preferably glutamate), 90-150 mM inorganic salt (preferably sodium chloride), 0.1-2.5 mg/mL surfactant (preferably selected from polysorbate 80 or poloxamer 188, preferably polysorbate 80); wherein the composition has a pH of 5.6-6.2.

**CF15:** In a particular embodiment, the liquid pharmaceutical composition comprises or, more preferably, consists of 175 mg/mL dupilumab, 20 mM succinate buffer, 20 mM lactate, 100 mM lysine (hydrochloride salt), 2 mg/mL polysorbate 80; wherein the composition has a pH of 5.9.

**CF16:** In a particular embodiment, the liquid pharmaceutical composition comprises or, more preferably, consists of 175 mg/mL dupilumab, 20 mM succinate buffer, 20 mM lactate, 100 mM lysine (hydrochloride salt), 2 mg/mL poloxamer 188; wherein the composition has a pH of 5.9.

**CF17:** In a particular embodiment, the liquid pharmaceutical composition comprises or, more preferably, consists of 175 mg/mL dupilumab, 20 mM phosphate buffer, 20 mM lactate, 100 mM lysine (hydrochloride salt), 2 mg/mL polysorbate 80; wherein the composition has a pH of 5.9.

**CF18:** In a particular embodiment, the liquid pharmaceutical composition comprises or, more preferably, consists of 175 mg/mL dupilumab, 20 mM succinate buffer, 20 mM lactate, 100 mM arginine hydrochloride, 2 mg/mL polysorbate 80; wherein the composition has a pH of 5.9.

**CF19:** In a particular embodiment, the liquid pharmaceutical composition comprises or, more preferably, consists of 175 mg/mL dupilumab, 20 mM succinate buffer, 20 mM glutamate, 100 mM arginine hydrochloride, 2 mg/mL polysorbate 80; wherein the composition has a pH of 5.9.

**CF20:** In a particular embodiment, the liquid pharmaceutical composition comprises or, more preferably, consists of 175 mg/mL dupilumab, 20 mM succinate buffer, 20 mM glutamate, 100 mM arginine hydrochloride, 1 mg/mL poloxamer 188; wherein the composition has a pH of 5.9.

**CF21:** In a particular embodiment, the liquid pharmaceutical composition comprises or, more preferably, consists of 175 mg/mL dupilumab, 20 mM lactate, 125 mM arginine hydrochloride, 2 mg/mL polysorbate 80; wherein the composition has a pH of 5.9.

**CF22:** In a particular embodiment, the liquid pharmaceutical composition comprises or, more preferably, consists of 175 mg/mL dupilumab, 20 mM lactate, 125 mM arginine hydrochloride, 2 mg/mL poloxamer 188; wherein the composition has a pH of 5.9.

**CF23:** In a particular embodiment, the liquid pharmaceutical composition comprises or, more preferably, consists of 175 mg/mL dupilumab, 20 mM glutamate, 125 lysine hydrochloride, 2 mg/mL polysorbate 80; wherein the composition has a pH of 5.9.

**CF24:** In a particular embodiment, the liquid pharmaceutical composition comprises or, more preferably, consists of 175 mg/mL dupilumab, 20 mM glutamate, 125 lysine hydrochloride, 2 mg/mL poloxamer 188; wherein the composition has a pH of 5.9.

**CF25:** In a particular embodiment, the liquid pharmaceutical composition comprises or, more preferably, consists of 175 mg/mL dupilumab, 20 mM succinate buffer, 125 lysine hydrochloride, 2 mg/mL polysorbate 80; wherein the composition has a pH of 5.9.

**CF26:** In a particular embodiment, the liquid pharmaceutical composition comprises or, more preferably, consists of 175 mg/mL dupilumab, 20 mM succinate buffer, 125 lysine hydrochloride, 2 mg/mL poloxamer 188; wherein the composition has a pH of 5.9.

**CF27:** In a particular embodiment, the liquid pharmaceutical composition comprises or, more preferably, consists of 175 mg/mL dupilumab, 20 mM succinate buffer, 20 mM lactate, 100 mM sodium chloride, 2 mg/mL polysorbate 80; wherein the composition has a pH of 5.9.

**CF28:** In a particular embodiment, the liquid pharmaceutical composition comprises or, more preferably, consists of 175 mg/mL dupilumab, 20 mM glutamate, 125 mM sodium chloride, 2 mg/mL polysorbate 80; wherein the composition has a pH of 5.9.

Where a composition according to one of the aforesaid embodiments "consists of..." stipulated ingredients, said composition preferably includes water (to make it a "liquid" pharmaceutical composition).

It will be appreciated by a skilled person that unqualified references to a basic amino acid, whether in general or specifically, generally refers to a salt thereof, though it may include partial salts thereof (e.g. 1-30% free base, 70-99% salt form) as may even be specified as such. A similar situation suitably applies in the case of the organic acid salt. A skilled person appreciates that compositions said to "consist of..." a list of ingredients include any cations, anions, salting acids, and the like, associated with such ingredients.

## METHOD OF MANUFACTURING A LIQUID PHARMACEUTICAL COMPOSITION

The present invention provides a method of manufacturing a liquid pharmaceutical composition, suitably as defined herein.

In an aspect of the invention, there is provided a method of manufacturing a liquid pharmaceutical composition (suitably as defined in any of aspects A1-A186 or any sub-definitions thereof directly and unambiguously derivable from this specification), the method comprising mixing together (or otherwise combining) the components included in the liquid pharmaceutical composition, most suitably in a diluent (preferably water for injection), optionally in any amounts, concentrations, and/or forms defined herein; and optionally adjusting any one or more parameters stipulated herein in relation to the liquid pharmaceutical composition (e.g. adjusting pH to achieve the desired or stipulated pH).

The method suitably comprises mixing together, in any particular order deemed appropriate, any relevant ingredients/components required to form a composition as defined herein - suitably such ingredients/components being defined by reference to the pharmaceutical composition (since other than ion-exchanges, proton exchanges, ionic associations, and ionic dissociations, suitably no actual chemical reactions take place during or after mixing). The skilled person may refer to the Examples or techniques well known in the art for forming pharmaceutical compositions (especially those for injection *via* syringe). Different embodiments will suitably require different combinations of components to be mixed, potentially in different amounts. The skilled person can readily deduce such combinations and amounts by reference to the foregoing disclosure relating to the liquid pharmaceutical composition.

Suitably the method involves dissolving all relevant ingredients / components in a diluent (e.g. water, suitably for injection).

In the context of the method of manufacture, the ingredients / components of the pharmaceutical composition may be listed in the method, for instance, in the context of mixing. Such a list of ingredients / components may be an exhaustive list (especially where a plurality of ingredients are mixed together, in which case a diluent is preferably present as well) - such embodiments correspond to embodiments of the pharmaceutical composition which are said to consist of (or consist essentially of) said ingredients / components.

Any, some, or all components may be pre-dissolved or pre-mixed with a diluent prior to mixing with other components.

The final composition may be filtered, suitably to remove particulate matter. Suitably filtration is through filters sized at or below 1 $\mu$m, suitably sized at 0.1-1 $\mu$m, most suitably sized at 0.22$\mu$m. Suitably, filtration is through either PES filters or PVDF filters, suitably with 0.22 $\mu$m PES filters.

The present invention also provides a pharmaceutical composition obtainable by, obtained by, or directly obtained by the method of manufacture herein described.

### *EXAMPLES*

The present invention is now further described by reference to the following further non-limiting examples.

### Materials and Equipment

Dupilumab was obtained (as the injectable liquid formulation, DUPIXENT®) from commercial sources. Such dupilumab formulations were then transformed into example formulations by either dialysis, tangential flow filtration (TFF) followed by spiking (e.g. with surfactant), or by complete purification of the antibody followed by mixing with the relevant excipients.

The various excipients used in formulation preparation are pharmaceutical grade and readily available. Where salts are used, these are generally sodium salts of the relevant acids (e.g. acetate, lactate, glutamate) or hydrochloride salts of the relevant bases (arginine, lysine). Buffers may be formed using a mixture of relevant forms thereof (i.e. mixtures of the relevant conjugate acid and conjugate base) or by using a single form and adjusting pH (e.g. with sodium hydroxide to raise pH, or HCl to lower pH). The skilled person is readily able to implement the formulations described herein.

### Analytical Techniques and Stress Testing Protocols

A person skilled in the art is readily able to implement common stress tests (e.g. thermal, light, mechanical, oxidation, and where relevant over time) and analytical techniques (e.g. SE-UPLC, RP-UPLC, LC-CAD, IcIEF, cGE, MFI, Turbidity, Opalescence (e.g. Nephelometry), Color, Visual Inspection, Protein Content, pH, Osmolality, etc.) known in the art. Some of these are described in more detail below.

**T0:** This is the formulation at time=0, before any stress testing has been applied.

**Tox:** This is the formulation after oxidative stress testing (spiking a formulation with $H_2O_2$ and the quenching with methionine).

**T1m:** This describes formulations after 1 month of thermal stress at 40°C. Plainly this may be extended to multiple months.

**T1m_25C:** This describes formulations after 1 month of thermal stress at 25°C. Plainly different temperatures and different time durations may be specified.

**T-mech:** This describes a mechanical stressing protocol.

**TFT:** This describes terminal freeze-thaw stressing (usually triply freeze-thawed).

**Visual Inspection/Appearance:** This analysis is typically performed to assess visible particles, colour, and turbidity.

**Nephelometry:** This analysis determines a formulation's turbidity in NTU units using a turbidimeter (eg Hach Lange).

**Micro-Flow Imaging (MFI):** Sub-visible particles ($\geq$ 2 $\mu$m, $\geq$ 5 $\mu$m, $\geq$ 10 $\mu$m and $\geq$ 25 $\mu$m) were analyzed by

Microflow imaging using a MFI-5200 particle analyzer system (ProteinSimple, Santa Clara, CA, USA).

**UV spectroscopy (soloVPE):** Concentration and turbidity are measurable using UV spectroscopy, in a manner well known in the art. UV spectroscopy is carried out with a soloVPE system from C Technologies (Bridgewater) coupled to an Agilent Cary 60 UV/VIS spectrometer (Agilent).

**Osmolality:** Osmolality is measurable by method of freezing-point depression using a Gonotec Osmomat 3000 (Gonotec).

**NanoDSF:** NanoDSF measurements utilizing the intrinsic fluorescence of protein are performed with a Prometheus Panta system, in a fashion well known in the art.

**Viscosity:** The dynamic viscosity is measured by using a Kinexus ultra plus rheometer (Malvern Instruments). Viscosity measurements are performed for 3 minutes at a fixed temperature of 25 °C by applying a shear rate of 200 s$^{-1}$, where the fluid exhibits newtonian rheological behaviour.

**RP-UPLC:** Assesses protein and polypeptide variants eg fragments, oxidation variants. It is performed on a (U) HPLC system equipped with a RP column.

**cGE** (NR): May also be used to measure fragmentation. It is performed on a CE system (e.g. PA800 plus (SCIEX) or Maurice C. instrument (ProteinSimple)), equipped with a fused silica capillary.

**Size exclusion ultra high performance chromatography (SE-UPLC):** SE-UPLC is used *inter alia* to assess aggregation. It is performed on a (U)HPLC system equipped with a SEC column.

**Imaged capillary isoelectric focusing (IcIEF):** IcIEF is used to assess main peaks as well as acidic and basic species, and is performed on a Maurice C. instrument (ProteinSimple).

**Anion exchange ultra high performance chromatgraphy (AEX UPLC):** Used inter alia to assess charge variants including main, acidic and basic variants.

## Formulations Screened

Several formulation screens were performed to examine the comparative viability of alternative dupilumab formulations to the originator's DUPIXENT® formulations.

**Screen 1:** A first screen tested formulations F1-F25 (where F1 is a DUPIXENT® comparator but with a 100 mg/mL dupilumab concentration), shown in Table 1. A 100 mg/mL concentration of dupilumab was used in this first screen because this still represents a comparably high antibody concentration that would yield informative results. A variety of formulation aspects were examined in this screen. NB. As indicated beneath Table 1, in practice the dupilumab concentration in these examples was 90 mg/mL.

**Screen 2:** A second screen tested formulations F26-F34 (where F27 and F28 represent different DUPIXENT® comparators), as shown in Table 5. This screen was principally performed for a better understanding of viscosity effects. The antibody used in this study was a dupilumab biosimilar.

**Screen 3:** A third screen tested formulations F35-F46 (where F35 served as the control comparator DUPIXENT®), as shown in Table 6. This screen built upon inventive insights gleaned from the earlier screens. NB. As indicated beneath Table 6, in practice the dupilumab concentration in these examples was closer to 155 mg/mL. However, combining the teachings presented herein with the common general knowledge, it is straightforward for the skilled person to furnish the formulations of Table 6 with 175 mg/mL dupilumab.

**Screen 4:** A third screen tested formulations F35, F39, F47-F53 (where F35 represented a DUPIXENT® comparator), as shown in Table 15. This screen built upon inventive insights gleaned from the earlier screens. The antibody used in this study was a dupilumab biosimilar.

**Screen X:** A further screen was considered for formulations CF1- CF28 against comparator CF_Control, as shown in Table 16.

The formulations of each of these screens are tabulated below (Table 1, Table 5, Table 6, Table 15, Table 16). Common abbreviations are used, for instance in respect of amino acids and for denoting hydrochloride salts - e.g. Arg.HCl (arginine hydrochloride), Lys.HCl (lysine hydrochloride).

As a skilled person will readily appreciate, Ingredients expressed as an anion, such as "lactate", "acetate", "succinate", "phosphate", etc. may generally be added in the form of a (or one or more where different ionisation states are possible) sodium salt(s) thereof, though a skilled person will readily appreciate there are other ways to add the same (achieving the same pH) - e.g. adding their conjugate acid (lactic acid, acetic acid) followed by a subsequent pH adjustment (e.g. with sodium hydroxide) to afford the same result.

Theoretical ionic strengths and theoretical osmolalities were calculated, ignoring/excluding any contributions from dupilumab. Ionic strengths and osmolalities are calculable by methods well known in the art. Buffers contribute to both ionic strength and osmolality in a pH-dependent manner and, as such, a range of values along with mid-points thereof are given to accommodate contributions from buffers. By way of example (based on pHs prevailing in the above tables, i.e. -5.5-6.1, generally 5.9):

- 20 mM histidine buffer (pKa of conjugate acid = 6.0) contributes an ionic strength of 0-20 mM (midpoint 10) and an osmolality of 20-40 mOsm/kg (midpoint 30), because at one extreme all is free histidine (with no ions) and at the other extreme all is imidazolium (protonated) histidine salt (fully ionic).

- 20 mM of any of succinate (pKa 5.6), adipate (pKa 5.4), or phosphate (pKa 7.2) buffers contributes an ionic strength of 20-60 mM (midpoint 40) and an osmolality of 40-60 mOsm/kg (midpoint 50) because at one extreme all is monoanionic salt and at the other extreme all is dianionic salt thereof (presuming sodium salts or other $M^{1+}$ salts).

- 20 mM MES buffer (pKa 6.15) contributes an ionic strength of 0-20 mM (midpoint 10) and an osmolality of 20-40 mOsm/kg (midpoint 30), because at one extreme all is neutral zwitterionic and at the other all is a protonated salt (fully ionic).

## Screen 1 - **Results and Discussion**

Formulations F1-F25 of Table 1 above were assessed by visual inspection, MFI, nanoDSF, IcIEF, cGE NR, SEC, RP, and AEX, at Time 0 (T0), after oxidative stress (Tox), and after 1 month of thermal stress at 40°C (T1m). The relevant results data are presented below in Table 2 (results at time 0), Table 3 (results after oxidative stress), and Table 4 (results after thermal stress).

The results obtained from Screen 1 led to inventive insights which informed the choice of formulations in later screens.

## Screen 2 - **Results and Discussion**

Formulations F26-F34 of Table 5 were assessed in a variety of ways, in particular in respect of viscosity effects.

Figure 1 is bar chart showing the viscosities (at 25C) of formulations F26-F34 at Time 0.

It was noted that higher viscosities were observed in samples characterised by an absence of a charged excipient: e.g. F26 (base formulation), F29 and F30 (both proline), and F33 (glycine). Viscosity reduction by arginine appeared to be concentration-dependent (25 mM F27 vs 50 mM F28). Viscosity reduction by lysine was slightly less pronounced at 50 mM (F31) compared to arginine (F28). However, both 50 mM lysine and 50 mM NaCl appeared to reduce viscosity.

The results obtained from Screen 2 led to inventive insights which informed the choice of formulations in later screens.

## Screen 3 - **Results and Discussion**

Formulations F35-F46 of Table 6 below were assessed by visual inspection, MFI, nanoDSF, IcIEF, cGE NR, SEC, RP, and AEX, at Time 0 (T0), after oxidative stress (Tox), and after 1 month of thermal stress at 40°C (T1m). The data are presented below in Table 7 (visibles/sub-visibles at Time 0 ), Table 8 (other results at Time 0), Table 9 (results after 3FT), Table 10 (results after mechanical stress), Table 11 (visibles/sub-visibles after 1m at 40C), Table 12 (other results after 1m at 40C), Table 13 (visibles/sub-visibles after 2m at 40C), Table 14 (other results after 2m at 40C).

The results obtained from Screen 3 led to inventive insights which informed the choice of formulations in later screens, in particular Screen 4.

**Table 1 - SCREEN 1 Formulations**

| ID | Dupilumab* | pH | Buffer | Organic Acid Salt (Carboxylate) | Non-ionic polyol | Basic amino acid | Inorganic Salt | Non-basic/non-acidic amino acid | Antioxidant | Surfactant | Theoretical Ionic Strength mol/L | Theoretical Osmolality mOsmol/kg |
|----|-----------|-----|--------|-------------------------------|-----------------|-----------------|---------------|-------------------------------|-------------|-----------|--------------------------------|--------------------------------|
| F1 | 100 mg/mL | 5.9 | 20 mM Histidine | 12.5 mM Acetate | 146 mM Sucrose | 25 mM Arg.HCl | | | | 0.2 % (w/v) PS80 | 47.5 (37.5-57.5) | 251 (241-261) |
| F2 | 100 mg/mL | 5.9 | 20 mM Histidine | | 146 mM Sucrose | 25 mM Arg.HCl | | | | 0.2 % (w/v) PS80 | 35 (25-45) | 226 (216-236) |
| F3 | 100 mg/mL | 5.9 | 20 mM Histidine | 12.5 mM Lactate | 146 mM Sucrose | | | | | 0.2 % (w/v) PS80 | 22.5 (12.5-32.5) | 201 (191-211) |
| F4 | 100 mg/mL | 5.9 | 20 mM Histidine | 12.5 mM Acetate | 146 mM Sucrose | | | | | 0.2 % (w/v) PS80 | 22.5 (12.5-32.5) | 201 (191-211) |
| F5 | 100 mg/mL | 5.9 | 20 mM Histidine | 12.5 mM Acetate | | 25 mM Arg.HCl | | | | 0.2 % (w/v) PS80 | 47.5 (37.5-57.5) | 105 (95-115) |
| F6 | 100 mg/mL | 5.9 | 20 mM Histidine | 12.5 mM Acetate | 146 mM Trehalose | | | | | 0.2 % (w/v) PS80 | 22.5 (12.5-32.5) | 201 (191-211) |
| F7 | 100 mg/mL | 5.9 | 20 mM Histidine | 12.5 mM Acetate | 146 mM Sorbitol | | | | | 0.2 % (w/v) PS80 | 22.5 (12.5-32.5) | 201 (191-211) |
| F8 | 100 mg/mL | 5.9 | 20 mM Histidine | 12.5 mM Acetate | 100 mM Sorbitol | | 50 mM NaCl | | | 0.2 % (w/v) PS80 | 72.5 (62.5-82.5) | 255 (245-265) |
| F9 | 100 mg/mL | 5.9 | 20 mM Histidine | 12.5 mM Acetate | 146 mM Glycerol | | | | | 0.2 % (w/v) PS80 | 22.5 (12.5-32.5) | 201 (191-211) |
| F10 | 100 mg/mL | 6.1 | 20 mM Histidine | 12.5 mM Acetate | 146 mM Sucrose | | | | | 0.2 % (w/v) PS80 | 22.5 (12.5-32.5) | 201 (191-211) |
| F11 | 100 mg/mL | 5.7 | 20 mM Histidine | 12.5 mM Acetate | 146 mM Sucrose | | | | | 0.2 % (w/v) PS80 | 22.5 (12.5-32.5) | 201 (191-211) |
| F12 | 100 mg/mL | 5.9 | 20 mM Succinate | | 146 mM Trehalose | 25 mM Arg.HCl | | | | 0.2 % (w/v) PS80 | 65 (45-85) | 246 (236-256) |
| F13 | 100 mg/mL | 5.9 | 20 mM Adipate | | 146 mM Trehalose | 25 mM Arg.HCl | | | | 0.2 % (w/v) PS80 | 65 (45-85) | 246 (236-256) |
| F14 | 100 mg/mL | 5.9 | | 20 mM Glutamate | 146 mM Trehalose | 25 mM Arg.HCl | | | | 0.2 % (w/v) PS80 | 45 | 236 |

| ID | Dupilumab* | pH | Buffer | Organic Acid Salt (Carboxylate) | Non-ionic polyol | Basic amino acid | Inorganic Salt | Non-basic/non-acidic amino acid | Antioxidant | Surfactant | Theoretical Ionic Strength mol/L | Theoretical Osmolality mOsmol/kg |
|---|---|---|---|---|---|---|---|---|---|---|---|---|
| F15 | 100 mg/mL | 5.9 | 20 mM Adipate | | 146 mM Trehalose | | | | | 0.2 % (w/v) PS80 | 40 (20-60) | 196 (186-206) |
| F16 | 100 mg/mL | 5.5 | 20 mM Adipate | | 146 mM Trehalose | | | | | 0.2 % (w/v) PS80 | 40 (20-60) | 196 (186-206) |
| F17 | 100 mg/mL | 5.9 | 20 mM Phosphate | | 146 mM Trehalose | | | | | 0.2 % (w/v) PS80 | 40 (20-60) | 196 (186-206) |
| F18 | 100 mg/mL | 5.9 | 20 mM MES | | 146 mM Trehalose | | | | | 0.2 % (w/v) PS80 | 10 (0-20) | 176 (166-186) |
| F19 | 100 mg/mL | 5.9 | | | 146 mM Trehalose | 25 mM Arg.HCl | | | | 0.2 % (w/v) PS80 | 25 | 196 |
| F20 | 100 mg/mL | 5.5 | | | 146 mM Trehalose | 25 mM Arg.HCl | | | | 0.2 % (w/v) PS80 | 25 | 196 |
| F21 | 100 mg/mL | 5.9 | 20 mM Succinate | | | 150 mM Lys.HCl | | | | 0.2 % (w/v) PS80 | 190 (170-210) | 350 (340-360) |
| F22 | 100 mg/mL | 5.9 | 20 mM Succinate | | | | | 150 mM Gly | | 0.2 % (w/v) PS80 | 40 (20-60) | 200 (190-210) |
| F23 | 100 mg/mL | 5.9 | 20 mM Succinate | | | | | 150 mM Pro | | 0.2 % (w/v) PS80 | 40 (20-60) | 200 (190-210) |
| F24 | 100 mg/mL | 5.9 | 20 mM Succinate | | 146 mM Trehalose | | | | 20 mM Met | 0.2 % (w/v) PS80 | 40 (20-60) | 216 (206-226) |
| F25 | 100 mg/mL | 5.9 | 20 mM Phosphate | 12.5 mM Lactate | 146 mM Trehalose | | | | | 0.2 % (w/v) PS80 | 52.5 (32.5-72.5) | 221 (211-231) |

* In practice the dupilumab concentration in these examples was slightly less, at 90 mg/mL.

EP 4 623 903 A1

Table 2 – Results of Screen 1 (F1-F25) at Time 0

| | Appearance | MFI #/container | | | | slope | | nanoDSF | | | | icIEF | | | cGE NR | | | SEC | | | RP | AEX | | |
|---|---|---|---|---|---|---|---|---|---|---|---|---|---|---|---|---|---|---|---|---|---|---|---|---|
| | Pass/Fail | ≥2 µm | ≥5 µm | ≥10 µm | ≥25 µm | 350 AU/mm | 550 AU/mm | TM1 | TM3 | Tmon | Taggon | acidic | main | basic | LMW | main | aggs | LMW | main | aggs | total ox | Acid | main | Basic |
| F1 | Pass | 4500 | 897 | 252 | 60 | 0.031 | 0.003 | 64.4 | 77.9 | 55.9 | 59.2 | 40.1 | 54.6 | 5.3 | 3.3 | 96.0 | 0.6 | 1.4 | 96.3 | 2.4 | 10.1 | 34.9 | 51.6 | 13.6 |
| F2 | Pass | 10566 | 1570 | 408 | 85 | 0.032 | 0.003 | 64.3 | 77.9 | 56.0 | 59.7 | 40.8 | 54.2 | 5.2 | 3.0 | 96.4 | 0.6 | 1.3 | 96.3 | 2.3 | 9.8 | 35.2 | 51.0 | 13.8 |
| F3 | Pass | 3676 | 483 | 106 | 35 | 0.028 | 0.003 | 64.9 | 77.9 | 56.4 | 71.8 | 40.1 | 54.1 | 5.8 | 3.4 | 96.1 | 0.4 | 1.3 | 96.2 | 2.4 | 10.6 | 35.5 | 50.7 | 13.8 |
| F4 | Pass | 8460 | 2540 | 932 | 217 | 0.032 | 0.003 | 65.0 | 78.0 | 56.3 | 52.0 | 41.2 | 53.8 | 5.1 | 2.8 | 96.6 | 0.6 | 1.3 | 96.2 | 2.4 | 9.9 | 35.3 | 50.9 | 13.8 |
| F5 | Pass | 6052 | 1572 | 948 | 70 | 0.032 | 0.004 | 63.5 | 77.9 | 54.7 | 58.0 | 40.0 | 54.6 | 5.4 | 3.0 | 96.5 | 0.5 | 1.3 | 96.4 | 2.3 | 10.3 | 35.0 | 50.8 | 14.2 |
| F6 | Pass | 4313 | 1005 | 326 | 30 | 0.032 | 0.003 | 64.9 | 78.1 | 56.4 | 61.7 | 40.5 | 53.8 | 6.0 | 2.9 | 96.6 | 0.5 | 1.3 | 96.3 | 2.4 | 9.9 | 35.2 | 51.1 | 13.7 |
| F7 | Pass | 4008 | 837 | 240 | 45 | 0.032 | 0.003 | 64.4 | 77.9 | 56.1 | 61.7 | 40.4 | 54.2 | 5.4 | 3.0 | 96.5 | 0.5 | 1.3 | 96.3 | 2.4 | 9.7 | 34.9 | 51.3 | 13.9 |
| F8 | Pass | 4950 | 926 | 267 | 25 | 0.033 | 0.003 | 63.6 | 77.8 | 55.4 | 59.8 | 40.3 | 54.2 | 5.5 | 2.9 | 96.5 | 0.7 | 1.3 | 96.4 | 2.3 | 9.7 | 34.7 | 51.4 | 14.0 |
| F9 | Pass | 5311 | 1476 | 446 | 81 | 0.033 | 0.003 | 64.1 | 77.8 | 55.4 | 60.5 | 40.1 | 54.6 | 5.7 | 3.3 | 96.1 | 0.6 | 1.3 | 96.3 | 2.4 | 9.5 | 35.6 | 50.7 | 13.8 |
| F10 | Pass | 5315 | 771 | 252 | 45 | 0.034 | 0.004 | 66.1 | 78.2 | 57.7 | 61.7 | 40.5 | 53.9 | 6.2 | 3.3 | 96.1 | 0.5 | 1.3 | 96.2 | 2.5 | 9.9 | 35.4 | 51.0 | 13.6 |
| F11 | Pass | 5314 | 841 | 533 | 40 | 0.029 | 0.003 | 63.9 | 78.1 | 55.7 | 60.8 | 40.0 | 54.1 | 5.9 | 2.8 | 96.6 | 0.5 | 1.3 | 96.5 | 2.2 | 9.8 | 35.5 | 50.8 | 13.7 |
| F12 | Pass | 3542 | 781 | 190 | 30 | 0.030 | 0.003 | 66.6 | 78.1 | 57.3 | 60.8 | 39.8 | 53.4 | 6.8 | 3.4 | 96.2 | 0.4 | 1.4 | 96.0 | 2.6 | 10.1 | 34.4 | 51.7 | 14.0 |
| F13 | Pass | 592 | 91 | 398 | 55 | 0.030 | 0.004 | 66.2 | 77.7 | 56.6 | 60.1 | 39.5 | 53.6 | 6.8 | 3.1 | 96.4 | 0.5 | 1.3 | 96.6 | 2.4 | 10.2 | 34.6 | 51.6 | 13.8 |
| F14 | Pass | 3652 | 863 | 292 | 58 | 0.030 | 0.003 | 66.5 | 78.0 | 56.3 | 60.1 | 39.7 | 54.0 | 6.3 | 2.9 | 96.3 | 0.6 | 1.2 | 96.2 | 2.6 | 10.4 | 34.5 | 51.7 | 13.8 |
| F15 | Pass | 1960 | 559 | 217 | 20 | 0.032 | 0.003 | 66.3 | 77.8 | 56.4 | 59.9 | 40.3 | 53.6 | 6.2 | 3.1 | 96.4 | 0.5 | 1.1 | 96.1 | 2.9 | 10.3 | 34.7 | 51.6 | 13.7 |
| F16 | Pass | 4772 | 1466 | 504 | 66 | 0.030 | 0.003 | 64.0 | 77.9 | 56.7 | 56.0 | 39.4 | 54.3 | 6.3 | 3.1 | 96.5 | 0.4 | 1.1 | 96.5 | 2.4 | 10.4 | 34.5 | 51.6 | 13.7 |
| F17 | Pass | 4038 | 872 | 207 | 40 | 0.034 | 0.003 | 66.6 | 78.0 | 56.7 | 59.3 | 40.2 | 53.6 | 6.1 | 3.4 | 96.0 | 0.6 | 1.2 | 95.7 | 3.1 | 9.9 | 34.0 | 51.6 | 13.7 |
| F18 | Pass | 3046 | 771 | 207 | 40 | 0.028 | 0.002 | 65.8 | 77.9 | 56.9 | 57.1 | 40.1 | 53.5 | 6.4 | 3.0 | 96.4 | 0.6 | 1.2 | 95.7 | 3.1 | 9.4 | 34.0 | 51.6 | 13.7 |
| F19 | Pass | 1828 | 353 | 106 | 20 | 0.032 | 0.003 | 66.6 | 77.9 | 56.7 | 60.9 | 40.6 | 53.8 | 5.6 | 3.1 | 96.1 | 0.8 | 1.2 | 96.0 | 2.6 | 9.7 | 34.4 | 51.6 | 14.2 |
| F20 | Pass | 485 | 287 | 90 | 0 | 0.029 | 0.003 | 64.2 | 78.1 | 56.6 | 60.8 | 39.4 | 54.4 | 6.2 | 2.9 | 96.4 | 0.6 | 1.2 | 96.4 | 2.4 | 9.8 | 34.5 | 51.7 | 13.9 |
| F21 | Pass | 1154 | 277 | 106 | 15 | 0.029 | 0.003 | 64.8 | 77.5 | 54.6 | 59.3 | 39.4 | 53.9 | 6.7 | 3.1 | 96.3 | 0.7 | 1.3 | 95.6 | 3.1 | 9.4 | 34.9 | 51.2 | 14.0 |
| F22 | Pass | 1703 | 297 | 45 | 0 | 0.033 | 0.004 | 65.6 | 77.9 | 56.1 | 59.3 | 39.9 | 54.1 | 5.9 | 3.1 | 96.3 | 0.7 | 1.2 | 96.0 | 2.4 | 9.9 | 35.4 | 50.6 | 13.8 |
| F23 | Pass | 8626 | 1275 | 307 | 45 | 0.034 | 0.004 | 65.8 | 77.8 | 56.4 | 58.7 | 40.4 | 53.7 | 6.0 | 4.4 | 95.1 | 0.5 | 1.2 | 96.2 | 2.6 | 10.5 | 34.9 | 51.3 | 13.8 |
| F24 | Pass | 3634 | 808 | 262 | 40 | 0.033 | 0.004 | 66.8 | 78.0 | 57.9 | 60.6 | 40.5 | 53.1 | 6.3 | 3.0 | 96.3 | 0.7 | 1.2 | 96.1 | 2.7 | 10.2 | 35.0 | 50.9 | 13.8 |
| F25 | Pass | 3809 | 925 | 290 | 60 | 0.033 | 0.003 | 66.7 | 78.1 | 56.8 | 59.9 | 39.8 | 53.9 | 6.2 | 3.8 | 96.0 | 0.7 | 1.3 | 95.8 | 2.9 | 9.8 | 34.9 | 51.2 | 13.8 |

Table 3 – Results of Screen 1 (F1-F25) after Oxidative Stress (Tox)

| | Appearance | MFI #/container | | | | slope | | IcIEF | | | cGE NR | | | SEC | | | RP |
|---|---|---|---|---|---|---|---|---|---|---|---|---|---|---|---|---|---|
| | Pass/Fail | ≥ 2 µm | ≥ 5 µm | ≥ 10 µm | ≥ 25 µm | 350 [AU/mm] | 550 [AU/mm] | acdic | main | basic | LMW | main | Aggs | LMW | main | Aggs | total ox. |
| F1 | Pass | 20939 | 4540 | 890 | 38 | 0.030 | 0.003 | 42.2 | 52.1 | 5.8 | 3.1 | 95.9 | 1.0 | 1.2 | 96.5 | 2.2 | 17.4 |
| F2 | Fail | 26492 | 5154 | 933 | 67 | 0.030 | 0.003 | 40.2 | 54.6 | 5.3 | 3.1 | 96.1 | 0.8 | 1.3 | 96.4 | 2.3 | 17.2 |
| F3 | Pass | 15871 | 3364 | 738 | 29 | 0.027 | 0.003 | 41.0 | 52.7 | 6.3 | 3.1 | 95.9 | 1.0 | 1.3 | 96.3 | 2.4 | 17.2 |
| F4 | Pass | 11212 | 2479 | 485 | 24 | 0.030 | 0.003 | 40.7 | 53.3 | 6.0 | 3.3 | 95.8 | 0.9 | 1.3 | 96.3 | 2.3 | 17.1 |
| F5 | Fail | 13710 | 3431 | 833 | 176 | 0.031 | 0.003 | 40.5 | 54.2 | 5.3 | 3.3 | 96.1 | 0.6 | 1.3 | 96.4 | 2.3 | 17.7 |
| F6 | Pass | 8109 | 1570 | 414 | 57 | 0.030 | 0.003 | 40.4 | 54.0 | 5.6 | 3.4 | 96.0 | 0.6 | 1.3 | 96.4 | 2.3 | 19.8 |
| F7 | Pass | 20530 | 4835 | 1109 | 109 | 0.031 | 0.003 | 40.2 | 53.6 | 6.2 | 3.2 | 96.1 | 0.7 | 1.3 | 96.3 | 2.4 | 17.4 |
| F8 | Pass | 12635 | 2570 | 509 | 52 | 0.031 | 0.003 | 40.9 | 52.9 | 6.2 | 3.3 | 95.7 | 1.0 | 1.3 | 96.4 | 2.3 | 17.2 |
| F9 | Pass | 12530 | 2789 | 595 | 67 | 0.031 | 0.003 | 40.3 | 53.9 | 5.8 | 3.2 | 96.0 | 0.8 | 1.3 | 96.3 | 2.4 | 16.8 |
| F10 | Pass | 8176 | 2175 | 676 | 109 | 0.032 | 0.004 | 41.5 | 53.6 | 5.0 | 3.1 | 96.2 | 0.7 | 1.3 | 96.2 | 2.5 | 16.9 |
| F11 | Pass | 21001 | 3755 | 700 | 48 | 0.028 | 0.003 | 40.8 | 54.1 | 5.1 | 3.1 | 96.2 | 0.8 | 1.3 | 96.4 | 2.3 | 16.5 |
| F12 | Pass | 11374 | 2042 | 462 | 29 | 0.029 | 0.003 | 40.0 | 54.3 | 5.8 | 3.1 | 96.0 | 1.0 | 1.3 | 96.1 | 2.6 | 17.8 |
| F13 | Pass | 7885 | 1504 | 352 | 48 | 0.029 | 0.003 | 39.9 | 54.3 | 5.9 | 3.4 | 95.6 | 1.0 | 1.3 | 96.3 | 2.4 | 16.7 |
| F14 | Pass | 8628 | 1732 | 324 | 29 | 0.029 | 0.003 | 39.3 | 55.3 | 5.5 | 3.2 | 96.1 | 0.7 | 1.3 | 96.4 | 2.2 | 16.9 |
| F15 | Pass | 5558 | 1047 | 190 | 29 | 0.032 | 0.004 | 40.1 | 54.3 | 5.7 | 3.1 | 95.8 | 1.1 | 1.3 | 96.2 | 2.5 | 16.6 |
| F16 | Fail | 16561 | 3198 | 676 | 76 | 0.029 | 0.003 | 40.2 | 54.3 | 5.5 | 3.3 | 96.1 | 0.6 | 1.3 | 96.3 | 2.3 | 16.7 |
| F17 | Pass | 20025 | 4635 | 1095 | 124 | 0.032 | 0.003 | 40.4 | 53.7 | 5.9 | 3.4 | 95.4 | 1.2 | 1.3 | 95.7 | 3.0 | 16.6 |
| F18 | Pass | 18088 | 3131 | 481 | 5 | 0.025 | 0.002 | 40.0 | 54.4 | 5.6 | 3.0 | 95.9 | 1.1 | 1.3 | 95.7 | 3.0 | 16.2 |
| F19 | Pass | 22305 | 4140 | 447 | 29 | 0.030 | 0.003 | 40.3 | 53.1 | 6.6 | 3.1 | 96.0 | 0.9 | 1.3 | 96.0 | 2.7 | 16.6 |
| F20 | Fail | 9494 | 1466 | 290 | 19 | 0.025 | 0.002 | 39.5 | 53.9 | 6.6 | 3.2 | 96.1 | 0.7 | 1.3 | 96.3 | 2.4 | 17.1 |
| F21 | Pass | 7362 | 1332 | 286 | 29 | 0.028 | 0.003 | 39.7 | 54.6 | 5.8 | 3.2 | 96.0 | 0.8 | 1.3 | 96.4 | 2.3 | 17.1 |
| F22 | Pass | 8042 | 1613 | 405 | 38 | 0.032 | 0.004 | 39.2 | 55.1 | 5.6 | 3.3 | 96.0 | 0.8 | 1.3 | 96.3 | 2.3 | 16.6 |
| F23 | Pass | 13729 | 2465 | 462 | 48 | 0.032 | 0.003 | 41.1 | 53.4 | 5.6 | 4.5 | 94.8 | 0.7 | 1.3 | 96.2 | 2.5 | 16.7 |
| F24 | Fail | 10907 | 2023 | 314 | 14 | 0.032 | 0.003 | 39.8 | 54.3 | 5.8 | 3.7 | 95.5 | 0.8 | 1.3 | 96.0 | 2.7 | 12.0 |
| F25 | Pass | 12520 | 2475 | 424 | 38 | 0.032 | 0.003 | 40.0 | 53.9 | 6.1 | 3.3 | 95.9 | 0.8 | 1.3 | 95.8 | 2.9 | 16.4 |

EP 4 623 903 A1

64

Table 4 – Results of Screen 1 (F1-F25) after thermal stress (at 40°C) for 1 month

| | Appearance Pass/Fail | MFI #/container | | | | slope | | IcIEF | | | cGE NR | | | SEC | | | RP | RP Fres | AEX | | |
|---|---|---|---|---|---|---|---|---|---|---|---|---|---|---|---|---|---|---|---|---|---|
| | | ≥ 2 µm | ≥ 5 µm | ≥ 10 µm | ≥ 25 µm | 350 AU/mm | 550 AU/mm | acdic | main | basic | LMW | main | aggs | LMW | main | HMW | total ox. | total ox. | Acid | main | Basic |
| F1 | Pass | 2993 | 907 | 292 | 15 | 0.048 | 0.004 | 54.8 | 37.6 | 7.5 | 5.5 | 93.2 | 1.2 | 1.8 | 94.7 | 3.5 | 13.2 | 11.81 | 49.9 | 36.0 | 14.0 |
| F2 | Fail | 10894 | 3855 | 1240 | 202 | 0.046 | 0.004 | 53.2 | 38.5 | 8.3 | 5.5 | 93.3 | 1.2 | 1.7 | 95.5 | 2.7 | 12.5 | | 49.6 | 36.3 | 14.1 |
| F3 | Fail | 2540 | 585 | 136 | 30 | 0.041 | 0.003 | 54.2 | 37.9 | 7.8 | 5.3 | 93.4 | 1.3 | 1.7 | 94.6 | 3.6 | 12.5 | | 49.7 | 36.2 | 14.1 |
| F4 | Fail | 1940 | 479 | 131 | 15 | 0.046 | 0.004 | 54.7 | 37.6 | 7.8 | 5.3 | 93.6 | 1.2 | 1.8 | 94.4 | 3.8 | 12.4 | | 50.5 | 35.7 | 13.8 |
| F5 | Fail | 7150 | 2257 | 660 | 111 | 0.047 | 0.004 | 53.4 | 38.8 | 7.8 | 5.9 | 93.0 | 1.1 | 1.8 | 94.7 | 3.4 | 12.0 | | 49.5 | 36.4 | 14.1 |
| F6 | Fail | 3794 | 1018 | 287 | 30 | 0.047 | 0.004 | 55.3 | 36.8 | 7.9 | 5.5 | 93.2 | 1.3 | 1.7 | 94.9 | 3.3 | 12.1 | | 50.9 | 35.3 | 13.8 |
| F7 | Pass | 3900 | 1204 | 333 | 35 | 0.046 | 0.004 | 54.7 | 37.8 | 7.6 | 5.4 | 93.3 | 1.2 | 1.7 | 94.4 | 3.8 | 12.0 | | 50.1 | 36.0 | 13.8 |
| F8 | Pass | 4091 | 1124 | 287 | 30 | 0.049 | 0.004 | 54.4 | 37.5 | 8.1 | 5.5 | 93.2 | 1.4 | 1.8 | 94.5 | 3.6 | 12.8 | | 49.5 | 35.9 | 14.6 |
| F9 | Pass | 9392 | 2938 | 690 | 35 | 0.048 | 0.004 | 55.0 | 37.3 | 7.7 | 5.3 | 93.5 | 1.3 | 1.7 | 94.1 | 4.1 | 12.2 | | 50.2 | 36.1 | 13.7 |
| F10 | Pass | 3275 | 962 | 227 | 10 | 0.059 | 0.004 | 58.6 | 34.5 | 6.9 | 6.1 | 92.2 | 1.7 | 1.9 | 93.4 | 4.7 | 14.5 | | 52.7 | 34.9 | 12.4 |
| F11 | Pass | 3774 | 1013 | 237 | 35 | 0.042 | 0.003 | 53.8 | 37.4 | 8.8 | 5.2 | 93.7 | 1.1 | 1.8 | 94.4 | 3.6 | | 10.5 | 49.4 | 36.1 | 14.5 |
| F12 | Fail | 2071 | 595 | 131 | 10 | 0.034 | 0.003 | 54.5 | 37.7 | 7.8 | 5.2 | 93.3 | 1.4 | 1.7 | 94.2 | 4.0 | 10.8 | | 49.4 | 36.5 | 14.1 |
| F13 | Fail | 3623 | 942 | 227 | 25 | 0.034 | 0.003 | 53.5 | 39.1 | 7.4 | 5.3 | 93.5 | 1.2 | 1.7 | 94.1 | 4.1 | 10.4 | | 49.0 | 37.2 | 13.8 |
| F14 | Pass | 2781 | 655 | 101 | 10 | 0.035 | 0.003 | 52.3 | 39.4 | 8.3 | 5.2 | 93.2 | 1.6 | 1.8 | 94.0 | 4.2 | 11.1 | | 47.4 | 38.4 | 14.2 |
| F15 | Pass | 2781 | 766 | 217 | 30 | 0.038 | 0.004 | 54.1 | 37.9 | 8.0 | 5.1 | 93.2 | 1.7 | 1.7 | 92.8 | 5.4 | 10.4 | | 48.1 | 38.0 | 14.0 |
| F16 | Pass | 3184 | 680 | 166 | 15 | 0.035 | 0.003 | 52.8 | 37.6 | 9.6 | 5.0 | 93.5 | 1.5 | 1.8 | 93.3 | 4.9 | 10.6 | 9.4 | 47.3 | 36.7 | 16.0 |
| F17 | Pass | 1562 | 358 | 86 | 20 | 0.040 | 0.004 | 55.2 | 36.3 | 8.5 | 5.6 | 92.3 | 2.1 | 1.7 | 92.7 | 5.5 | 11.3 | | 49.0 | 36.5 | 14.5 |
| F18 | Pass | 2726 | 680 | 237 | 20 | 0.030 | 0.003 | 53.5 | 38.5 | 8.1 | 4.9 | 93.3 | 1.9 | 1.7 | 93.3 | 5.0 | 10.9 | | 47.8 | 38.0 | 14.2 |
| F19 | Fail | 3235 | 887 | 161 | 15 | 0.036 | 0.004 | 52.3 | 39.6 | 8.2 | 5.2 | 93.4 | 1.4 | 1.7 | 94.1 | 4.1 | 10.4 | | 47.0 | 39.0 | 14.0 |
| F20 | Pass | 1769 | 302 | 60 | 5 | 0.030 | 0.003 | 50.9 | 39.5 | 9.7 | 5.0 | 93.8 | 1.2 | 1.8 | 94.1 | 4.1 | 10.4 | | 45.5 | 38.6 | 16.0 |
| F21 | Pass | 2217 | 600 | 151 | 5 | 0.038 | 0.003 | 52.7 | 38.9 | 8.4 | 6.3 | 92.7 | 1.1 | 1.9 | 94.2 | 3.8 | 11.6 | | 48.7 | 37.2 | 14.1 |
| F22 | Pass | 3567 | 1255 | 353 | 76 | 0.044 | 0.004 | 59.5 | 33.2 | 7.3 | 5.8 | 91.4 | 2.7 | 1.9 | 91.5 | 6.5 | 12.5 | | 52.8 | 34.3 | 12.9 |
| F23 | Fail | 6359 | 1718 | 418 | 40 | 0.040 | 0.004 | 55.1 | 37.1 | 7.8 | 7.1 | 91.3 | 1.5 | 1.8 | 93.6 | 4.5 | 10.6 | | 49.1 | 37.3 | 13.6 |
| F24 | Fail | 4127 | 927 | 262 | 35 | 0.038 | 0.004 | 55.7 | 37.0 | 7.3 | 5.5 | 92.7 | 1.8 | 1.8 | 93.4 | 4.7 | 9.9 | 8.7 | 49.6 | 36.8 | 13.6 |
| F25 | Fail | 4671 | 1461 | 338 | 60 | 0.036 | 0.004 | 54.7 | 36.7 | 8.7 | 5.3 | 92.6 | 2.1 | 1.8 | 93.0 | 5.1 | 10.5 | | 48.3 | 36.8 | 14.8 |

**Table 5** - **SCREEN 2 Formulations**

| ID | Dupilumab | pH | Buffer | Organic Acid Salt (Carboxylate) | Non-ionic polyol | Basic amino acid | Inorganic Salt | Non-basic/non-acidic amino acid | Antioxidant | Surfactant | Theoretical Ionic Strength mol/L | Theoretical Osmolality mOsmol/kg |
|---|---|---|---|---|---|---|---|---|---|---|---|---|
| F26 | 152 mg/mL | 5.9 | 20 mM Histidine | 12.5 mM Acetate | 146 mM Sucrose | | | | | 0.2 % (w/v) PS80 | 22.5 (12.5-32.5) | 201 (191-211) |
| F27 | 152 mg/mL | 5.9 | 20 mM Histidine | 12.5 mM Acetate | 146 mM Sucrose | 25 mM Arg.HCl | | | | 0.2 % (w/v) PS80 | 47.5 (37.5-57.5) | 251 (241-261) |
| F28 | 152 mg/mL | 5.9 | 20 mM Histidine | 12.5 mM Acetate | 146 mM Sucrose | 50 mM Arg.HCl | | | | 0.2 % (w/v) PS80 | 72.5 (62.5-82.5) | 301 (291-311) |
| F29 | 152 mg/mL | 5.9 | 20 mM Histidine | 12.5 mM Acetate | 146 mM Sucrose | | | 50 mM proline | | 0.2 % (w/v) PS80 | 22.5 (12.5-32.5) | 251 (241-261) |
| F30 | 152 mg/mL | 5.9 | 20 mM Histidine | 12.5 mM Acetate | 146 mM Sucrose | | | 150 mM proline | | 0.2 % (w/v) PS80 | 22.5 (12.5-32.5) | 351 (341-361) |
| F31 | 152 mg/mL | 5.9 | 20 mM Histidine | 12.5 mM Acetate | 146 mM Sucrose | 50 mM lysine.HCl | | | | 0.2 % (w/v) PS80 | 72.5 (62.5-82.5) | 301 (291-311) |
| F32 | 152 mg/mL | 5.9 | 20 mM Histidine | 12.5 mM Acetate | 146 mM Sucrose | 150 mM lysine.HCl | | | | 0.2 % (w/v) PS80 | 172.5 (162.5-182.5) | 501 (491-511) |
| F33 | 152 mg/mL | 5.9 | 20 mM Histidine | 12.5 mM Acetate | 146 mM Sucrose | | | 150 mM glycine | | 0.2 % (w/v) PS80 | 22.5 (12.5-32.5) | 351 (341-361) |
| F34 | 152 mg/mL | 5.9 | 20 mM Histidine | 12.5 mM Acetate | 146 mM Sucrose | | 50 mM NaCl | | | 0.2 % (w/v) PS80 | 72.5 (62.5-82.5) | 301 (291-311) |

**Table 6 - SCREEN 3 Formulations**

| ID | Dupilumab* | pH | Buffer | Organic Acid Salt (Carboxylate) | Non-ionic polyol | Basic amino acid | Inorganic Salt | Non-basic/non-acidic amino acid | Surfactant | Theoretical Ionic Strength mol/L | Theoretical Osmolality mOsmol/kg |
|---|---|---|---|---|---|---|---|---|---|---|---|
| F35 | 175 mg/ml | 5.9 | 20 mM Histidine | 12.5 mM Acetate | 146 mM Sucrose | 50 mM Arg.HCl | | | 0.2 % (w/v) PS80 | 72.5 (62.5-82.5) | 301 (291-311) |
| F36 | 175 mg/ml | 5.7 | 20 mM succinate | | | 125 mM Lys.HCl | | | 0.2 % (w/v) PS80 | 165 (145-185) | 300 (290-310) |
| F37 | 175 mg/ml | 5.9 | 20 mM succinate | | 146 mM Trehalose | 50 mM Lys.HCl | | | 0.2 % (w/v) PS80 | 90 (70-110) | 296 (286-306) |
| F38 | 175 mg/ml | 5.7 | 20 mM succinate | | | | 125 mM NaCl | | 0.2 % (w/v) PS80 | 165 (145-185) | 300 (290-310) |
| F39 | 175 mg/ml | 5.7 | 20 mM succinate | | 146 mM Trehalose | 50 mM Lys.HCl | | | 0.2 % (w/v) PS80 | 90 (70-110) | 296 (286-306) |
| F40 | 175 mg/ml | 5.9 | 20 mM MES | 12.5 mM lactic acid | 146 mM Trehalose | 50 mM Lys.HCl | | | 0.2 % (w/v) PS80 | 72.5 (62.5-82.5) | 301 (291-311) |
| F41 | 175 mg/ml | 5.7 | | 20 mM glutamate | 146 mM Trehalose | 50 mM Lys.HCl | | | 0.2 % (w/v) PS80 | 70 | 286 |
| F42 | 175 mg/ml | 5.9 | 20 mM phosphate | 12.5 mM lactate | | 125 mM Lys.HCl | | | 0.2 % (w/v) PS80 | 177.5 (157.5-197.5) | 325 (315-335) |
| F43 | 175 mg/ml | 5.9 | | | 146 mM Trehalose | 75 mM Lys.HCl | | | 0.2 % (w/v) PS80 | 75 | 296 |
| F44 | 175 mg/ml | 5.9 | | 12.5 mM lactate | 146 mM Trehalose | 50 mM Lys.HCl | | | 0.2 % (w/v) PS80 | 62.5 | 271 |
| F45 | 175 mg/ml | 5.9 | | 12.5 mM lactate | 146 mM Trehalose | 50 mM Lys.HCl | | | 0.2% (w/v) Px188 | 62.5 | 271 |
| F46 | 175 mg/ml | 5.9 | | 12.5 mM lactate | | 122.7 mM Arg.HCl + 12.3 mM Arg | | | 0.2 % (w/v) PS80 | 135.2 | 282.7 |

In practice the dupilumab concentration in these examples was slightly less, at about 155 mg/mL.

Table 7 – Visibles and Sub-visibles results of Screen 3 (F35-F46) at Time 0

| Method readout | Appearance Pass/Fail | MFI #/container | | | | MFI #/container (non-silicon) | | | | MFI #/container (silicon) | | | |
|---|---|---|---|---|---|---|---|---|---|---|---|---|---|
| | | MFI ≥ 2 µm | MFI ≥ 5 µm | MFI ≥ 10 µm | MFI ≥ 25 µm | MFI ≥ 2 µm | MFI ≥ 5 µm | MFI ≥ 10 µm | MFI ≥ 25 µm | MFI ≥ 2 µm | MFI ≥ 5 µm | MFI ≥ 10 µm | MFI ≥ 25 µm |
| F35 | Pass | 20864 | 2157 | | | 18750 | | | | 2114 | | | 0 |
| F36 | Pass | 34817 | 4568 | 801 | | 31642 | 2963 | 502 | | | | | 0 |
| F37 | Pass | 16036 | 2114 | | | 16947 | | | | | | | 0 |
| F38 | Fail | | | 1977 | 136 | 57607 | 9199 | 1698 | 124 | | 3050 | | |
| F39 | Pass | 12893 | | | | 11492 | | | | | | | |
| F40 | Pass | 43247 | 5703 | 768 | 43 | 40259 | 4041 | 528 | | | | | |
| F41 | Pass | 23560 | 2796 | | | 19631 | | | | | 2207 | | |
| F42 | Pass | 18306 | 2151 | | | 16674 | | | | | | | |
| F43 | Pass | 11672 | | | | 10065 | | | | | | | |
| F44 | Pass | 27726 | 4010 | 583 | | 22327 | | | | | 3155 | | |
| F45 | Pass | 34156 | 6942 | 1283 | 81 | 25370 | | | | | 5936 | 1159 | 68 |
| F46 | Pass | 11554 | | | | 10116 | | | | | | | |

Table 8 – Other results of Screen 3 (F35-F46) at Time 0

| | Turbidity NTU | Visco mPa's | nanoDSF | | | | | icIEF | | | AEX | | | cGE NR | | | SEC | | | RP | | |
|---|---|---|---|---|---|---|---|---|---|---|---|---|---|---|---|---|---|---|---|---|---|---|
| | | | Tm1 | Tm2 | Tm3 | Tonset | Tagg | acidic | main | basic | acidic | main | basic | LMW | main | aggregates | LMW | main | aggregates | total ox. | ox. Fc/2 | ox. Fd |
| F35 | 22.9 | 8.7 | 64.2 | 69.3 | 77.6 | 55.7 | 58.8 | 40.8 | 54.0 | 5.1 | 34 | 51.9 | 14.1 | 2.9 | 96.6 | 0.5 | 0.0 | 95.9 | 2.4 | 8.6 | 5.6 | 1.3 |
| F36 | 27.3 | 8.7 | 63.4 | 67.8 | 77.2 | 55.4 | 57.8 | 39.0 | 54.9 | 6.1 | 33.7 | 52.2 | 14.1 | 2.9 | 96.5 | 0.6 | 0.0 | 95.9 | 2.5 | 8.6 | 5.4 | 1.3 |
| F37 | 27.5 | 8.6 | 66.3 | n.d. | 78.1 | 58.2 | 60.7 | 39.3 | 54.6 | 6.1 | 33.9 | 51.9 | 14.2 | 3.1 | 96.3 | 0.6 | 0.0 | 95.5 | 2.9 | 8.2 | 5.7 | 1.4 |
| F38 | 28.3 | 8.4 | 62.1 | 68.0 | 77.1 | 54.7 | 57.4 | 39.3 | 54.6 | 6.1 | 33.5 | 52.4 | 14.0 | 3.0 | 96.1 | 0.8 | 0.1 | 95.0 | 4.4 | 9.1 | 5.7 | 1.4 |
| F39 | 28.4 | 9.4 | 64.9 | 70.0 | 78.2 | 55.6 | 58.7 | 39.1 | 54.2 | 6.6 | 33.8 | 51.9 | 14.2 | 3.0 | 96.2 | 0.8 | 0.1 | 95.1 | 3.2 | 8.8 | 5.6 | 1.4 |
| F40 | 28.3 | 9.5 | 65.1 | 70.0 | 78.1 | 55.7 | 58.2 | 39.0 | 54.5 | 6.5 | 33.7 | 52.1 | 14.1 | 3.0 | 96.3 | 0.7 | 0.1 | 95.4 | 2.9 | 8.8 | 5.4 | 1.4 |
| F41 | 28.7 | 9.4 | 65.1 | 70.0 | 79.2 | 55.1 | 58.2 | 38.4 | 54.6 | 6.1 | 34.0 | 51.9 | 14.1 | 3.1 | 96.2 | 0.7 | 0.0 | 95.8 | 2.6 | 8.9 | 5.6 | 1.3 |
| F42 | 31.7 | 8.2 | 64.5 | 68.6 | 77.5 | 54.8 | 58.3 | 39.0 | 54.9 | 6.1 | 33.8 | 52.1 | 14.2 | 2.9 | 96.5 | 0.6 | 0.1 | 95.8 | 2.6 | 8.7 | 5.4 | 1.4 |
| F43 | 29.2 | 8.0 | 66.0 | 69.9 | 78.1 | 55.7 | 59.3 | 39.5 | 54.1 | 6.4 | 33.7 | 52.1 | 14.2 | 3.2 | 96.0 | 0.8 | 0.1 | 95.6 | 2.6 | 8.7 | 5.4 | 1.3 |
| F44 | 30.1 | 9.2 | 66.1 | n.d. | 78.3 | 55.8 | 59.3 | 39.5 | 54.4 | 6.2 | 33.8 | 52.1 | 14.2 | 3.1 | 96.1 | 0.8 | 0.0 | 95.7 | 2.7 | 9.0 | 5.6 | 1.3 |
| F45 | 34.5 | 8.7 | 66.4 | 70.0 | 78.1 | 56.0 | 58.8 | 39.7 | 54.0 | 6.3 | 34.2 | 51.6 | 14.2 | 3.2 | 96.3 | 0.5 | 0.1 | 95.6 | 2.8 | 8.5 | 5.3 | 1.3 |
| F46 | 24.4 | 9.3 | 64.8 | n.d. | 77.2 | 55.6 | 58.7 | 39 | 54.6 | 6.0 | 33.7 | 52.0 | 14.3 | 3.0 | 96.1 | 0.8 | 0.1 | 95.8 | 2.0 | 8.7 | 5.5 | 1.2 |

Table 9 - Results of Screen 3 (F35-F46) after 3 freeze-thaw cycles

| Method | Appearance | MFI #/container | | | | MFI #/container (non-silicon) | | | | MFI #/container (silicon) | | | | Turbidity | SEC | | | AEX | | |
|---|---|---|---|---|---|---|---|---|---|---|---|---|---|---|---|---|---|---|---|---|
| readout | Pass/Fail | ≥ 2 µm | ≥ 5 µm | ≥ 10 µm | ≥ 25 µm | ≥ 2 µm | ≥ 5 µm | ≥ 10 µm | ≥ 25 µm | ≥ 2 µm | ≥ 5 µm | ≥ 10 µm | ≥ 25 µm | NTU | LMW | main | Aggs | acidic | main | basic |
| F35 | Pass | 81852 | 10029 | 1116 | 124 | 72404 | 4023 | 682 | 118 | 9248 | 6006 | 434 | 6 | 22.7 | 0.1 | 95.7 | 2.4 | 34.7 | 51.3 | 14.0 |
| F36 | Pass | 84863 | 8008 | 948 | 87 | 76136 | 3304 | 576 | 81 | 8727 | 4705 | 372 | 6 | 31.8 | 0.1 | 95.7 | 2.4 | 33.9 | 52.0 | 14.1 |
| F37 | Pass | 80772 | 6502 | 738 | 50 | 73179 | 1946 | 248 | 43 | 7593 | 4556 | 490 | 6 | 27.1 | 0.1 | 95.2 | 2.9 | 33.7 | 52.2 | 14.1 |
| F38 | Fail | 106936 | 11697 | 1091 | 31 | 93126 | 4141 | 508 | 25 | 13810 | 7457 | 583 | 6 | 36.4 | 0.1 | 94.7 | 3.4 | 33.8 | 52.2 | 14.1 |
| F39 | Pass | 102777 | 14108 | 1252 | 124 | 89289 | 4519 | 391 | 93 | 13488 | 9589 | 862 | 31 | 27.0 | 0.1 | 95.5 | 2.6 | 33.9 | 52.0 | 14.1 |
| F40 | Pass | 117715 | 13779 | 1736 | 68 | 106868 | 6942 | 1066 | 56 | 10847 | 6837 | 669 | 12 | 28.6 | 0.1 | 95.5 | 2.7 | 33.8 | 52.1 | 14.1 |
| F41 | Fail | 93262 | 7531 | 713 | 74 | 83630 | 1711 | 186 | 56 | 9632 | 5820 | 527 | 19 | 26.7 | 0.1 | 95.6 | 2.6 | 33.8 | 52.0 | 14.2 |
| F42 | Fail | 109651 | 11114 | 651 | 37 | 99659 | 4661 | 229 | 31 | 9992 | 6453 | 422 | 6 | 32.2 | 0.1 | 95.6 | 2.6 | 33.6 | 52.3 | 14.1 |
| F43 | Pass | 137166 | 11715 | 1884 | 37 | 122327 | 2405 | 198 | 12 | 14839 | 9310 | 1686 | 25 | 29.5 | 0.1 | 95.5 | 2.7 | 33.6 | 52.1 | 14.3 |
| F44 | Pass | 100564 | 7153 | 564 | 6 | 92587 | 2461 | 87 | 6 | 7977 | 4692 | 477 | 0 | 29.6 | 0.0 | 95.6 | 2.8 | 33.8 | 51.8 | 14.4 |
| F45 | Fail | 238957 | 30596 | 2678 | 62 | 197217 | 5535 | 223 | 0 | 41740 | 25060 | 2455 | 62 | 34.7 | 0.0 | 95.6 | 2.8 | 33.8 | 52.1 | 14.2 |
| F46 | Pass | 98928 | 9496 | 1816 | 19 | 85750 | 1798 | 211 | 12 | 13178 | 7699 | 1605 | 6 | 24.8 | 0.1 | 95.9 | 2.6 | 33.5 | 52.3 | 14.3 |

Table 10 - Results of Screen 3 (F35-F46) after mechanical stress

| Method readout | Appearance Pass/Fail | MFI #/container | | | | MFI #/container (non-silicon) | | | | MFI #/container (silicon) | | | | Turbidity NTU | SEC | | | AEX | | |
|---|---|---|---|---|---|---|---|---|---|---|---|---|---|---|---|---|---|---|---|---|
| | | ≥ 2 µm | ≥ 5 µm | ≥ 10 µm | ≥ 25 µm | ≥ 2 µm | ≥ 5 µm | ≥ 10 µm | ≥ 25 µm | ≥ 2 µm | ≥ 5 µm | ≥ 10 µm | ≥ 25 µm | | LMW | main | HMW | acidic | main | basic |
| F35 | Pass | 25730 | 4010 | 676 | 37 | 22023 | 1798 | 329 | 37 | 3707 | 2213 | 347 | 0 | 26.2 | 0.1 | 84.0 | 14.7 | 33.8 | 51.9 | 14.3 |
| F36 | Pass | 146625 | 39137 | 11380 | 1308 | 144827 | 38083 | 11213 | 1308 | 1798 | 1054 | 167 | 0 | 43.5 | 0.1 | 80.3 | 18.3 | 33.4 | 52.3 | 14.3 |
| F37 | Pass | 31011 | 3874 | 434 | 19 | 26343 | 1203 | 236 | 12 | 4667 | 2672 | 198 | 6 | 31.4 | 0.1 | 89.8 | 8.9 | 33.3 | 52.3 | 14.3 |
| F38 | Pass | 36788 | 5628 | 1264 | 105 | 34420 | 4469 | 1097 | 105 | 2368 | 1159 | 167 | 0 | 39.5 | 0.1 | 89.5 | 9.1 | 33.7 | 52.2 | 14.2 |
| F39 | Pass | 28060 | 3316 | 477 | 31 | 24056 | 930 | 149 | 19 | 4004 | 2386 | 329 | 12 | 33.7 | 0.1 | 77.9 | 20.8 | 33.3 | 52.3 | 14.4 |
| F40 | Pass | 74921 | 20845 | 7971 | 341 | 72417 | 19252 | 7779 | 341 | 2504 | 1593 | 192 | 0 | 35.3 | 0.1 | 83.8 | 14.9 | 33.6 | 52.1 | 14.3 |
| F41 | Pass | 27540 | 3824 | 304 | 0 | 22618 | 725 | 118 | 0 | 4922 | 3099 | 186 | 0 | 31.8 | 0.1 | 82.8 | 15.8 | 33.8 | 51.9 | 14.4 |
| F42 | Pass | 11783 | 1339 | 267 | 37 | 10296 | 483 | 124 | 31 | 1488 | 855 | 143 | 6 | 37.5 | 0.1 | 84.4 | 14.2 | 33.8 | 52.0 | 14.2 |
| F43 | Pass | 13816 | 1494 | 335 | 12 | 12180 | 589 | 155 | 12 | 1636 | 905 | 180 | 0 | 46.6 | 0.1 | 62.3 | 36.3 | 34.2 | 51.3 | 14.5 |
| F44 | Pass | 91384 | 10854 | 1141 | 6 | 74456 | 1277 | 99 | 0 | 16928 | 9577 | 1041 | 6 | 39.1 | 0.1 | 74.7 | 23.9 | 33.6 | 52.0 | 14.4 |
| F45 | Pass | 13110 | 1903 | 403 | 12 | 10823 | 508 | 105 | 6 | 2287 | 1395 | 298 | 6 | 34.5 | 0.1 | 95.7 | 3.0 | 33.9 | 51.8 | 14.3 |
| F46 | Pass | 61142 | 8622 | 1333 | 112 | 50344 | 2907 | 632 | 74 | 10798 | 5715 | 700 | 37 | 25.3 | 0.1 | 92.3 | 6.3 | 34.1 | 51.7 | 14.2 |

EP 4 623 903 A1

Table 11 – Visibles and sub-visibles results of Screen 3 (F35-F46) after thermal stress (at 40°C) for 1 month

| Method readout | Appearance Pass/Fail | MFI #/container (Total) | | | | MFI #/container (non-silicon) | | | | MFI #/container (silicon) | | | |
|---|---|---|---|---|---|---|---|---|---|---|---|---|---|
| | | MFI ≥ 2 µm | MFI ≥ 5 µm | MFI ≥ 10 µm | MFI ≥ 25 µm | MFI ≥ 2 µm | MFI ≥ 5 µm | MFI ≥ 10 µm | MFI ≥ 25 µm | MFI ≥ 2 µm | MFI ≥ 5 µm | MFI ≥ 10 µm | MFI ≥ 25 µm |
| F35 | Pass | 26604 | 4203 | 638 | 37 | 22048 | 1227 | 205 | 25 | 4556 | 2975 | 434 | 12 |
| F36 | Fail | 477437 | 190733 | 51336 | 1860 | 420114 | 140395 | 33466 | 1624 | 57323 | 50338 | 17870 | 236 |
| F37 | Pass | 42292 | 4215 | 496 | 37 | 37625 | 1711 | 310 | 37 | 4667 | 2504 | 186 | 0 |
| F38 | Fail | 761432 | 266404 | 68227 | 2814 | 714101 | 223374 | 51838 | 2238 | 47332 | 43030 | 16389 | 576 |
| F39 | Pass | 23213 | 2386 | 322 | 25 | 20641 | 917 | 87 | 19 | 2572 | 1469 | 236 | 6 |
| F40 | Fail | 291626 | 102095 | 26176 | 961 | 272547 | 86140 | 19166 | 750 | 19079 | 15955 | 7010 | 211 |
| F41 | Pass | 163683 | 20294 | 1934 | 130 | 135647 | 4432 | 229 | 25 | 28036 | 15862 | 1705 | 105 |
| F42 | Pass | 37321 | 4618 | 570 | 31 | 32158 | 1810 | 217 | 12 | 5163 | 2808 | 353 | 19 |
| F43 | Pass | 131649 | 23201 | 3068 | 174 | 102485 | 4141 | 316 | 19 | 29164 | 19060 | 2752 | 155 |
| F44 | Pass | 33751 | 4395 | 638 | 50 | 28023 | 986 | 112 | 19 | 5727 | 3409 | 527 | 31 |
| F45 | Pass | 7785 | 1928 | 428 | 93 | 6019 | 576 | 229 | 43 | 1767 | 1351 | 198 | 50 |
| F46 | Pass | 26232 | 2820 | 353 | 19 | 23722 | 1506 | 198 | 6 | 2510 | 1314 | 155 | 12 |

Table 12 – Other results of Screen 3 (F35-F46) after thermal stress (at 40°C) for 1 month

| Method | Turbidity | IcIEF | | | AEX | | | cGE NR | | | SEC | | | RP | | |
|---|---|---|---|---|---|---|---|---|---|---|---|---|---|---|---|---|
| readout | NTU | acidic | main | basic | acidic | main | basic | LMW | main | aggregates | LMW | main | HMW | total ox. | ox. Fc/2 | ox. Fd |
| F35 | 24.1 | 53.8 | 38.8 | 7.4 | 48.7 | 36.9 | 14.4 | 5.7 | 93.1 | 1.2 | 0.1 | 93.6 | 4.2 | 11.7 | 7.8 | 1.7 |
| F36 | 55.1 | 51.4 | 40.5 | 8.2 | 46.6 | 38.4 | 15.0 | 5.2 | 93.5 | 1.3 | 0.1 | 93.3 | 4.7 | 10.1 | 5.8 | 1.7 |
| F37 | 30.2 | 53.7 | 39.1 | 7.2 | 47.9 | 37.9 | 14.2 | 5.5 | 92.8 | 1.7 | 0.1 | 93.0 | 5.1 | 10.7 | 6.7 | 2.0 |
| F38 | 60.7 | 52.2 | 40.1 | 7.7 | 46.1 | 39.2 | 14.8 | 4.9 | 93.1 | 1.9 | 0.1 | 92.1 | 6.0 | 9.5 | 5.8 | 1.6 |
| F39 | 28.7 | 53.2 | 38.5 | 8.3 | 47.1 | 37.6 | 15.3 | 5.3 | 93.2 | 1.6 | 0.1 | 93.0 | 5.0 | 10.8 | 6.5 | 1.7 |
| F40 | 37.4 | 52.4 | 39.6 | 8.1 | 46.7 | 38.3 | 15.0 | 5.1 | 93.3 | 1.7 | 0.1 | 92.8 | 5.3 | 10.1 | 6.0 | 1.7 |
| F41 | 29.1 | 51.9 | 40.0 | 8.1 | 46.3 | 38.1 | 15.7 | 5.2 | 93.2 | 1.6 | 0.1 | 93.0 | 5.1 | 11.2 | 6.9 | 1.7 |
| F42 | 35.2 | 52.6 | 40.3 | 7.1 | 47.0 | 37.7 | 15.3 | 6.2 | 91.9 | 1.9 | 0.1 | 92.5 | 5.4 | 11.4 | 6.7 | 1.9 |
| F43 | 30.5 | 52.4 | 39.9 | 7.8 | 46.1 | 39.2 | 14.8 | 5.3 | 93.1 | 1.6 | 0.1 | 93.3 | 4.8 | 10.6 | 6.4 | 1.8 |
| F44 | 30.4 | 52.1 | 40.2 | 7.7 | 46.1 | 38.5 | 15.5 | 5.1 | 93.4 | 1.5 | 0.1 | 93.2 | 4.8 | 10.5 | 6.3 | 1.6 |
| F45 | 33.5 | 51.7 | 40.3 | 8.0 | 45.9 | 38.7 | 15.4 | 5.0 | 93.0 | 2.0 | 0.1 | 93.0 | 5.0 | 10.3 | 6.4 | 1.5 |
| F46 | 25.9 | 51.1 | 41.5 | 7.4 | 46.0 | 38.3 | 14.7 | 5.8 | 92.6 | 1.6 | 0.1 | 93.3 | 4.6 | 11.4 | 7.2 | 1.5 |

EP 4 623 903 A1

72

Table 13 - Visibles and sub-visibles results of Screen 3 (F35-F46) after thermal stress (at 40°C) for 2 month

| Method | Appearance | MFI #/container (Total) | | | | MFI #/container (non-silicon) | | | | MFI #/container (silicon) | | | |
|---|---|---|---|---|---|---|---|---|---|---|---|---|---|
| readout | Pass/Fail | MFI ≥ 2 μm | MFI ≥ 5 μm | MFI ≥ 10 μm | MFI ≥ 25 μm | MFI ≥ 2 μm | MFI ≥ 5 μm | MFI ≥ 10 μm | MFI ≥ 25 μm | MFI ≥ 2 μm | MFI ≥ 5 μm | MFI ≥ 10 μm | MFI ≥ 25 μm |
| F35 | Pass | 66974 | 11709 | 2120 | 242 | 4488 | 880 | 229 | 43 | 62487 | 10829 | 1891 | 198 |
| F36 | Fail | 2237523 | 868536 | 204103 | 8349 | 675583 | 153995 | 40024 | 1494 | 1561940 | 714541 | 164080 | 6856 |
| F37 | Pass | 81026 | 11095 | 2114 | 161 | 4041 | 874 | 229 | 43 | 76985 | 10221 | 1884 | 118 |
| F38 | Fail | 1314478 | 455997 | 104928 | 4506 | 325283 | 69714 | 15980 | 750 | 989195 | 386282 | 88948 | 3756 |
| F39 | Pass | 74946 | 11814 | 3161 | 384 | 3577 | 682 | 198 | 74 | 71369 | 11132 | 2963 | 310 |
| F40 | Fail | 337792 | 103093 | 16810 | 589 | 66987 | 11232 | 2045 | 136 | 270805 | 91861 | 14765 | 452 |
| F41 | Pass | 176904 | 28048 | 4246 | 279 | 5523 | 855 | 149 | 31 | 171381 | 27193 | 4097 | 248 |
| F42 | Pass | 74754 | 12254 | 2250 | 378 | 3620 | 924 | 223 | 31 | 71134 | 11331 | 2027 | 347 |
| F43 | Pass | 172107 | 33156 | 6955 | 576 | 5510 | 787 | 161 | 31 | 166596 | 32368 | 6794 | 545 |
| F44 | Pass | 196051 | 39943 | 10004 | 725 | 6099 | 862 | 186 | 19 | 189952 | 39081 | 9818 | 707 |
| F45 | Pass | 19773 | 3608 | 880 | 192 | 2579 | 1141 | 434 | 93 | 17195 | 2467 | 446 | 99 |
| F46 | Pass | 322791 | 42348 | 4271 | 229 | 10854 | 2052 | 415 | 56 | 311938 | 40296 | 3855 | 174 |

Table 14 - Other results of Screen 3 (F35-F46) after thermal stress (at 40°C) for 2 month

| Method | Turbidity | IcIEF | | | AEX | | | cGE NR | | | SEC | | | RP | | | | LC-CAD |
|---|---|---|---|---|---|---|---|---|---|---|---|---|---|---|---|---|---|---|
| readout | NTU | acidic | main | basic | acidic | main | basic | LMW | main | aggregates | LMW | main | HMW | total ox. | ox. Fc/2 | ox. Fd | ox. LC | % target |
| F35 | 26.4 | 65.3 | 27.7 | 7.0 | 61.5 | 25.9 | 12.6 | 8.1 | 90.1 | 1.8 | 0.2 | 91.9 | 6.4 | 13.5 | 9.0 | 1.3 | 3.2 | 83.9 |
| F36 | 58.1 | 62.5 | 30.0 | 7.5 | 59.0 | 27.6 | 13.4 | 7.9 | 89.8 | 2.3 | 0.2 | 91.7 | 6.4 | 10.7 | 6.1 | 1.3 | 3.2 | 55.0 |
| F37 | 31.2 | 65.6 | 27.7 | 6.7 | 61.2 | 26.4 | 9.8 | 8.6 | 88.9 | 2.4 | 0.1 | 91.4 | 7.0 | 17.6 | 9.2 | 1.2 | 7.1 | 86.4 |
| F38 | 65.0 | 63.6 | 29.4 | 7.0 | 55.0 | 28.1 | 12.9 | 7.4 | 89.4 | 3.2 | 0.1 | 89.8 | 8.6 | 11.2 | 6.3 | 1.4 | 3.6 | 49.1 |
| F39 | 29.8 | 63.9 | 28.2 | 7.9 | 59.7 | 26.7 | 13.6 | 8.1 | 89.0 | 2.9 | 0.1 | 91.2 | 7.1 | 13.2 | 7.6 | 1.2 | 4.4 | 98.1 |
| F40 | 44.4 | 62.9 | 29.3 | 7.9 | 58.3 | 28.4 | 13.4 | 7.3 | 89.8 | 2.9 | 0.2 | 91.2 | 7.1 | 11.0 | 6.1 | 1.2 | 3.7 | 95.8 |
| F41 | 31.5 | 62.6 | 28.9 | 8.5 | 58.1 | 27.4 | 14.5 | 8.1 | 88.9 | 3.0 | 0.1 | 91.3 | 7.0 | 12.9 | 8.1 | 1.4 | 3.4 | 105.4 |
| F42 | 38.8 | 63.9 | 27.6 | 8.6 | 59.5 | 26.1 | 14.4 | 10.1 | 86.9 | 3.0 | 0.2 | 89.8 | 8.4 | 13.4 | 8.1 | 1.4 | 3.9 | 95.8 |
| F43 | 33.7 | 62.3 | 29.9 | 7.8 | 55.2 | 28.0 | 13.4 | 8.4 | 88.7 | 2.9 | 0.1 | 91.7 | 6.7 | 12.7 | 7.9 | 1.5 | 3.3 | 99.1 |
| F44 | 32.2 | 62.2 | 29.3 | 8.5 | 57.5 | 28.2 | 14.4 | 7.9 | 89.3 | 2.9 | 0.1 | 91.2 | 7.1 | 12.2 | 7.7 | 1.2 | 3.3 | 99.4 |
| F45 | 36.3 | 62.0 | 29.5 | 8.5 | 58.5 | 27.3 | 14.2 | 7.8 | 89.2 | 3.0 | 0.1 | 91.4 | 7.0 | 11.8 | 7.3 | 1.3 | 3.2 | 90.1 |
| F46 | 28.7 | 61.3 | 30.7 | 8.0 | 57.3 | 29.3 | 13.4 | 8.7 | 89.1 | 2.2 | 0.2 | 91.0 | 7.2 | 12.8 | 7.9 | 1.3 | 3.6 | 92.0 |

**Table 15** - **SCREEN 4 Formulations**

| ID | Dupilumab | pH | Buffer | Organic Acid Salt (Carboxylate) | Non-ionic polyol | Basic amino acid | Inorganic Salt | Non-basic/non-acidic amino acid | Surfactant | Theoretical Ionic Strength mol/L | Theoretical Osmolality mOsmol/kg |
|---|---|---|---|---|---|---|---|---|---|---|---|
| F35 | 175 mg/ml | 5.9 | 20 mM Histidine | 12.5 mM Acetate | 146 mM Sucrose | 50 mM Arg.HCl | | | 0.2 % (w/v) PS80 | 72.5 (62.5-82.5) | 301 (291-311) |
| F47 | 175 mg/ml | 5.9 | | 12.5 mM Lactate | | 135 mM Arg.HCl | | | 0.2 % (w/v) PS80 | 147.5 | 295 |
| F48 | 175 mg/ml | 5.9 | 20 mM succinate | | 100 mM Trehalose | 75 mM Lys.HCl | | | 0.2 % (w/v) PS80 | 115 (95-135) | 300 (290-310) |
| F49 | 175 mg/ml | 5.7 | 20 mM succinate | | 146 mM Trehalose | 50 mM Lys.HCl | | | 0.2 % (w/v) Px188 | 90 (70-110) | 296 (286-306) |
| F50 | 175 mg/ml | 5.9 | | 20 mM Glutamate | 146 mM Trehalose | 50 mM Lys.HCl | | | 0.2 % (w/v) PS80 | 70 | 286 |

**Table 16** - **SCREEN X Formulations**

| ID | Dupilumab | pH | Buffer | Organic Acid Salt (Carboxylate) | Non-ionic polyol | Basic amino acid | Inorganic Salt | Surfactant | Theoretical Ionic Strength mol/L | Theoretical Osmolality mOsmol/kg |
|---|---|---|---|---|---|---|---|---|---|---|
| CF_Control | 175 mg/ml | 5.9 | 20 mM histidine | 12.5 mM acetate | 146 mM sucrose | 50 mM Arg.HCl | | 0.2 % (w/v) PS80 | 72.5 (62.5-82.5) | 301 (291-311) |
| CF1 | 175 mg/ml | 5.9 | 10 mM succinate | 12.5 mM lactate | 146 mM trehalose | 50 mM Lys.HCl | | 0.2 % (w/v) PS80 | 72.5-92.5 (82.5) | 291-301 (296) |
| CF2 | 175 mg/ml | 5.9 | 10 mM succinate | 12.5 mM lactate | 146 mM trehalose | 50 mM Lys.HCl | | 0.2 % (w/v) Px188 | 72.5-92.5 (82.5) | 291-301 (296) |
| CF3 | 175 mg/ml | 5.9 | 10 mM phosphate | 12.5 mM lactate | 146 mM trehalose | 50 mM Lys.HCl | | 0.2 % (w/v) PS80 | 72.5-92.5 (82.5) | 291-301 (296) |
| CF4 | 175 mg/ml | 5.9 | 10 mM succinate | 12.5 mM lactate | 146 mM trehalose | 67.5 mM Arg.HCl 7.5 mM Arg | | 0.2 % (w/v) PS80 | 90-110 (100) | 333.5-343.5 (338.5) |
| CF5 | 175 mg/ml | 5.9 | 10 mM succinate | 20 mM glutamate | 146 mM trehalose | 67.5 mM Arg.HCl 7.5 mM Arg | | 0.2 % (w/v) PS80 | 97.5-117.5 (107.5) | 348.5-358.5 (353.5) |
| CF6 | 175 mg/ml | 5.9 | 10 mM succinate | 20 mM glutamate | 146 mM trehalose | 67.5 mM Arg.HCl 7.5 mM Arg | | 0.1 % (w/v) Px188 | 97.5-117.5 (107.5) | 348.5-358.5 (353.5) |
| CF7 | 175 mg/ml | 5.9 | - | 12.5 mM lactate | 146 mM trehalose | 67.5 mM Arg.HCl 7.5 mM Arg | | 0.2 % (w/v) PS80 | 80 | 313.5 |
| CF8 | 175 mg/ml | 5.9 | - | 12.5 mM lactate | 146 mM trehalose | 67.5 mM Arg.HCl 7.5 mM Arg | | 0.2 % (w/v) Px188 | 80 | 313.5 |
| CF9 | 175 mg/ml | 5.9 | - | 20 mM glutamate | 146 mM trehalose | 50 mM Lys.HCl | | 0.2 % (w/v) PS80 | 70 | 286 |

(continued)

| ID | Dupilumab | pH | Buffer | Organic Acid Salt (Carboxylate) | Non-ionic polyol | Basic amino acid | Inorganic Salt | Surfactant | Theoretical Ionic Strength mol/L | Theoretical Osmolality mOsmol/kg |
|---|---|---|---|---|---|---|---|---|---|---|
| CF10 | 175 mg/ml | 5.9 | - | 20 mM glutamate | 146 mM trehalose | 50 mM Lys.HCl | | 0.2 % (w/v) Px188 | 70 | 286 |
| CF11 | 175 mg/ml | 5.9 | - | 20 mM glutamate | 146 mM trehalose | 45 mM Lys.HCl 5 mM Lys | | 0.2 % (w/v) PS80 | 65 | 281 |
| CF12 | 175 mg/ml | 5.9 | - | 20 mM glutamate | 146 mM trehalose | 45 mM Lys.HCl 5 mM Lys | | 0.2 % (w/v) Px188 | 65 | 281 |
| CF13 | 175 mg/ml | 5.9 | 10 mM succinate | 12.5 mM lactate | 146 mM trehalose | | 50 mM NaCl | 0.2 % (w/v) PS80 | 72.5-92.5 (82.5) | 291-301 (296) |
| CF14 | 175 mg/ml | 5.9 | - | 20 mM glutamate | 146 mM trehalose | | 50 mM NaCl | 0.2 % (w/v) PS80 | 70 | 286 |
| CF15 | 175 mg/ml | 5.9 | 20 mM succinate | 20 mM lactate | | 100 mM Lys.HCl | | 0.2 % (w/v) PS80 | 160 (140-180) | 290 (280-300) |
| CF16 | 175 mg/ml | 5.9 | 20 mM succinate | 20 mM lactate | | 100 mM Lys.HCl | | 0.2 % (w/v) Px188 | 160 (140-180) | 290 (280-300) |
| CF17 | 175 mg/ml | 5.9 | 20 mM phosphate | 20 mM lactate | | 100 mM Lys.HCl | | 0.2 % (w/v) PS80 | 160 (140-180) | 290 (280-300) |
| CF18 | 175 mg/ml | 5.9 | 20 mM succinate | 20 mM lactate | | 100 mM Arg.HCl | | 0.2 % (w/v) PS80 | 160 (140-180) | 290 (280-300) |
| CF19 | 175 mg/ml | 5.9 | 20 mM succinate | 20 mM glutamate | | 100 mM Arg.HCl | | 0.2 % (w/v) PS80 | 160 (140-180) | 290 (280-300) |
| CF20 | 175 mg/ml | 5.9 | 20 mM succinate | 20 mM glutamate | | 100 mM Arg.HCl | | 0.1 % (w/v) Px188 | 160 (140-180) | 290 (280-300) |
| CF21 | 175 mg/ml | 5.9 | - | 20 mM lactate | | 125 mM Arg.HCl | | 0.2 % (w/v) PS80 | 145 | 290 |
| CF22 | 175 mg/ml | 5.9 | - | 20 mM lactate | | 125 mM Arg.HCl | | 0.2 % (w/v) Px188 | 145 | 290 |

EP 4 623 903 A1

| ID | Dupilumab | pH | Buffer | Organic Acid Salt (Carboxylate) | Non-ionic polyol | Basic amino acid | Inorganic Salt | Surfactant | Theoretical Ionic Strength mol/L | Theoretical Osmolality mOsmol/kg |
|---|---|---|---|---|---|---|---|---|---|---|
| CF23 | 175 mg/ml | 5.9 | - | 20 mM glutamate | | 125 mM Lys.HCl | | 0.2 % (w/v) PS80 | 145 | 290 |
| CF24 | 175 mg/ml | 5.9 | - | 20 mM glutamate | | 125 mM Lys.HCl | | 0.2 % (w/v) Px188 | 145 | 290 |
| CF25 | 175 mg/ml | 5.9 | 20 mM succinate | | | 125 mM Lys.HCl | | 0.2 % (w/v) PS80 | 165 (145-185) | 300 (290-310) |
| CF26 | 175 mg/ml | 5.9 | 20 mM succinate | | | 125 mM Lys.HCl | | 0.2 % (w/v) Px188 | 165 (145-185) | 300 (290-310) |
| CF27 | 175 mg/ml | 5.9 | 20 mM succinate | 20 mM lactate | | | 100 mM NaCl | 0.2 % (w/v) PS80 | 160 (140-180) | 290 (280-300) |
| CF28 | 175 mg/ml | 5.9 | - | 20 mM glutamate | | | 125 mM NaCl | 0.2 % (w/v) PS80 | 145 | 290 |
| • 10 mM of succinate or phosphate contributes an ionic strength of 10-30 mM (midpoint 20) and an osmolality of 20-30 mOsm/kg (midpoint 25) • 20 mM of succinate or phosphate contributes an ionic strength of 20-60 mM (midpoint 40) and an osmolality of 40-60 mOsm/kg (midpoint 50) | | | | | | | | | | |

*ABBREVIATIONS*

|  |  |
|---|---|
| AEX | Anion-exchange chromatography |
| CDR | complementarity-determining region |
| CEX | Cation-exchange chromatography |
| cGE | capillary gel electrophoresis. If NR then non-reduced. |
| DSF | Differential scanning fluorimetry |
| HMW | high molecular weight species |
| HPLC | high performance liquid chromatography |
| IclEF | Imaged capillary isoelectric focusing |
| MES | 2-(N-morpholino)ethanesulfonic acid |
| MFI | Micro-Flow Imaging |
| MW | molecular weight |
| nanoDSF | nano differential scanning fluorimetry |
| OD | Optical density |
| PES | polyether sulfone |
| pI | isoelectric point |
| PS | polysorbate |
| RP-HPLC | reversed-phase high performance liquid chromatography (sometimes just RP) |
| RT | Room temperature |
| s.c. | subcutaneous |
| RP-UPLC | Reverse phase UPLC |
| SEC | Size exclusion chromatography |
| SE-UPLC | Size exclusion UPLC |
| SE-HPLC | Size exclusion high performance liquid chromatography |
| TFF | Tangential flow filtration |
| UPLC | ultra-performance liquid chromatography |
| UV | ultraviolet |
| Vis | visible |

**Claims**

1. A liquid pharmaceutical composition comprising dupilumab, a surfactant, and water, wherein the composition:

   • has a pH of 5.6-6.2;
   • is free of at least one of histidine, acetate, arginine, and sucrose.

2. The liquid pharmaceutical composition as claimed in claim 1, wherein the composition is free of all of histidine, acetate, and sucrose.

3. The liquid pharmaceutical composition as claimed in any preceding claim, wherein the composition comprises 90-210 mg/mL dupilumab and 0.1-3 mg/mL surfactant.

4. The liquid pharmaceutical composition as claimed in any preceding claim, wherein the surfactant is polysorbate 80 or poloxamer 188; wherein said surfactant is preferably present at a concentration of 0.5-2.5 mg/mL.

5. The liquid pharmaceutical composition as claimed in any preceding claim, wherein the composition further comprises a non-ionic polyol that is a sugar component, wherein the sugar component is trehalose, sorbitol, or mannitol, most preferably trehalose; wherein the non-ionic polyol is preferably present at a concentration of 70-230 mM.

6. The liquid pharmaceutical composition as claimed in any preceding claim, wherein the composition further comprises an organic acid salt, wherein the organic acid salt is a lactate salt, a glutamate salt, or an acetate salt, most preferably a lactate salt or a glutamate salt; wherein the organic acid salt is preferably present at a concentration of 2-25 mM.

7. The liquid pharmaceutical composition as claimed in any preceding claim, wherein the composition further comprises a basic amino acid, wherein the basic amino acid is lysine or arginine (most preferably lysine); wherein the basic amino acid is preferably present at a concentration of 15-150 mM, more preferably 15-100 mM.

8. The liquid pharmaceutical composition as claimed in any preceding claim, wherein the composition further comprises an inorganic salt, wherein the inorganic salt is sodium chloride; wherein the inorganic salt is preferably present at a concentration of 20-80 mM.

9. The liquid pharmaceutical composition as claimed in any preceding claim, wherein the composition further comprises a buffer, which buffer is a succinate buffer, a phosphate buffer, or an MES buffer; wherein said buffer is preferably present at a concentration of 3-30 mM.

10. The liquid pharmaceutical composition as claimed in any of claims 1 to 8, wherein the composition is free of any buffer having conjugate acid form with a pKa that is within +/-1 of the pH.

11. The liquid pharmaceutical composition as claimed in any preceding claim, wherein the composition has an ionic strength is 50-190 mM.

12. The liquid pharmaceutical composition as claimed in any preceding claim, wherein the composition has a pH of 5.6-6.2 and comprises (more preferably consists of, together with any other components stipulated in any preceding claim, especially water) either:

> • dupilumab, a non-ionic polyol, a basic amino acid, and surfactant in a respective molar ratio of 1 : 20-500 : 5-300 : 0.01-3; OR
> • dupilumab, a non-ionic polyol, a basic amino acid, and surfactant in a respective molar ratio of 1 : 50-250 : 10-200 : 0.1-2; OR
> • dupilumab, a non-ionic polyol, a basic amino acid, and surfactant in a respective molar ratio of 1 : 100-150 : 25-150 : 1-1.7; OR
> • dupilumab, a non-ionic polyol, an inorganic salt, and surfactant in a respective molar ratio of 1 : 20-500 : 5-300 : 0.01-3; OR
> • A liquid pharmaceutical composition comprising dupilumab, a non-ionic polyol, an inorganic salt, and surfactant in a respective molar ratio of 1 : 50-250 : 10-200 : 0.1-2;OR
> • dupilumab, a non-ionic polyol, an inorganic salt, and surfactant in a respective molar ratio of 1 : 100-150 : 25-150 : 1-1.7; OR
> • dupilumab, a non-ionic polyol, an organic acid salt, and surfactant in a respective molar ratio of 1 : 20-500 : 2-50 : 0.01-3; OR
> • dupilumab, a non-ionic polyol, an organic acid salt, and surfactant in a respective molar ratio of 1 : 50-250 : 5-40 : 0.1-2; OR
> • dupilumab, a non-ionic polyol, an organic acid salt, and surfactant in a respective molar ratio of 1 : 100-150 : 8-25 : 1-1.7; OR
> • dupilumab, a non-ionic polyol, an organic acid salt, a basic amino acid, and surfactant in a respective molar ratio of 1 : 20-500 : 2-50 : 5-300 : 0.01-3; OR
> • dupilumab, a non-ionic polyol, an organic acid salt, a basic amino acid, and surfactant in a respective molar ratio of 1 : 50-250 : 5-40 : 10-200 : 0.1-2; OR
> • dupilumab, a non-ionic polyol, an organic acid salt, a basic amino acid, and surfactant in a respective molar ratio of 1 : 100-150 : 8-25 : 25-150 : 1-1.7; OR
> • dupilumab, a non-ionic polyol, an organic acid salt, an inorganic salt, and surfactant in a respective molar ratio of 1 : 20-500 : 2-50 : 5-300 : 0.01-3; OR

• dupilumab, a non-ionic polyol, an organic acid salt, an inorganic salt, and surfactant in a respective molar ratio of 1 : 50-250 : 5-40 : 10-200 : 0.1-2; OR

• dupilumab, a non-ionic polyol, an organic acid salt, an inorganic salt, and surfactant in a respective molar ratio of 1 : 100-150 : 8-25 : 25-150 : 1-1.7; OR

• dupilumab, a basic amino acid, and surfactant in a respective molar ratio of 1 : 5-300 : 0.01-3; OR

• dupilumab, a basic amino acid, and surfactant in a respective molar ratio of 1 : 10-200 : 0.1-2; OR

• dupilumab, a basic amino acid, and surfactant in a respective molar ratio of 1 : 25-150 : 1-1.7; OR

• dupilumab, an inorganic salt, and surfactant in a respective molar ratio of 1 : 5-300 : 0.01-3; OR

• A liquid pharmaceutical composition comprising dupilumab, an inorganic salt, and surfactant in a respective molar ratio of 1 : 10-200 : 0.1-2; OR

• dupilumab, an inorganic salt, and surfactant in a respective molar ratio of 1 : 25-150 : 1-1.7; OR

• dupilumab, an organic acid salt, and surfactant in a respective molar ratio of 1 : 2-50 : 0.01-3; OR

• dupilumab, an organic acid salt, and surfactant in a respective molar ratio of 1 : 5-40 : 0.1-2; OR

• dupilumab, an organic acid salt, and surfactant in a respective molar ratio of 1 : 8-25 : 1-1.7; OR

• dupilumab, an organic acid salt, a basic amino acid, and surfactant in a respective molar ratio of 1 : 2-50 : 5-300 : 0.01-3; OR

• dupilumab, an organic acid salt, a basic amino acid, and surfactant in a respective molar ratio of 1 : 5-40 : 10-200 : 0.1-2; OR

• dupilumab, an organic acid salt, a basic amino acid, and surfactant in a respective molar ratio of 1 : 8-25 : 25-150 : 1-1.7; OR

• dupilumab, an organic acid salt, an inorganic salt, and surfactant in a respective molar ratio of 1 : 2-50 : 5-300 : 0.01-3; OR

• dupilumab, an organic acid salt, an inorganic salt, and surfactant in a respective molar ratio of 1 : 5-40 : 10-200 : 0.1-2; OR

• dupilumab, an organic acid salt, an inorganic salt, and surfactant in a respective molar ratio of 1 : 8-25 : 25-150 : 1-1.7;

wherein the composition preferably has an ionic strength between 10-200 mM (more preferably between 25-100 mM);

optionally wherein the composition further comprises a buffer (preferably selected from a succinate buffer, a phosphate buffer, or an MES buffer, most preferably a succinate buffer), in which case the molar ratio of said buffer to dupilumab is preferably 2-50 : 1.

13. The liquid pharmaceutical composition as claimed in any preceding claim, wherein the composition has a pH of 5.6-6.0 and comprises (more preferably consists of, along with water) either:

• 90-210 mg/mL dupilumab, 3-30 mM buffer (preferably selected from succinate, phosphate, or MES buffer), 70-230 mM sugar component (preferably trehalose), 20-80 mM basic amino acid (preferably lysine), and 0.5-2.5 mg/mL surfactant (preferably polysorbate 80); OR

• 90-210 mg/mL dupilumab, 3-30 mM buffer (preferably selected from succinate, phosphate, or MES buffer), 70-230 mM sugar component (preferably trehalose), 20-80 mM basic amino acid (preferably lysine), and 0.5-2.5 mg/mL surfactant (preferably polysorbate 80); OR

• 90-210 mg/mL dupilumab, 5-30 mM organic acid salt (preferably glutamate), 70-230 mM sugar component (preferably trehalose), 20-80 mM basic amino acid (preferably lysine), and 0.5-2.5 mg/mL surfactant (preferably polysorbate 80); OR

• 90-210 mg/mL dupilumab, 5-25 mM organic acid salt (preferably lactate), 70-230 mM sugar component (preferably trehalose), 20-80 mM basic amino acid (preferably lysine), and 0.5-2.5 mg/mL surfactant (preferably polysorbate 80); OR

• 90-210 mg/mL dupilumab, 5-25 mM organic acid salt (preferably lactate), 70-230 mM sugar component (preferably trehalose), 20-80 mM basic amino acid (preferably lysine), and 0.5-2.5 mg/mL surfactant (preferably poloxamer 188); OR

• 90-210 mg/mL dupilumab, 5-25 organic acid salt (preferably lactate), 50-140 mM basic amino acid (preferably arginine, preferably partial hydrochloride salt thereof), and 0.5-2.5 mg/mL surfactant (preferably polysorbate 80); OR

• 90-210 mg/mL mg/mL dupilumab, 3-30 mM buffer (preferably selected from succinate, phosphate, or MES buffer), 50-230 mM sugar component (preferably trehalose), 50-100 mM basic amino acid (preferably lysine), and 0.5-2.5 mg/mL surfactant (preferably polysorbate 80); OR

• 90-210 mg/mL mg/mL dupilumab, 3-30 mM buffer (preferably selected from succinate, phosphate, or MES buffer), 90-150 mM basic amino acid (preferably lysine), and 0.5-2.5 mg/mL surfactant (preferably poloxamer 188); OR

• 90-210 mg/mL mg/mL dupilumab, 5-30 mM organic acid salt (preferably lactate), 90-150 mM basic amino acid (preferably arginine), and 0.5-2.5 mg/mL surfactant (preferably polysorbate 80); OR

• 90-210 mg/mL mg/mL dupilumab, 5-30 mM organic acid salt (preferably lactate), 90-150 mM basic amino acid (preferably arginine), and 0.5-2.5 mg/mL surfactant (preferably poloxamer 188); OR

• 90-210 mg/mL mg/mL dupilumab, 5-30 mM organic acid salt (preferably glutamate), 50-230 mM sugar component (preferably trehalose), 40-80 mM basic amino acid (preferably lysine), and 0.5-2.5 mg/mL surfactant (preferably polysorbate 80); OR

• 90-210 mg/mL mg/mL dupilumab, 3-30 mM buffer (preferably selected from succinate, phosphate, or MES buffer), 50-230 mM sugar component (preferably trehalose), 40-80 mM basic amino acid (preferably lysine), and 0.5-2.5 mg/mL surfactant (preferably poloxamer 188).

14. The liquid pharmaceutical composition as claimed in any preceding claim, wherein the composition has a pH of 5.6-6.2 and comprises (more preferably consists of) either:

• 90-210 mg/mL dupilumab, 5-25 mM buffer (preferably selected from succinate, phosphate, or MES, most preferably succinate), 5-25 mM organic acid salt (preferably selected from lactate or acetate, most preferably lactate), 70-230 mM sugar component (preferably trehalose), 20-100 mM basic amino acid (preferably selected from lysine or arginine, most preferably lysine), 0.1-2.5 mg/mL surfactant (preferably selected from polysorbate 80 or poloxamer 188, preferably polysorbate 80); OR

• 90-210 mg/mL dupilumab, 5-25 mM buffer (preferably selected from succinate, phosphate, or MES, most preferably succinate), 5-25 mM organic acid salt (preferably selected from lactate or acetate, most preferably lactate), 70-230 mM sugar component (preferably trehalose), 20-100 mM basic amino acid (preferably selected from lysine or arginine, most preferably lysine), 0.1-2.5 mg/mL surfactant (preferably selected from poloxamer 188 or polysorbate 80, preferably poloxamer 188); OR

• 90-210 mg/mL dupilumab, 5-25 mM buffer (preferably selected from succinate, phosphate, or MES, most preferably phosphate), 5-25 mM organic acid salt (preferably selected from lactate or acetate, most preferably lactate), 70-230 mM sugar component (preferably trehalose), 20-100 mM basic amino acid (preferably selected from lysine or arginine, most preferably lysine), 0.1-2.5 mg/mL surfactant (preferably selected from polysorbate 80 or poloxamer 188, preferably polysorbate 80); OR

• 90-210 mg/mL dupilumab, 5-25 mM buffer (preferably selected from succinate, phosphate, or MES, most preferably succinate), 5-25 mM organic acid salt (preferably selected from lactate or acetate, most preferably lactate), 70-230 mM sugar component (preferably trehalose), 20-100 mM basic amino acid (preferably selected from arginine or lysine, most preferably arginine), 0.1-2.5 mg/mL surfactant (preferably selected from polysorbate 80 or poloxamer 188, preferably polysorbate 80); OR

• 90-210 mg/mL dupilumab, 5-25 mM buffer (preferably selected from succinate, phosphate, or MES, most preferably succinate), 5-30 mM organic acid salt (preferably selected from glutamate, lactate, or acetate, most preferably glutamate), 70-230 mM sugar component (preferably trehalose), 20-100 mM basic amino acid (preferably selected from arginine or lysine, most preferably arginine), 0.1-2.5 mg/mL surfactant (preferably selected from polysorbate 80 or poloxamer 188, preferably polysorbate 80); OR

• 90-210 mg/mL dupilumab, 5-25 mM buffer (preferably selected from succinate, phosphate, or MES, most preferably succinate), 5-30 mM organic acid salt (preferably selected from glutamate, lactate, or acetate, most preferably glutamate), 70-230 mM sugar component (preferably trehalose), 20-100 mM basic amino acid (preferably selected from arginine or lysine, most preferably arginine), 0.1-1.5 mg/mL surfactant (preferably selected from poloxamer 188 or polysorbate 80, preferably poloxamer 188); OR

• 90-210 mg/mL dupilumab, 5-25 mM organic acid salt (preferably selected from lactate or acetate, most preferably lactate), 70-230 mM sugar component (preferably trehalose), 20-100 mM basic amino acid (preferably selected from arginine or lysine, most preferably arginine), 0.1-2.5 mg/mL surfactant (preferably selected from polysorbate 80 or poloxamer 188, preferably polysorbate 80); OR

• 90-210 mg/mL dupilumab, 5-25 mM organic acid salt (preferably selected from lactate or acetate, most preferably lactate), 70-230 mM sugar component (preferably trehalose), 20-100 mM basic amino acid (preferably selected from arginine or lysine, most preferably arginine), 0.1-2.5 mg/mL surfactant (preferably selected from poloxamer 188 or polysorbate 80, preferably poloxamer 188); OR

• 90-210 mg/mL dupilumab, 5-30 mM organic acid salt (preferably selected from glutamate, lactate, or acetate, most preferably glutamate), 70-230 mM sugar component (preferably trehalose), 20-100 mM basic amino acid (preferably selected from lysine or arginine, most preferably lysine), 0.1-2.5 mg/mL surfactant (preferably selected from polysorbate 80 or poloxamer 188, preferably polysorbate 80); OR

- 90-210 mg/mL dupilumab, 5-30 mM organic acid salt (preferably selected from glutamate, lactate, or acetate, most preferably glutamate), 70-230 mM sugar component (preferably trehalose), 20-100 mM basic amino acid (preferably selected from lysine or arginine, most preferably lysine), 0.1-2.5 mg/mL surfactant (preferably selected from poloxamer 188 or polysorbate 80, preferably poloxamer 188); OR

- 90-210 mg/mL dupilumab, 5-30 mM organic acid salt (preferably selected from glutamate, lactate, or acetate, most preferably glutamate), 70-230 mM sugar component (preferably trehalose), 20-100 mM basic amino acid partial salt (preferably with 1-30% free base and 70-99% salt, preferably selected from lysine or arginine, most preferably lysine), 0.1-2.5 mg/mL surfactant (preferably selected from polysorbate 80 or poloxamer 188, preferably polysorbate 80); OR

- 90-210 mg/mL dupilumab, 5-30 mM organic acid salt (preferably selected from glutamate, lactate, or acetate, most preferably glutamate), 70-230 mM sugar component (preferably trehalose), 20-100 mM basic amino acid partial salt (preferably with 1-30% free base and 70-99% salt, preferably selected from lysine or arginine, most preferably lysine), 0.1-2.5 mg/mL surfactant (preferably selected from poloxamer 188 or polysorbate 80, preferably poloxamer 188); OR

- 90-210 mg/mL dupilumab, 5-25 mM buffer (preferably selected from succinate, phosphate, or MES, most preferably succinate), 5-25 mM organic acid salt (preferably selected from lactate or acetate, most preferably lactate), 70-230 mM sugar component (preferably trehalose), 10-100 mM inorganic salt (preferably sodium chloride), 0.1-2.5 mg/mL surfactant (preferably selected from polysorbate 80 or poloxamer 188, preferably polysorbate 80); OR

- 90-210 mg/mL dupilumab, 5-30 mM organic acid salt (preferably selected from glutamate, lactate, or acetate, most preferably glutamate), 70-230 mM sugar component (preferably trehalose), 10-100 mM inorganic salt (preferably sodium chloride), 0.1-2.5 mg/mL surfactant (preferably selected from polysorbate 80 or poloxamer 188, preferably polysorbate 80).

- 90-210 mg/mL dupilumab, 5-30 mM buffer (preferably selected from succinate buffer, phosphate, or MES, most preferably succinate), 5-30 mM organic acid salt (preferably selected from lactate or acetate, most preferably lactate), 50-150 mM basic amino acid (preferably selected from lysine or arginine, most preferably lysine), 0.1-2.5 mg/mL surfactant (preferably selected from polysorbate 80 or poloxamer 188, preferably polysorbate 80); OR

- 90-210 mg/mL dupilumab, 5-30 mM buffer (preferably selected from succinate buffer, phosphate, or MES, most preferably succinate), 5-30 mM organic acid salt (preferably selected from lactate or acetate, most preferably lactate), 50-150mM basic amino acid (preferably selected from lysine or arginine, most preferably lysine), 0.1-2.5 mg/mL surfactant (preferably selected from polysorbate 80 or poloxamer 188, preferably poloxamer 188); OR

- 90-210 mg/mL dupilumab, 5-30 mM phosphate buffer, 5-30 mM organic acid salt (preferably selected from lactate or acetate, most preferably lactate), 50-150mM basic amino acid (preferably selected from lysine or arginine, most preferably lysine), 0.1-2.5 mg/mL surfactant (preferably selected from polysorbate 80 or poloxamer 188, preferably polysorbate 80); OR

- 90-210 mg/mL dupilumab, 5-30 mM buffer (preferably selected from succinate buffer, phosphate, or MES, most preferably succinate), 5-30 mM organic acid salt (preferably selected from lactate or acetate, most preferably lactate), 50-150 mM basic amino acid (preferably selected from arginine or lysine, most preferably arginine), 0.1-2.5 mg/mL surfactant (preferably selected from polysorbate 80 or poloxamer 188, preferably polysorbate 80); OR

- 90-210 mg/mL dupilumab, 5-30 mM buffer (preferably selected from succinate buffer, phosphate, or MES, most preferably succinate), 5-30 mM organic acid salt (preferably selected from glutamate, lactate, or acetate, most preferably glutamate), 50-150 mM basic amino acid (preferably selected from arginine or lysine, most preferably arginine), 0.1-2.5 mg/mL surfactant (preferably selected from polysorbate 80 or poloxamer 188, preferably polysorbate 80); OR

- 90-210 mg/mL dupilumab, 5-30 mM buffer (preferably selected from succinate buffer, phosphate, or MES, most preferably succinate), 5-30 mM organic acid salt (preferably selected from glutamate, lactate, or acetate, most preferably glutamate), 50-150 mM basic amino acid (preferably selected from arginine or lysine, most preferably arginine), 0.1-1.5 mg/mL surfactant (preferably selected from polysorbate 80 or poloxamer 188, preferably poloxamer 188); OR

- 90-210 mg/mL dupilumab, 5-30 mM organic acid salt (preferably selected from lactate or acetate, most preferably lactate), 90-150 mM basic amino acid (preferably selected from arginine or lysine, most preferably arginine), 0.1-2.5 mg/mL surfactant (preferably selected from polysorbate 80 or poloxamer 188, preferably polysorbate 80); OR

- 90-210 mg/mL dupilumab, 5-30 mM organic acid salt (preferably selected from lactate or acetate, most preferably lactate), 90-150 mM basic amino acid (preferably selected from arginine or lysine, most preferably arginine), 0.1-2.5 mg/mL surfactant (preferably selected from polysorbate 80 or poloxamer 188, preferably poloxamer 188); OR

• 90-210 mg/mL dupilumab, 5-30 mM organic acid salt (preferably selected from glutamate, lactate, or acetate, most preferably glutamate), 90-150 basic amino acid (preferably selected from lysine or arginine, most preferably lysine), 0.1-2.5 mg/mL surfactant (preferably selected from polysorbate 80 or poloxamer 188, preferably polysorbate 80); OR

• 90-210 mg/mL dupilumab, 5-30 mM organic acid salt (preferably selected from glutamate, lactate, or acetate, most preferably glutamate), 90-150 basic amino acid (preferably selected from lysine or arginine, most preferably lysine), 0.1-2.5 mg/mL surfactant (preferably selected from polysorbate 80 or poloxamer 188, preferably poloxamer 188); OR

• 90-210 mg/mL dupilumab, 5-30 mM buffer (preferably selected from succinate buffer, phosphate, or MES, most preferably succinate), 90-150 basic amino acid (preferably selected from lysine or arginine, most preferably lysine), 0.1-2.5 mg/mL surfactant (preferably selected from polysorbate 80 or poloxamer 188, preferably polysorbate 80); OR

• 90-210 mg/mL dupilumab, 5-30 mM buffer (preferably selected from succinate buffer, phosphate, or MES, most preferably succinate), 90-150 basic amino acid (preferably selected from lysine or arginine, most preferably lysine), 0.1-2.5 mg/mL surfactant (preferably selected from polysorbate 80 or poloxamer 188, preferably poloxamer 188); OR

• 90-210 mg/mL dupilumab, 5-30 mM buffer (preferably selected from succinate buffer, phosphate, or MES, most preferably succinate), 5-30 mM organic acid salt (preferably selected from lactate or acetate, most preferably lactate), 50-150 mM inorganic salt (preferably sodium chloride), 0.1-2.5 mg/mL surfactant (preferably selected from polysorbate 80 or poloxamer 188, preferably polysorbate 80); OR

• 90-210 mg/mL dupilumab, 5-30 mM organic acid salt (preferably selected from glutamate, lactate, or acetate, most preferably glutamate), 90-150 mM inorganic salt (preferably sodium chloride), 0.1-2.5 mg/mL surfactant (preferably selected from polysorbate 80 or poloxamer 188, preferably polysorbate 80).

15. A liquid pharmaceutical composition, as claimed in any preceding claim, for use in treating a disease or medical disorder in a patient in need of such treatment, wherein the disease or medical disorder is preferably atopic dermatitis (suitably moderate-to-severe), asthma (suitably moderate-to-severe), chronic rhinosinusitis with nasal polyposis (CRSwNP), and/or eosinophilic esophagitis.

**Figure 1**

Europäisches
Patentamt
European
Patent Office
Office européen
des brevets

# EUROPEAN SEARCH REPORT

**Application Number**

EP 24 16 7606

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/215267 A1 (REGENERON PHARMA [US]) 9 November 2023 (2023-11-09) | 1-4, 6-11,15 | INV.<br>A61K9/08<br>A61K39/395 |
| Y | * claims 1,3 * | 5 | A61K47/12 |
| A | * paragraph [0125]; table 1 *<br>* paragraph [0123] *<br>* paragraph [0066]; claims 11,13,19,23-26 * | 12-14 | A61K47/18<br>A61K47/26<br>A61K47/34<br>A61P11/02 |
| | ----- | | A61P11/06 |
| X | WO 2023/011502 A1 (GAN & LEE PHARMACEUTICALS CO LTD [CN]) 9 February 2023 (2023-02-09) | 1-15 | A61P17/00<br>A61P37/00 |
| Y | * claim 33; examples 1-4,6,7,9,10,12,13,15,17-19 * | 1-15 | |
| | ----- | | |
| Y | EP 3 939 609 A1 (SUZHOU CONNECT BIOPHARMACEUTICALS LTD [CN]) 19 January 2022 (2022-01-19)<br>* claim 1 *<br>* paragraph [0058]; example 4; compounds 1-8 *<br>* paragraph [0004] * | 1-15 | |
| | ----- | | |

**TECHNICAL FIELDS SEARCHED (IPC)**

A61K
C07K
A61P

The present search report has been drawn up for all claims

| Place of search | Date of completion of the search | Examiner |
|---|---|---|
| Munich | 19 August 2024 | Cetinkaya, Murat |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 24 16 7606

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2024

| Patent document cited in search report | Publication date | Patent family member(s) | | Publication date |
|---|---|---|---|---|
| WO 2023215267 A1 | 09-11-2023 | TW | 202406572 A | 16-02-2024 |
| | | US | 2024024472 A1 | 25-01-2024 |
| | | WO | 2023215267 A1 | 09-11-2023 |
| WO 2023011502 A1 | 09-02-2023 | CN | 117693362 A | 12-03-2024 |
| | | WO | 2023011502 A1 | 09-02-2023 |
| EP 3939609 A1 | 19-01-2022 | AU | 2020235770 A1 | 28-10-2021 |
| | | BR | 112021017890 A2 | 07-12-2021 |
| | | CA | 3133138 A1 | 17-09-2020 |
| | | CN | 111686247 A | 22-09-2020 |
| | | CN | 113660950 A | 16-11-2021 |
| | | EP | 3939609 A1 | 19-01-2022 |
| | | IL | 286235 A | 31-10-2021 |
| | | JP | 2022524632 A | 09-05-2022 |
| | | KR | 20210137489 A | 17-11-2021 |
| | | SA | 521430342 B1 | 11-12-2023 |
| | | SG | 11202109975Y A | 28-10-2021 |
| | | TW | 202100142 A | 01-01-2021 |
| | | US | 2022348666 A1 | 03-11-2022 |
| | | WO | 2020182197 A1 | 17-09-2020 |
| | | ZA | 202107515 B | 21-12-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012047954 A1
- US 7608693 B
- US 8735095 B
- WO 2016077675 A1

**Non-patent literature cited in the description**

- *CHEMICAL ABSTRACTS*, 1190264-60-8